# EUROPEAN PATENT APPLICATION

(11) **EP 4 714 939 A1**
(43) Date of publication of application: **25.03.2026**
(21) Application number: 24810302.0
(22) Date of filing: 17.05.2024
(51) Int. Cl.: C07D 207/08, A61K 31/4025, A61P 9/00, A61K 31/40, A61P 7/02

(54) **SULFONAMIDE COMPOUND, PREPARATION METHOD THEREFOR, AND PHARMACEUTICAL USE THEREOF**

(30) Priority: 19.05.2023 CN 202310570515; 05.07.2023 CN 202310820552; 28.07.2023 CN 202310949410; 02.08.2023 CN 202310972687
(71) Applicant: Shenzhen Salubris Pharmaceuticals Co., Ltd., Shenzhen, Guangdong 518017 (CN)
(72) Inventor: WU, Junjun, Shenzhen, Guangdong 518017 (CN); LU, Yinsuo, Shenzhen, Guangdong 518017 (CN); ZOU, Peng, Shenzhen, Guangdong 518017 (CN); XIAO, Ying, Shenzhen, Guangdong 518017 (CN); SONG, Qing, Shenzhen, Guangdong 518017 (CN); ZENG, Junnan, Shenzhen, Guangdong 518017 (CN); DING, Xiaohong, Shenzhen, Guangdong 518017 (CN)
(74) Representative: Vitillo, Giuseppe
(86) International application number: PCT/CN2024/093784
(87) International publication number: WO 2024/240056

(57) **Abstract**

The present invention belongs to the technical field of chemical pharmaceuticals and provides a sulfonamide compound (I), a preparation method therefor, and pharmaceutical use thereof.

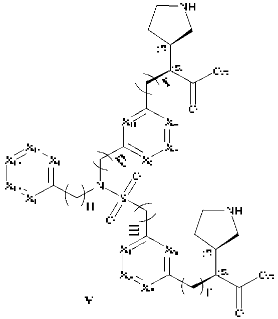

## Description

### TECHNICAL FIELD

The present application belongs to the technical field of chemical pharmaceuticals and provides a sulfonamide compound, a preparation method therefor, and pharmaceutical use thereof.

### BACKGROUND

LPA is the name of a gene encoding apolipoprotein (a) (apo(a)), the gene is mainly expressed in the liver, and its expression is limited to humans and non-primates. Apolipoprotein (a) is attached to apo(B)-100 through disulfide bonds and combines with a lipid core into lipoprotein (a) (Lp(a)) particles. The Lp(a) particles are special large molecule lipoprotein rich in cholesterol, with a surface wrapped in cholesterol and phospholipids, and embedded with hydrophilic apolipoprotein components apolipoprotein (a) and apo(B)-100. Lp(a) can enter and deposit on a vascular wall, which can promote atherosclerosis. The Lp(a) is structurally homologous to plasminogen (PLG) and can compete with the plasminogen to bind to fibrin sites, thereby inhibiting fibrinogen hydrolysis and promoting thrombosis. Therefore, the LP(a) is closely related to atherosclerosis and thrombosis. Studies have shown that a level of Lp(a) in blood is an independent risk factor for cardiovascular disease, stroke and atherosclerotic stenosis. The level of Lp(a) in humans is determined by genes and does not significantly change with changes in diet, exercise, or other lifestyles.

Currently reported LP(a) inhibitors are mainly large molecule or small nucleic acid drugs, for example: WO2023046093A1 discloses a bispecific fusion polypeptide; CN111465694A discloses a nucleic acid for inhibiting the expression of LPA in cells; CN108368506A also discloses a composition and method for inhibiting gene expression of LPA; and CN113166759A discloses a chemically modified RNAi construct and its use. At present, there are no small molecule LP(a) drugs on the market. Among the projects under research, only the patent CN114008021A reports a pyrrolidine compound as a small molecule LP(a) drug. Therefore, there is an urgent need to provide more small molecule LP(a) drugs.

### SUMMARY

In view of the problems in the prior art, the present application provides a small molecule LP(a) drug to solve the problem of lack of small molecule LP(a) drugs in the prior art.

The present application is implemented through the following technical solution:
The present application provides a sulfonamide compound, or an isomer thereof, or a racemate thereof, or a pharmaceutic salt thereof, wherein a structure of the sulfonamide compound is as shown in general formula I: 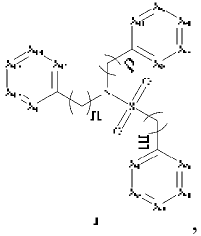
wherein X₁-X₁₅ are each independently selected from the group consisting of -N- and -CR₁, R₁ is selected from the group consisting of H, halogen, -OH, -NH₂, -CN, -NO₂, -COOH, -SO₃H, substituted or unsubstituted C₁-C₆ alkyl, substituted or unsubstituted C₁-C₆ alkoxy, substituted or unsubstituted C₃-C₁₀ cycloalkyl, substituted or unsubstituted 3-10-membered heterocycloalkyl, substituted or unsubstituted C₆-C₁₀ aryl, substituted or unsubstituted 5-10-membered heteroaryl, and 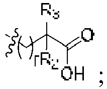
at least one of X₁-X₁₅ is -N-; or
when X₁-X₁₅ are each independently selected from -CR₁, wherein at least one R₁ is not hydrogen, at least one R₁ in X₁-X₅ is independently selected from 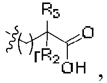 and at least one R₁ in X₆-X₁₀ is independently selected from 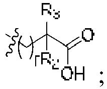 or
when X₁-X₁₅ are each independently selected from -CR₁, any two adjacent R₁ in X₁-X₁₅, together with connected carbon atoms, form substituted or unsubstituted C₆-C₁₀ aryl or 5-10-membered heteroaryl;
R₂ is selected from the group consisting of -H, halogen, substituted or unsubstituted C₁-C₆ alkyl, and substituted or unsubstituted C₁-C₆ alkoxy;
R₃ is selected from -(CH₂)_{q}-(5-8-membered heterocyclyl);
substituents in substituted C₁-C₆ alkyl, substituted C₁-C₆ alkoxy, substituted C₃-C₁₀ cycloalkyl, substituted 3-10-membered heterocycloalkyl, substituted C₆-C₁₀ aryl or substituted 5-10-membered heteroaryl are independently selected from the group consisting of halogen, hydroxyl, carboxyl, nitro, cyano, C₁-C₆ alkyl, C₁-C₆ halogenated alkyl, C₁-C₆ halogenated alkoxy, C₁-C₆ alkoxy, C₃-C₆ cycloalkyl, 3-6-membered heterocycloalkyl, C₃-C₆ cycloalkoxy, C₆-C₁₀ aryl, 5-10-membered heteroaryl, and a combination thereof; and
n, p and r are integers independently selected from 1, 2 or 3, and m and q are integers selected from 0, 1, 2 or 3.

As a preferred technical solution of the present application, R₁ is selected from the group consisting of H, halogen, -OH, -NH₂, -CN, -NO₂, -COOH, C₁-C₆ alkyl, C₁-C₆ halogenated alkyl, C₁-C₆ alkoxy, C₁-C₆ halogenated alkoxy, substituted or unsubstituted C₃-C₁₀ cycloalkyl, substituted or unsubstituted 3-10-membered heterocycloalkyl, substituted or unsubstituted C₆-C₁₀ aryl, substituted or unsubstituted 5-10-membered heteroaryl, and 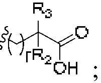 R₂ is selected from the group consisting of -H, and substituted or unsubstituted C₁-C₆ alkyl; R₃ is selected from 5-8-membered heterocyclyl; and substituents in substituted C₃-C₁₀ cycloalkyl, substituted 3-10-membered heterocycloalkyl, substituted C₆-C₁₀ aryl or substituted 5-10-membered heteroaryl are independently selected from the group consisting of halogen, hydroxyl, carboxyl, nitro, cyano, C₁-C₆ alkyl, C₁-C₆ halogenated alkyl, C₁-C₆ halogenated alkoxy, C₁-C₆ alkoxy, C₃-C₆ cycloalkyl, 3-6-membered heterocycloalkyl, C₃-C₆ cycloalkoxy, C₆-C₁₀ aryl, 5-10-membered heteroaryl, and a combination thereof.

As a preferred technical solution of the present application, 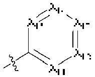 is selected from quinolyl; and 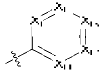 is further preferably

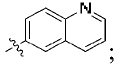

As a preferred technical solution of the present application, one of X₄ or X₅ is selected from 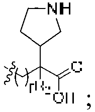 one of X₉ or X₁₀ is selected from 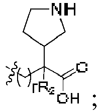 and one of X₁₁ or X₁₂ is selected from

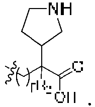

As a preferred technical solution of the present application, X₄ or X₅ is selected from the group consisting of 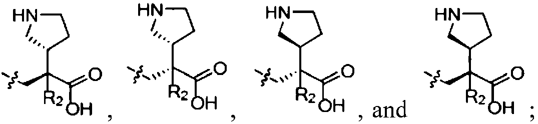 X₉ or X₁₀ is selected from the group consisting of 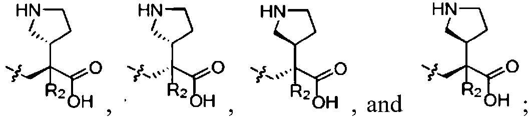 and X₁₁ or X₁₂ is selected from the group consisting of 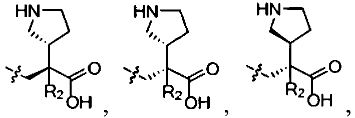 and

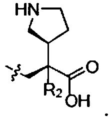

As a preferred technical solution of the present application, a structure of the sulfonamide compound is as shown in general formula II or III: 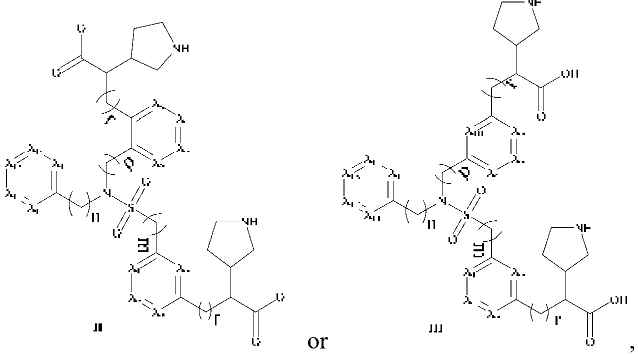 wherein X₁-X₁₅ are each independently selected from the group consisting of -N- and -CR₁, R₁ is selected from the group consisting of H, halogen, substituted or unsubstituted C₁-C₆ alkyl, substituted or unsubstituted C₁-C₆ alkoxy, and 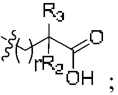 R₂ is selected from the group consisting of H and substituted or unsubstituted C₁-C₆ alkyl; R₃ is selected from 5-8-membered heterocyclyl; and substituents in substituted C₁-C₆ alkyl and substituted C₁-C₆ alkoxy are independently selected from the group consisting of halogen, hydroxyl, carboxyl, nitro, cyano, C₁-C₆ alkyl, C₁-C₆ halogenated alkyl, C₁-C₆ halogenated alkoxy, C₁-C₆ alkoxy, C₃-C₆ cycloalkyl, 3-6-membered heterocycloalkyl, C₃-C₆ cycloalkoxy, C₆-C₁₀ aryl, 5-10-membered heteroaryl, and a combination thereof.

As a preferred technical solution of the present application, a structure of the sulfonamide compound is as shown in general formula IV or V: 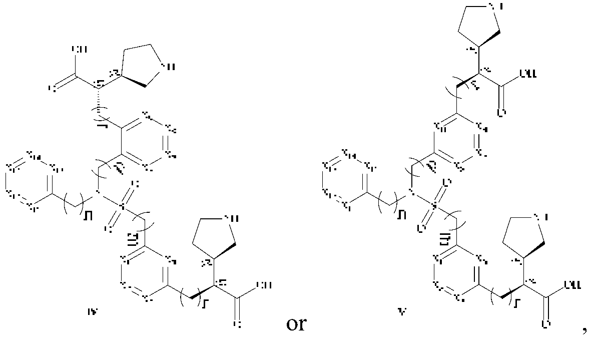 wherein X₁-X₁₅ are each independently selected from the group consisting of -N- and -CR₁, R₁ is selected from the group consisting of H, halogen, substituted or unsubstituted C₁-C₆ alkyl, substituted or unsubstituted C₁-C₆ alkoxy, and 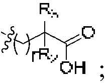 R₂ is selected from the group consisting of H and substituted or unsubstituted C₁-C₆ alkyl; R₃ is selected from 5-8-membered heterocyclyl; and substituents in substituted C₁-C₆ alkyl and substituted C₁-C₆ alkoxy are independently selected from the group consisting of halogen, hydroxyl, carboxyl, nitro, cyano, C₁-C₆ alkyl, C₁-C₆ halogenated alkyl, C₁-C₆ halogenated alkoxy, C₁-C₆ alkoxy, C₃-C₆ cycloalkyl, 3-6-membered heterocycloalkyl, C₃-C₆ cycloalkoxy, C₆-C₁₀ aryl, 5-10-membered heteroaryl, and a combination thereof.

As a preferred technical solution of the present application, any one of X₁₁, X₁₂, X₁₃, X₁₄ or X₁₅ is independently selected from -CR₁, and R₁ is selected from 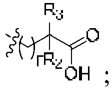 and preferably, R₁ is any one selected from the group consisting of

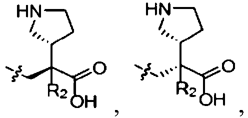

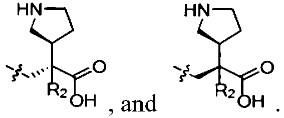

As a preferred technical solution of the present application, R₁ is selected from the group consisting of H, fluorine, chlorine, bromine, iodine, trifluoromethyl, methyl, ethyl, propyl, methoxy, ethoxy, piperazinyl, -CH₂COOH, -CH₂CH₂COOH, -CH₂CH(CH₃)COOH, 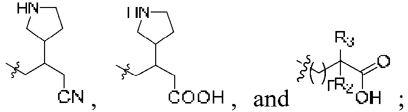 and R₃ is selected from pyrrolidyl, piperidyl, piperazinyl, morpholinyl, pyridyl, pyrimidinyl, and pyrazinyl.

As a preferred technical solution of the present application, 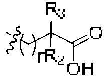 is selected from the group consisting of:

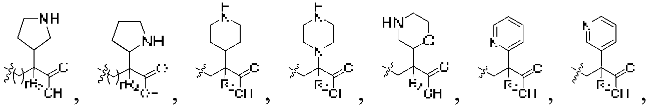

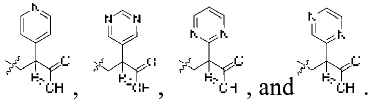

As a preferred technical solution of the present application, 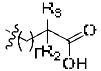 is selected from the group consisting of: 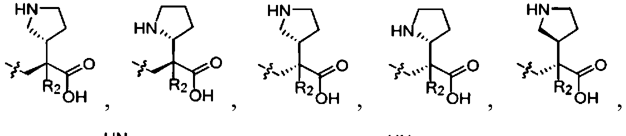 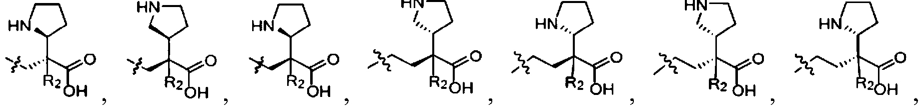 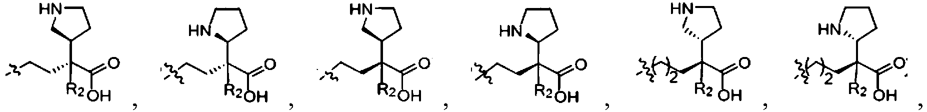 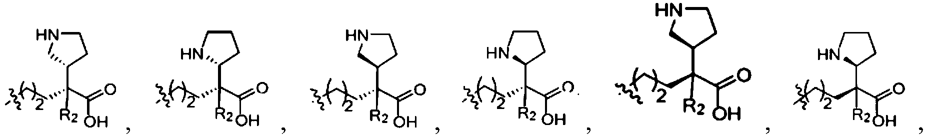 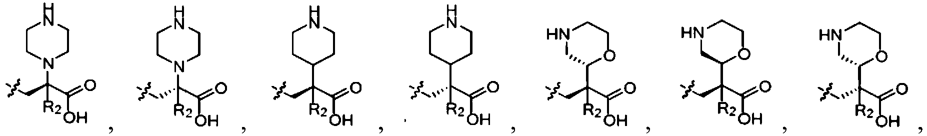 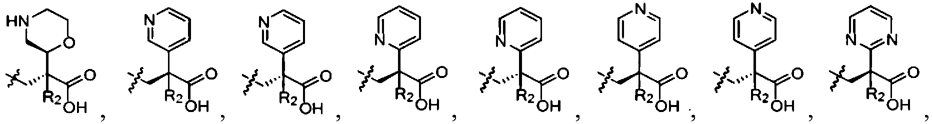 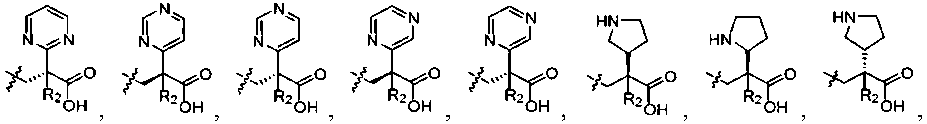 and

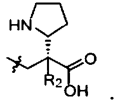

As a preferred technical solution of the present application, 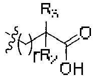 is selected from the group consisting of:

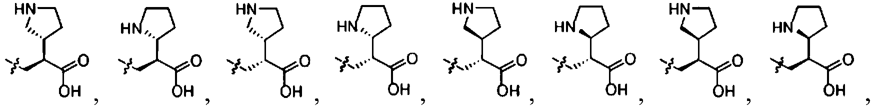

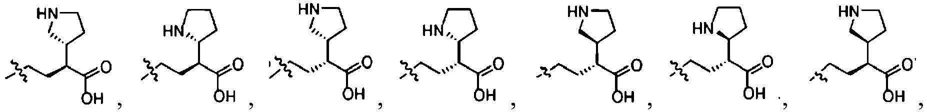

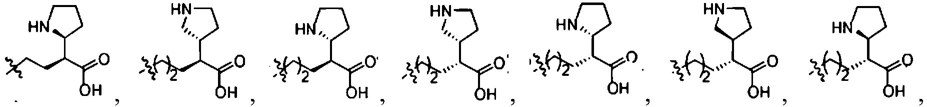

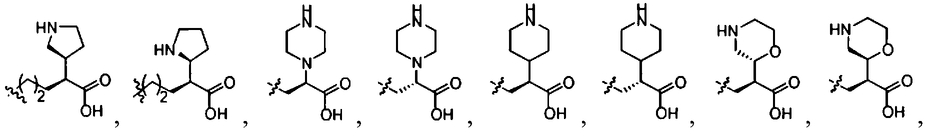

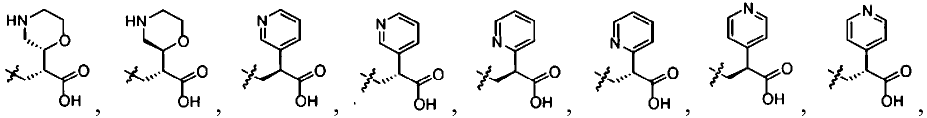

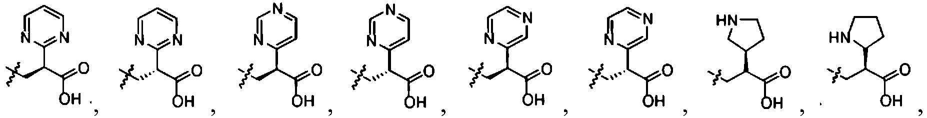

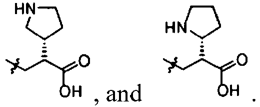

In the chemical structure of the compound in the present application, a bond " 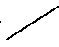 " denotes an unspecified configuration, that is, if there is a chiral isomer in the chemical structure, the bond " 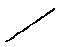 " may be " 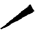 " or " 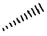 ", or simultaneously contains two configurations of " 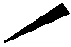 " and " 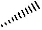 ".

In the chemical structure of the compound in the present application, a bond " 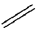 " does not specify a configuration, that is, it may be a Z configuration or an E configuration, or simultaneously contains two configurations.

The compound and intermediates in the present application may also exist in different tautomeric forms, and all such forms are within the scope of the present disclosure. The term "tautomer" or "tautomeric form" refers to structural isomers of different energies that can undergo interconversion via low energy barriers. For example, proton tautomers (also known as proton transfer tautomers) include interconversion via proton transfer, such as ketone-enol, imine-enamine, and lactam-lactim isomerization. An instance of lactam-lactim equilibrium is between A and B as shown below.

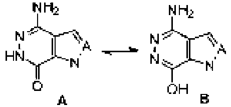

All compounds in the present application may be depicted as the type A or the type B. All tautomeric forms are within the scope of the present disclosure. The naming of the compounds does not exclude any tautomers.

As a preferred technical solution of the present application, C₁-C₆ alkyl is preferably C₁-C₂, C₁-C₃, C₁-C₄ or C₁-C₅ alkyl. Instances of the alkyl include: methyl, ethyl, propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neo-hexyl, 2-methylpentyl, 1,2-dimethylbutyl, and 1-ethylbutyl.

As a preferred technical solution of the present application, C₁-C₆ alkoxy is preferably C₁-C₂, C₁-C₃, C₁-C₄ or C₁-C₅ alkoxy, and further, the alkoxy is specifically selected from the group consisting of methoxy, ethoxy, propoxy, isopropoxy, n-butoxy, isobutoxy, sec-butoxy, and tert-butoxy.

As a preferred technical solution of the present application, C₃-C₁₀ cycloalkyl is preferably selected from the group consisting of: C₃-C₈ cycloalkyl, C₃-C₆ cycloalkyl, andC₃-C₅ cycloalkyl, preferably C₃-C₈ cycloalkyl, and the cycloalkyl is specifically selected from the group consisting of cyclopropyl, cyclobutyl, cyclopentyl, cyclohexyl, cycloheptyl, and cyclooctyl.

As a preferred technical solution of the present application, in some embodiments, "heterocyclyl" is 5-10-membered heterocyclyl consisting of 5-10 annular atoms. In other embodiments, "heterocyclyl" is 5-8-membered heterocyclyl consisting of 5-8 annular atoms. In other embodiments, "heterocyclyl" is 6-8-membered heterocyclyl consisting of 6-8 annular atoms. In other embodiments, "heterocyclyl" is 5-6-membered heterocyclyl consisting of 5-6 annular atoms. Instances of the heterocyclyl include, but are not limited to: pyranyl, tetrahydropyranyl, oxetanyl, tetrahydrofuranyl, dihydrofuranyl, 1,4-dioxanyl, morpholinyl, 1,4-dithianyl, piperazinyl, piperidyl, 1,3-dioxolanyl, imidazolidinyl, imidazolinyl, pyrrolinyl, pyrrolidinyl, tetrahydropyranyl, dihydropyranyl, oxathiolanyl, dithiolanyl, 1,3-dioxanyl, 1,4-dioxanyl, 1,3-dithianyl, oxathianyl, thiomorpholinyl, tetrahydro-thiapyran-1,1-dioxide, and 1,4-diazepanyl.

As a preferred technical solution of the present application, in some embodiments, "heterocycloalkyl" is preferably 3-12-membered heterocycloalkyl, more preferably 3-10-membered heterocycloalkyl, more preferably 5-8-membered heterocycloalkyl, and most preferably 5-6-membered heterocycloalkyl. Instances of the heterocycloalkyl include, but are not limited to: aziridinyl, oxiranyl, azetidinyl, oxetanyl, pyrrolidyl, tetrahydrofuranyl, tetrahydro-thienyl, pyrazolidyl, imidazolidinyl, oxazolidinyl, isooxazolidinyl, thiazolidyl, piperidyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, oxazepanyl, thiazanyl, 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3.1]nonyl, 3-thia-9-aza-bicyclo[3.3.1]nonyl, and 2,6-diaza-spiro[3.3]heptyl. More particular instances of the heterocycloalkyl are pyrrolidyl, pyrazolidyl, imidazolidinyl, oxazolidinyl, isooxazolidinyl, thiazolidyl, piperidyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, oxazepanyl, thiazanyl, and 2,6-diaza-spiro[3.3]heptyl.

As a preferred solution of the present application, substituted or unsubstituted C₆-C₁₀ aryl is preferably substituted or unsubstituted C₆-C₈ aryl, and substituted or unsubstituted C₆-C₇ aryl. When the aryl is substituted, substituents are preferably selected from the group consisting of H, halogen, -OH, -NH₂, -CN, -NO₂, -COOH, -SO₃H, C₁-C₆ alkyl, C₁-C₆ halogenated alkyl, C₁-C₆ alkoxy, C₁-C₆ halogenated alkoxy, C₃-C₁₀ cycloalkyl, C₆-C₁₀ substituted or unsubstituted aryl, and C₅-C₁₀ substituted or unsubstituted heteroaryl, and the aryl is specifically selected from the group consisting of phenyl, naphthyl, anthryl, and phenanthryl, and the like.

Substituted or unsubstituted 5-10-membered heteroaryl is preferably substituted or unsubstituted 5-8-membered heteroaryl, substituted or unsubstituted 5-7-membered heteroaryl or substituted or unsubstituted 5-6-membered heteroaryl. When the heteroaryl is substituted, substituents are preferably selected from the group consisting of H, halogen, -OH, -NH₂, -CN, -NO₂, -COOH, -SO₃H, C₁-C₆ alkyl, C₁-C₆ halogenated alkyl, C₁-C₆ alkoxy, C₁-C₆ halogenated alkoxy, C₃-C₁₀ cycloalkyl, substituted or unsubstituted C₅-C₁₀ aryl, and substituted or unsubstituted 5-10-membered heteroaryl, and heteroatoms in the heteroaryl are one or more of N, O and S. The heteroaryl is preferably selected from the group consisting of: imidazolyl, furyl, thienyl, thiazolyl, pyrazolyl, oxazolyl, pyrryl, tetrazyl, pyridyl, pyrimidinyl, thiadiazole, pyrazine, 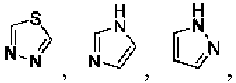 pyrazolyl, isoxazolyl, thiazolyl, pyrazolyl, tetrazyl, pyridazinyl, quinolyl, isoquinolyl, triazolyl, and tetrazyl, and the like.

As a preferred technical solution of the present application, the sulfonamide compound, or the isomer thereof, or the racemate thereof, or the pharmaceutic salt thereof is selected from the group consisting of compounds shown in Table 1A or Table 1B.

**Table 1A**

| No. | Compound | No. | Compound | No. | Compound |
|---|---|---|---|---|---|
| A1 | 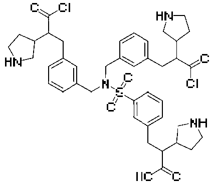 | A2 | 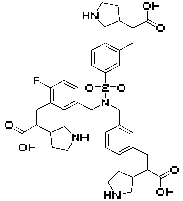 | A3 | 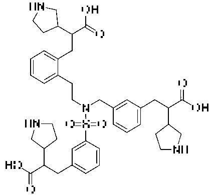 |
| A4 | 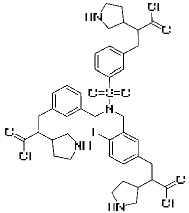 | A5 | 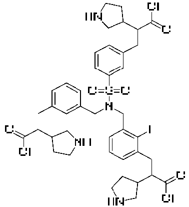 | A6 | 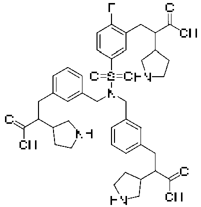 |
| A7 | 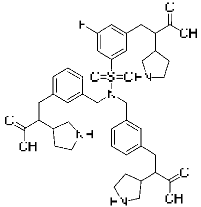 | A8 | 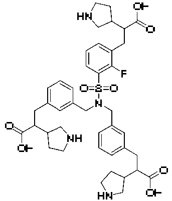 | A9 | 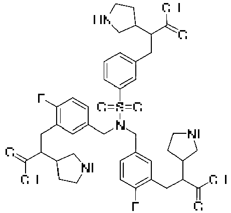 |
| A1 0 | 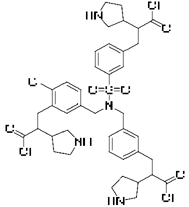 | A11 | 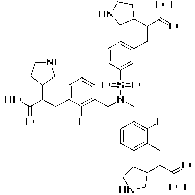 | A1 2 | 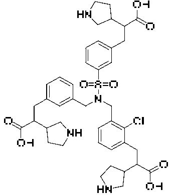 |
| A1 3 | 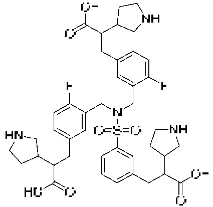 | A1 4 | 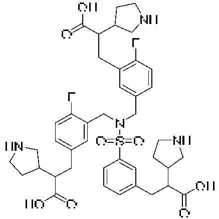 | A1 5 | 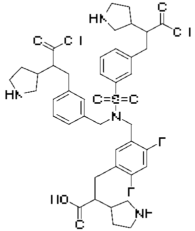 |
| A1 6 | 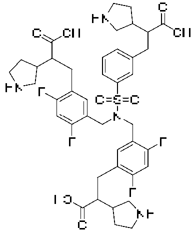 | A1 7 | 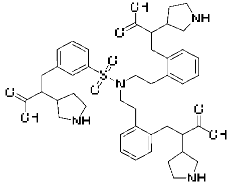 | A1 8 | 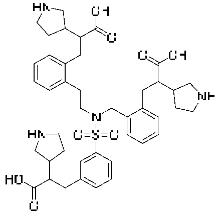 |
| A1 9 | 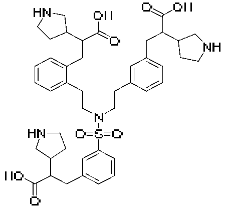 | A2 0 | 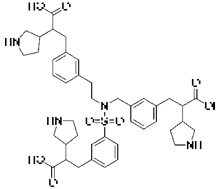 | A2 1 | 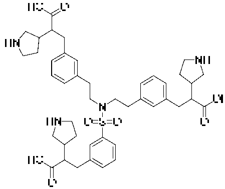 |
| A2 2 | 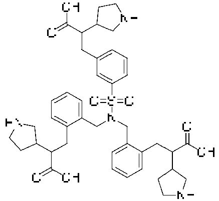 | A2 3 | 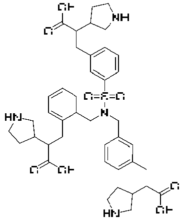 | A2 4 | 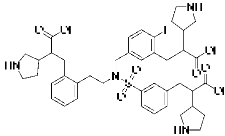 |
| A2 5 | 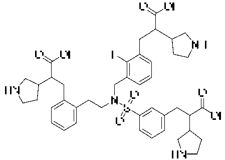 | A2 6 | 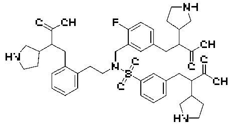 | A2 7 | 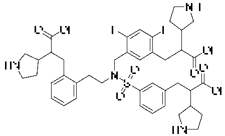 |
| A2 8 | 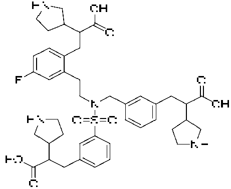 | A2 9 | 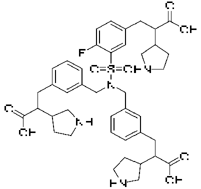 | A3 0 | 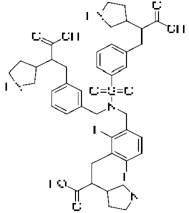 |
| A3 1 | 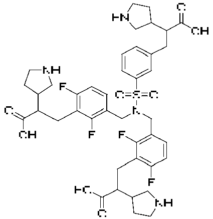 | A3 2 | 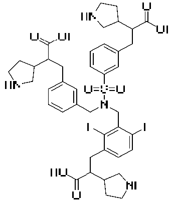 | A3 3 | 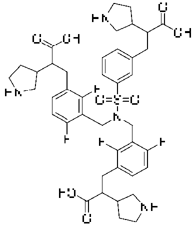 |
| A3 4 | 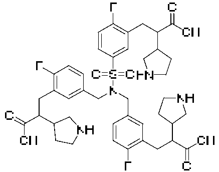 | A3 5 | 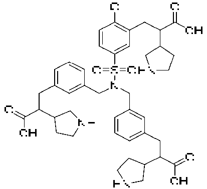 | A3 6 | 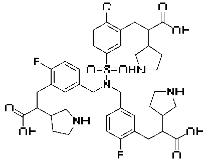 |
| A3 7 | 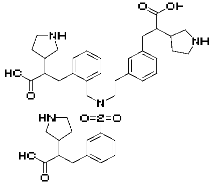 | A3 8 | 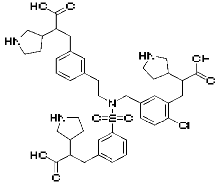 | A3 9 | 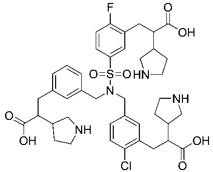 |
| A4 0 | 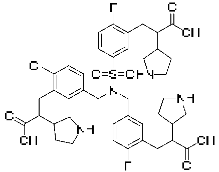 | A4 1 | 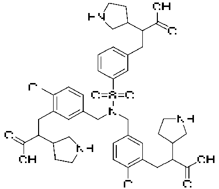 | A4 2 | 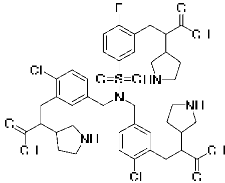 |
| A4 3 | 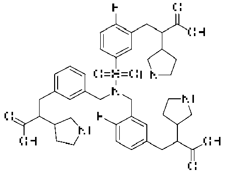 | A4 4 | 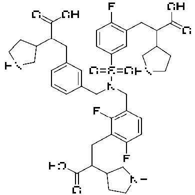 | A4 5 | 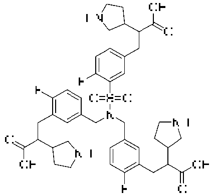 |
| A4 6 | 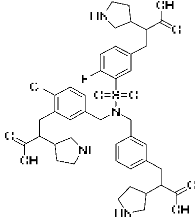 | A4 7 | 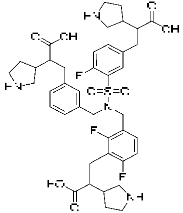 | | |

**Table 1B**

| No. | Compound | No. | Compound | No. | Compound |
|---|---|---|---|---|---|
| 1 | 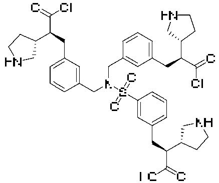 | 2 | 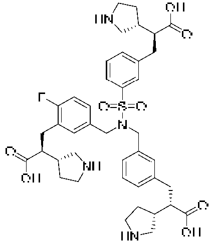 | 3 | 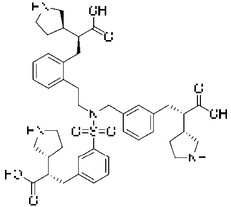 |
| 4 | 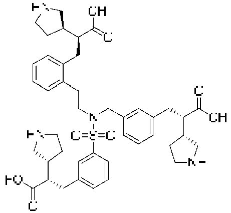 | 5 | 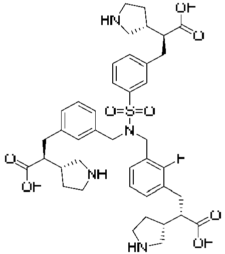 | 6 | 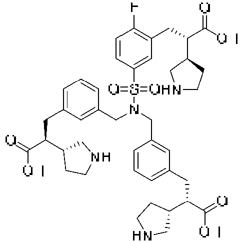 |
| 7 | 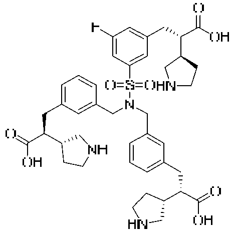 | 8 | 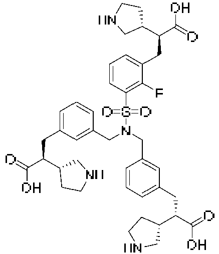 | 9 | 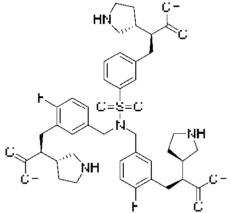 |
| 10 | 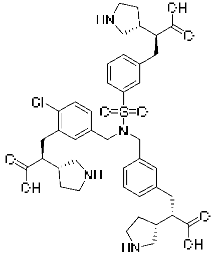 | 11 | 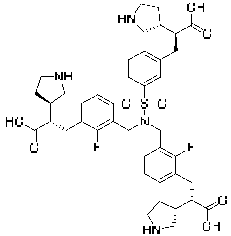 | 12 | 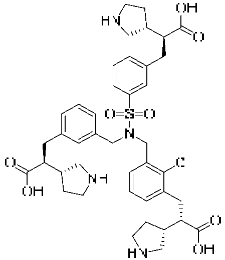 |
| 13 | 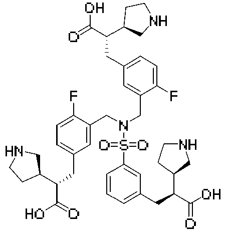 | 14 | 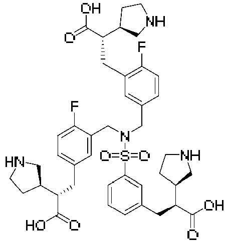 | 15 | 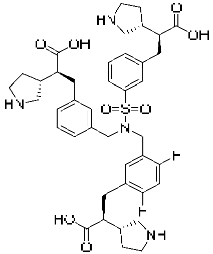 |
| 16 | 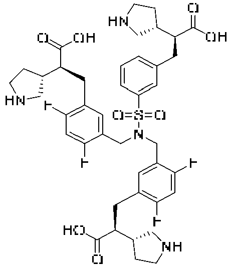 | 17 | 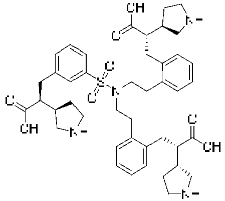 | 18 | 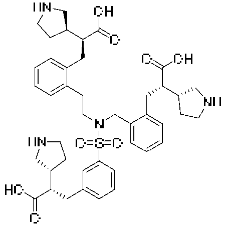 |
| 19 | 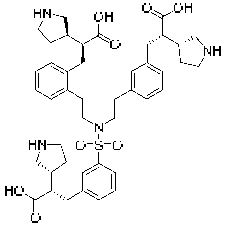 | 20 | 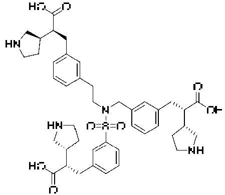 | 21 | 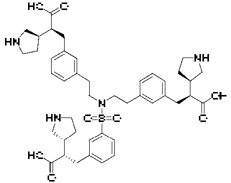 |
| 22 | 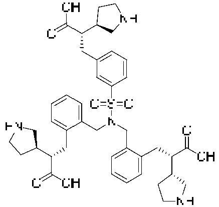 | 23 | 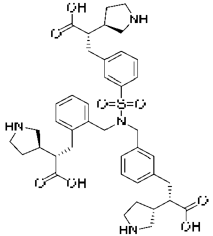 | 24 | 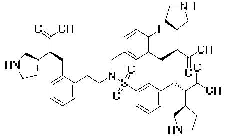 |
| 25 | 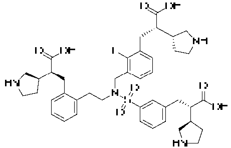 | 26 | 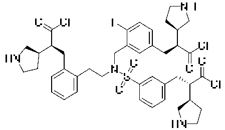 | 27 | 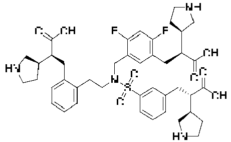 |
| 28 | 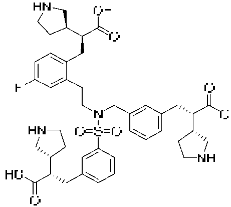 | 29 | 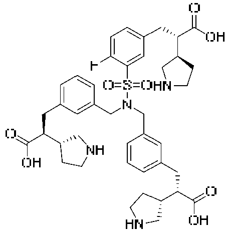 | 30 | 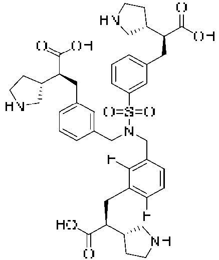 |
| 31 | 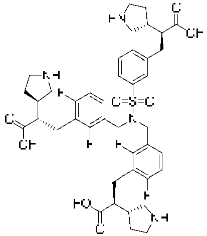 | 32 | 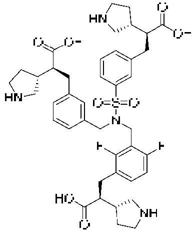 | 33 | 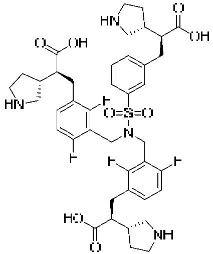 |
| 34 | 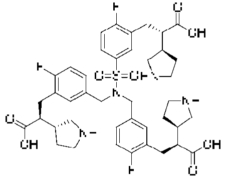 | 35 | 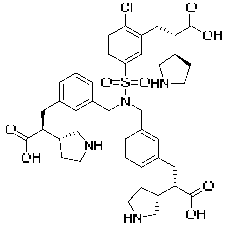 | 36 | 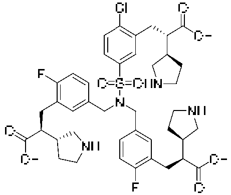 |
| 37 | 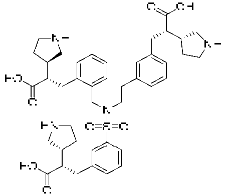 | 38 | 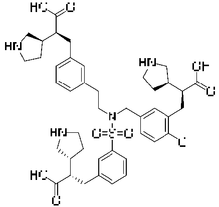 | 39 | 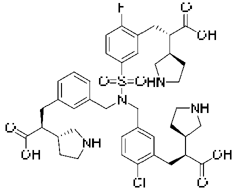 |
| 40 | 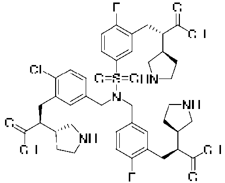 | 41 | 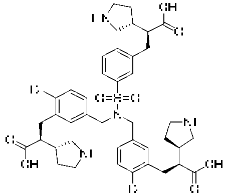 | 42 | 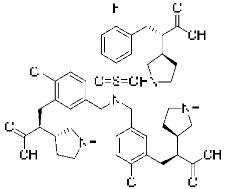 |
| 43 | 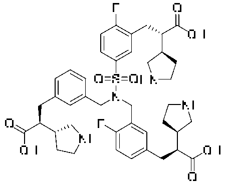 | 44 | 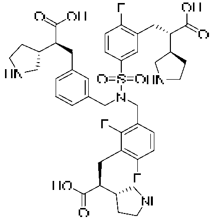 | 45 | 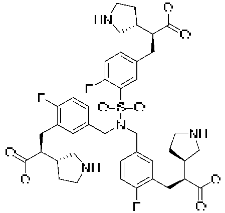 |
| 46 | 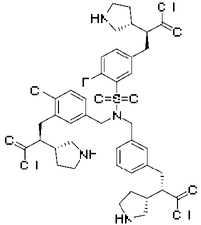 | 47 | 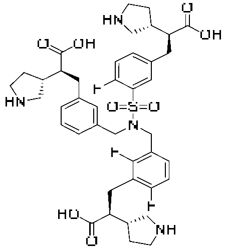 | | |

The present application further provides a preparation method for the sulfonamide compound, or the isomer thereof, or the racemate thereof, or the pharmaceutic salt thereof, and preparation is carried out with reference to a method in the patent CN114008021A and methods known in the art.

The present application further provides a pharmaceutical composition, wherein the pharmaceutical composition includes the sulfonamide compound, or the isomer thereof, or the racemate thereof, or the pharmaceutic salt thereof in general formula I, general formula II, general formula III, general formula IV or general formula V, and one or more pharmaceutically acceptable excipients and/or carriers.

The present application further provides an application of a sulfonamide compound, or an isomer thereof, or a racemate thereof, or a pharmaceutic salt thereof in preparation of medicines for preventing or treating a LP(a)-related disease.

Further, the Lp(a)-related disease is selected from a cardiovascular disease.

Further, the cardiovascular disease is selected from the group consisting of stroke, atherosclerosis, thrombosis, coronary heart disease, aortic stenosis, and any other disease related to an elevated Lp(a) level.

Compared with the prior art, the present application includes, but is not limited to, the following beneficial effects:
compared with the prior art, the sulfonamide compound in the present application has a lower IC₉₀ value.

For clarity, general terms used in the description of the compounds are defined herein.

Unless otherwise stated, the following terms and phrases used herein are intended to have the following meanings. A specific term or phrase should not be considered uncertain or unclear without a specific definition, but should be understood according to the common meanings. When a trade name appears herein, it is intended to refer to its corresponding commodity or its active ingredient. The term "pharmaceutically acceptable" used here refers to compounds, materials, compositions, and/or dosage forms that are suitable for use in contact with human and animal tissue within the scope of reliable medical judgment, without excessive toxicity, irritation, allergic reactions, or other issues or complications, and are commensurate with a reasonable benefit/risk ratio.

The term "pharmaceutic salt" refers to a salt of the compound in the present application, and is prepared from compounds with specific substituents discovered in the present application and a pharmaceutic acid or base.

In addition to the salt form, the compound provided by the present application also has a prodrug form. A prodrug of the compound described herein easily undergoes chemical changes under physiological conditions, thereby transforming into the compound in the present application. In addition, the prodrug may be converted into the compound in the present application through chemical or biochemical methods in the in vivo environment.

Some compounds in the present application may exist in a non-solvated or solvated form, including a hydrate form. Generally speaking, both the solvated and non-solvated forms are equivalent and are within the scope of the present application.

The compound in the present application may have specific geometric or stereoisomeric forms. The present application envisions all such compounds, including cis and trans isomers, (-)- and (+)- enantiomers, (R)- and (S)- enantiomers, diastereomers, (D)-isomers, (L)- isomers, as well as racemic mixtures thereof and other mixtures, such as enantiomer or diastereomer enriched mixtures, all of which are within the scope of the present application. There may be additional asymmetric carbon atoms in alkyl and other substituents. All of these isomers and their mixtures are included within the scope of the present application.

Optically active (R)- and (S)- isomers, as well as D and L isomers, may be prepared through chiral synthesis or chiral reagents or other conventional techniques. If one wishes to obtain an enantiomer of a certain compound in the present application, it may be prepared by asymmetric synthesis or by derivatization with chiral adjuvants, wherein a resulting diastereomer mixture is separated, and an auxiliary group is cleaved to provide the desired pure enantiomer. Alternatively, when molecules contain alkaline functional groups (such as amino) or acidic functional groups (such as carboxyl), salts of diastereomers are formed with appropriate optically active acids or bases, and then the diastereomers are split using conventional methods known in the art, followed by recovery to obtain the pure enantiomer. In addition, the separation of enantiomers and diastereomers is typically achieved through the use of chromatography, which employs chiral stationary phases and optionally combines with chemical derivatization methods (such as generating carbamate from amine).

"Alkyl" refers to saturated aliphatic hydrocarbon groups, including linear and branched groups with 1 to 20 carbon atoms. Preferred is alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, and various branched isomers thereof. Alkyl may be substituted or unsubstituted, when the alkyl is substituted, substituents may be substituted on any available link point, and the substituents are preferably one or more of the following groups, which are independently selected from the group consisting of halogen, deuterium, hydroxyl, oxo, nitro, cyano, C₁-C₆ alkyl, C₁₋C₆ alkoxy, C₂-C₆ alkenyloxy, C₂-C₆ alkynyloxy, C₃-C₆ cycloalkyl, 3-6-membered heterocycloalkyl, C₅-C₈ cycloalkenyl, C₃-C₆ cycloalkoxy, 3-6-membered heterocycloalkoxy, C₅-C₈ cycloalkenyloxy, C₆-C₁₀ aryl, and5-6-membered heteroaryl. C₁-C₆ alkyl, C₁-C₆ alkoxy, C₂-C₆ alkenyloxy, C₂-C₆ alkynyloxy, C₃-C₆ cycloalkyl, 3-6-membered heterocycloalkyl, C₅-C₈ cycloalkenyl, C₃-C₆ cycloalkoxy, 3-6-membered heterocycloalkoxy, C₅-C₈ cycloalkenyloxy, C₆-C₁₀ aryl or 5-6-membered heteroaryl is optionally substituted by one or more of halogen, deuterium, hydroxyl, oxo, nitro and cyano.

The term "alkoxy" refers to -O-(alkyl) and -O-(unsubstituted cycloalkyl), wherein the definition of alkyl is as described above. Non-limiting instances of alkoxy include: methoxy, ethoxy, propoxy, butoxy, cyclopropoxy, cyclobutoxy, cyclopentyloxy, and cyclohexoxy.

The term "halogenated alkoxy" refers to alkoxy, wherein at least one of hydrogen atoms in the alkoxy has been substituted by the same or different halogen atoms. The term "fully halogenated alkoxy" refers to alkoxy in which all hydrogen atoms of the alkoxy have been substituted by the same or different halogen atoms. Instances of the halogenated alkoxy include fluoromethoxy, difluoromethoxy, trifluoromethoxy, trifluoroethoxy, trifluoromethylethoxy, trifluorodimethylethoxy, and pentafluoroethoxy. Particular halogenated alkoxy is trifluoromethoxy and 2,2-difluoroethoxy.

The term "halogen" refers to fluorine, chlorine, bromine, or iodine.

The term "cycloalkyl" or "carboatomic ring" refers to a saturated monocyclic or polycyclic cyclic hydrocarbon substituent, wherein a cycloalkyl ring contains 3 to 10 carbon atoms, preferably 3 to 8 carbon atoms, more preferably 3 to 6 carbon atoms. Non-limiting instances of monocyclic cycloalkyl include cyclopropyl, cyclobutyl, cyclopentyl, cyclohexyl, etc.; and polycyclic cycloalkyl includes spirocyclic, fused cyclic, and bridged cyclic cycloalkyl.

The term "aryl" or "aromatic ring" refers to a 6-10-membered all-carbon monocyclic or fused polycyclic (i.e., rings sharing adjacent carbon atom pairs) group with a conjugated π electron system, preferably being 6-8-membered, such as phenyl and naphthyl.

The term "heteroaryl" or "heteroaromatic ring" refers to a heteroaromatic system containing 1 to 3 heteroatoms and 5 to 12 annular atoms, wherein the heteroatoms are selected from the group consisting of oxygen, sulfur, and nitrogen. The heteroaryl is preferably 5-10-membered and 5-8-membered heteroaryl, more preferably, 5- or 6-membered heteroaryl. Pyrryl, furyl, thienyl, imidazolyl, oxazolyl, thiazolyl, triazolyl, oxadiazolyl, thiadiazolyl, tetrazyl, pyridyl, pyrazinyl, pyrazolyl, pyridazinyl, pyrimidyl, triazinyl, azepinyl, diazepinyl, isoxazolyl, benzofuryl, isothiazolyl, benzothiazolyl, indyl, isoindyl, isobenzofuryl, benzimidazolyl, benzoxazolyl, benzoisoxazolyl, benzothiazolyl, benzoisothiazolyl, benzoxadiazolyl, benzothiadiazolyl, benzotriazolyl, purinyl, quinolyl, isoquinolyl, quinazolinyl and quinoxalyl. Particular heteroaryl includes pyrryl, furyl, thienyl, imidazolyl, oxazolyl, thiazolyl, triazolyl, oxadiazolyl, thiadiazolyl, tetrazyl, pyridyl, pyrazinyl, pyrazolyl, pyridazinyl, pyrimidyl, isoxazolyl and isothiazolyl. More particular heteroaryl includes imidazolyl, oxazolyl, thiazolyl, oxadiazolyl, thiadiazolyl, tetrazyl, pyridyl, pyrazinyl, pyrazolyl, pyridazinyl, pyrimidyl, isoxazolyl, isothiazolyl, 2-fluoropyridyl, 3-fluoropyridyl, 4-fluoropyridyl, 2-chloropyridyl, 3-chloropyridyl, 4-chloropyridyl, 2,3-difluoropyridyl, 3,4-difluoropyridyl, 4,5-difluoropyridyl, 3,5-difluoropyridyl, 2-chloro-3-fluoro-pyridyl, 3-fluoro-4-chloro-pyridyl, 3-fluoro-5-chloro-pyridyl, 4-fluoropyrimidyl, 5-fluoropyrimidyl, 4-chloropyrimidyl, 5-chloropyrimidyl, 4-chloro-5-fluoropyrimidyl, 4-cyanopyrimidyl, 5-cyanopyrimidyl, 4,5-dicyanopyrimidyl, 4-methylpyrimidyl, 5-methylpyrimidyl or 4,5-dimethylpyrimidyl.

The term "heterocyclyl" refers to a saturated or partially unsaturated monocyclic heterocyclic ring (i.e., monocyclic heterocyclyl), polycyclic heterocyclic system (i.e., polycyclic heterocyclyl), or heteroaromatic ring system, which contains at least one (e.g., 1, 2, 3, or 4) heteroatom selected from the group consisting of nitrogen, oxygen, and sulfur in its ring (said nitrogen may be optionally oxidized to form nitrogen oxides; and said sulfur may be optionally oxygenated to form sulfoxide or sulfone, but does not include -O-O-, -O-S-, or -S-S-), and has 3 to 20 (e.g. 3, 4, 5, 6, 7, 8, 9, 10, 11, 12, 13, 14, 15, 16, 17, 18, 19, or 20) annular atoms (i.e., 3-20-membered heterocyclyl). The heterocyclyl is preferably heterocyclyl with 3 to 12 annular atoms (i.e., 3-12-membered heterocyclyl); further preferably, heterocyclyl with 3 to 8 annular atoms (i.e., 3-8-membered heterocyclyl); more preferably, heterocyclyl with 5 to 8 annular atoms (i.e., 5-8-membered heterocyclyl); and most preferably, heterocyclyl with 5 to 6 annular atoms (i.e., 5-6-membered heterocyclyl). In some examples, the 5-8-membered heterocyclyl is selected from the group consisting of: 5-8-membered heterocycloalkyl, and 5-8-membered heteroaryl, and in some examples, the 5-6-membered heterocyclyl is selected from the group consisting of: 5-6-membered heterocycloalkyl, and 5-6-membered heteroaryl.

The term "heterocycloalkyl" refers to cycloalkyl with carbon atoms thereon substituted by 1-4 heteroatoms, wherein the heteroatoms are selected from the group consisting of N, O, S, and a combination thereof; preferably 3-12-membered heterocycloalkyl, more preferably 3-10-membered heterocycloalkyl, more preferably 5-8-membered heterocycloalkyl, and most preferably 5-6-membered heterocycloalkyl. Instances of the heterocycloalkyl include, but are not limited to: aziridinyl, oxiranyl, azetidinyl, oxetanyl, pyrrolidyl, tetrahydrofuranyl, tetrahydro-thienyl, pyrazolidyl, imidazolidinyl, oxazolidinyl, isooxazolidinyl, thiazolidyl, piperidyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, oxazepanyl, thiazanyl, 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3.1]nonyl, 3-thia-9-aza-bicyclo[3.3.1]nonyl, and 2,6-diaza-spiro[3.3]heptyl. More particular instances of the heterocycloalkyl are pyrrolidyl, pyrazolidyl, imidazolidinyl, oxazolidinyl, isooxazolidinyl, thiazolidyl, piperidyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, oxazepanyl, thiazanyl, and 2,6-diaza-spiro[3.3]heptyl.

Atoms of molecules of the compound in the present application are isotopes, and isotope derivatization may generally be used to prolong a half-life, reduce a clearance rate, stabilize metabolism, increase in-vivo activity and achieve other effects. An embodiment is included, in which at least one atom is substituted by an atom with the same atomic number (proton number) and different mass numbers (sum of protons and neutrons). Instances of isotopes included in the compound in the present application include hydrogen atoms, carbon atoms, nitrogen atoms, oxygen atoms, phosphorus atoms, sulfur atoms, fluorine atoms and chlorine atoms, which include ²H, ³H, ¹³C, ¹⁴C, ¹⁵N, ¹⁷O, ¹⁸O, ³¹P, ³²P, ³⁵S, ¹⁸F, and ³⁶Cl respectively. Particularly, radioactive isotopes such as ³H or ¹⁴C that emit radiation as they decay may be used for local anatomical examination of drug formulations or in-vivo compounds. Stable isotopes do not decay or change with their quantity, nor do they have radioactivity, so they can be safely used. When the atoms constituting the molecules of the compound in the present application are isotopes, the isotopes may be converted according to a general method by replacing reagents used in synthesis with reagents containing corresponding isotopes.

The term "halogenated alkyl" refers to alkyl with one or more hydrogen atoms substituted by halogen, such as trifluoromethyl, and trifluoroethyl.

The term "halogenated alkoxy" refers to alkoxy with one or more hydrogen atoms substituted by halogen, such as trifluoromethoxy, and trifluoroethoxy.

The compound in the present application may contain non-natural proportions of atomic isotopes on one or more atoms constituting the compound. For example, the compound may be labeled with radioactive isotopes such as deuterium (²H), iodine-125 (¹²⁵I), or C-14 (¹⁴C). Transformations of all isotope compositions of the compound in the present application, whether radioactive or not, are included within the scope of the present application.

Further, the compound in the present application has one or more hydrogen atoms substituted by the isotope deuterium (²H). After deuteration, the compound in the present application has the effects such as a prolonged half-life, a reduced clearance rate, stable metabolism, and increased in-vivo activity.

A preparation method for isotopic derivatives usually includes: a phase-transfer catalysis method. For example, a preferred deuteration method uses a phase-transfer catalyst (such as tetraalkylammonium salts, NBu₄HSO₄). Using the phase-transfer catalyst to exchange methylene protons of diphenylmethane compounds results in the introduction of higher deuterium levels compared to using deuterated silane (such as triethyldeuterated silane) in the presence of acids (such as methanesulfonic acid) or using Lewis acids such as aluminum trichloride to reduce with deuterated sodium borate.

The term "pharmaceutically acceptable carrier" refers to any formulation carrier or medium capable of delivering effective amounts of active substance of the present application, without interfering with the biological activity of the active substance, and without toxic and side effects to hosts or patients. Representative carriers include water, oil, vegetables and minerals, cream bases, lotion bases, ointment bases, etc. These bases include suspension agents, thickening agents, transdermal enhancers, etc. Their formulations are well known to those skilled in the art of cosmetics or topical drugs. For other information about the carrier, please refer to Remington: The Science and Practice of Pharmacy, 21st Ed., Lippincott, Williams & Wilkins (2005), the content of this literature being incorporated herein by reference.

The term "excipient" typically refers to a carrier, diluent, and/or medium required for the formulation of an effective pharmaceutical composition.

For drugs or pharmacologically active agents, the term "effective amount" or "therapeutic effective amount" refers to a sufficient amount of non-toxic drugs or agents that can achieve the expected effect. For an oral dosage form in the present application, the "effective amount" of one active substance in the composition refers to an amount required to achieve the expected effect when combined with another active substance in the composition. The determination of the effective amount varies from person to person, depending on the age and general situation of a receptor, as well as the specific active substance. The appropriate effective amount in an individual case can be determined by those skilled in the art according to routine experiments.

The term "active ingredient", "therapeutic agent", "active substance" or "active agent" refers to a chemical entity that can effectively treat target disorders, diseases or conditions.

"Optional" or "optionally" refers to the possibility, but not necessarily, of a subsequent event or situation being described, and the description includes the circumstances in which the event or situation occurs and the circumstances in which the event or situation does not occur.

### DETAILED DESCRIPTION

The present application is further described in detail below in combination with examples, but the content of the present application is not limited to the examples.

### Example 1

### Synthesis of (2S,2'S)-3,3'-((3-((S)-2-carboxyl-2-(R)-pyrrolid-3-yl)ethyl)phenyl)sulfonyl) azadiyl)bis(methylenyl)bis(3,1-phenylenyl)bis(2-(R)-pyrrolid-3-yl)propanoic acid)

A specific synthetic route is as follows:

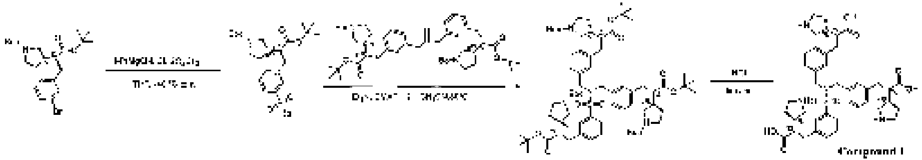

### Step A: synthesis of tert-butyl (R)-3-(S)-1-tert-butoxy-3-(3-chlorosulfonyl)phenyl-1-oxoprop-2-yl)pyrrolidine-1-carboxylate

At a room temperature, tert-butyl (R)-3-(S)-3-(3-bromophenyl)-1-tert-butoxy-1-oxoprop-2-y l)pyrrolidine-1-carboxylate (200 mg, 0.44 mmol) was dissolved in anhydrous THF (3 mL), under nitrogen protection, stirring was conducted at -40°C, then isopropylmagnesium chloride and lithium chloride liquid (1.1 mL, 2.5 mol/L) was added to react for two hours, then sulfuryl chloride (101 mg, 0.53 mmol) was added, a temperature was slowly raised to the room temperature, stirring was continued for 14.0 hours, then detection on a TLC spot plate was carried out, after raw materials were reacted completely, an ammonium chloride aqueous solution (10 mL) was added at -30°C for quenching, EA (20 mL × 3 times) was used for extraction, a collected organic phase was dried with anhydrous sodium sulfate, reduced pressure distillation was carried out, and a crude product was subjected to column chromatography through n-hexane: ethyl acetate=3:1 to obtain 113 mg of tert-butyl (R)-3-(S)-1-tert-butoxy-3-(3-chlorosulfonyl)phenyl-1-oxoprop-2-yl)pyrrolidine-1-carboxylat e. LCMS: RT = 2.17 min, [M+H]⁺ = 474.17.

### Step B: synthesis of 3,3'-((2S,2'S)-di-tert-butyl(3-(S)-3-tert-butoxy)-2-(R)-1-(tert-butoxycarbonyl)pyrrolid-3-yl)-3-oxopropyl)phenyl)sulfonyl)azadiyl)dimethylenyl)bis(3 ,1-phenylenyl)bis(3-tert-butoxy)-3-oxoprop-1,2-diyl)(3R,3'R)-bis(pyrrolidine-1-carboxy late)

At a room temperature, tert-butyl (R)-3-(S)-1-tert-butoxy-3-(3-chlorosulfonyl)phenyl-1-oxoprop-2-yl)pyrrolidine-1-carboxylate (55 mg, 0.12 mmol) was dissolved in acetonitrile (2.5 mL) and stirred, then di-tert-butyl 3,3'-((2S,2'S)-((azadiyl bis(methylenyl))bis(3,1-phenylenyl))bis-(3-(tert-butoxy)-3-oxoprop-1,2-diyl))(3R,3'R)-bis(p yrrolidine-1-formate) (101 mg, 0.13 mmol), 4-dimethylamino pyridine (4.0 mg, 0.036 mmol) and triethylamine (15.4 mg, 0.15 mmol) were added, after stirring at 80°C for 14 hours, TLC detection was carried out, a reaction was ended after raw materials reacted completely, water (6 mL) was added for quenching, ethyl acetate (10 mL × 3 times) was used for extraction, a collected organic phase was dried with anhydrous sodium sulfate, reduced pressure distillation was carried out, and a crude product was obtained and subjected to column chromatography through n-hexane: ethyl acetate=3:1 to obtain 100 mg of 3,3'-((2S,2'S)-di-tert-butyl (3-(S)-3-tert-butoxy)-2-(R)-1-(tert-butoxycarbonyl)pyrrolid-3-yl) -3-oxopropyl)phenyl)sulfonyl)azadiyl)dimethylenyl)bis(3,1-phenylenyl)bis(3-tert-butoxy)-3 -oxoprop-1,2-diyl)(3R,3'R)-bis(pyrrolidine-1-carboxylate).

### Step C: synthesis of (2S,2'S)-3,3'-(3-((S)-2-carboxyl-2-(R)-pyrrolid-3-yl)ethyl)phenyl) sulfonyl)azadiyl)bis(methylenyl)bis(3,1-phenylenyl)bis(2-(R)-pyrrolid-3-yl)propanoic acid)

3,3'-((2S,2'S)-di-tert-butyl(3-(S)-3-tert-butoxy)-2-(R)-1-(tert-butoxycarbonyl)pyrrolid-3-yl)-3-oxopropyl)phenyl)sulfonyl)azadiyl)dimethylenyl)bis(3,1-phenylenyl)bis(3-tert-butoxy)-3-oxoprop-1,2-diyl)(3R,3'R)-bis(pyrrolidine-1-carboxylate) (100 mg, 0.081 mmol) was dissolved in 1,4-dioxane (3.0 mL), concentrated hydrochloric acid (1.0 mL) was added to react at the room temperature for 10 hours, LCMS detection was carried out, and a reaction was ended if the reaction was complete. Vacuum concentration was conducted to obtain a crude product of a target product, and the crude product was purified through preparative high performance liquid chromatography to obtain 5.2 mg of (2S,2'S)-3,3'-(3-((S)-2-carboxyl-2-(R)-pyrrolid-3-yl)ethyl)phenyl)sulfonyl)azadiyl)bis(meth ylenyl)bis(3,1-phenylenyl)bis(2-(R)-pyrrolid-3-yl)propanoic acid). LCMS: RT = 1.49 min, [M-H]⁻ = 759.43, HPLC: 90.22%.

### Example 2

### Synthesis of (2S,2'S)-3,3'-(((3-((S)-2-carboxyl-2-((R)-pyrrolid-3-yl)ethyl)benzyl)aza diyl)bis(methylenyl)bis(6-fluoro-3,1-phenylenyl))bis(2-(((R)-pyrrolid-3-yl)propanoic acid)

A specific synthetic route is as follows:

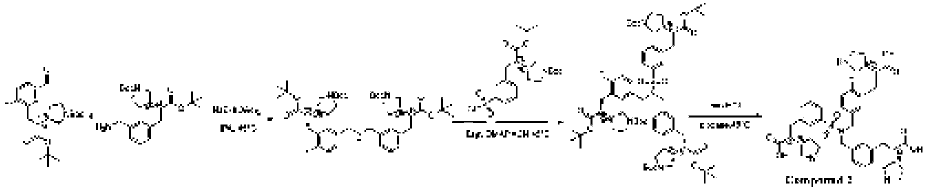

### Step A: synthesis of tert-butyl (R)-3-((S)-1-(tert-butoxy)-3-(3-(((3-(S)-3-(tert-butoxy)-2-((R)-1-(tert-butoxycarbonyl)pyrrolid-3-yl)-3-oxopropyl)-4-fluorobenzyl)amino)meth yl)phenyl)-1-oxoprop-2-yl)pyrrolidine-1-formate

At a room temperature, tert-butyl (R)-3-((S)-1-(tert-butoxy)-3-(2-fluoro-5-formylphenyl)-1-oxoprop-2-yl)pyrrolidine-1-formate (200 mg, 0.47 mmol), tert-butyl (R)-3-((S)-3-(3-(aminomethyl)phenyl)-1-(tert-butoxy)-1-oxoprop-2-yl)pyrrolidine-1-formate (192 mg, 0.47 mmol) and anhydrous isopropanol (5 mL) were added in sequence, the temperature was raised to 45°C, and then triacetoxyl sodium borohydride (201 mg, 0.94 mmol) was added in three batches to react at 45°C for 18 hours.

The reaction was ended, a saturated ammonium chloride aqueous solution (10 mL) was added for quenching, ethyl acetate (20 mL × 2 times) was used for extraction, concentration was carried out to dryness, and a residue was purified through silica-gel column chromatography (eluent: ethyl acetate/n-hexane=2/1) to obtain 210 mg of tert-butyl (R)-3-((S)-1-(tert-butoxy)-3-(3-(((3-(S)-3-(tert-butoxy)-2-((R)-1-(tert-butoxycarbonyl)pyrrol id-3-yl)-3-oxopropyl)-4-fluorobenzyl)amino)methyl)phenyl)-1-oxoprop-2-yl)pyrrolidine-1-f ormate. LCMS: RT=2.03min, [M+H]⁺=810.08.

### Step B: tert-butyl (R)-3-((S)-1-(tert-butoxy)-3-(3-(((3-((S)-3-(tert-butoxy)-2-((R)-1-(tert-butoxycarbonyl)pyrrolid-3-yl)-3-oxopropyl)-N-(3-((S)-3-(tert-butoxy)-2-((R)-1-(te rt-butoxycarbonyl)pyrrolid-3-yl)-3-oxopropyl)-4-fluorobenzyl)phenyl)sulfonylamino)m ethyl)phenyl)-1-oxoprop-2-yl)pyrrolidine-1-carboxylate

At a room temperature, tert-butyl (R)-3-((S)-1-(tert-butoxy)-3-(3-(((3-(S)-3-(tert-butoxy)-2-((R)-1-(tert-butoxycarbonyl)pyrrolid-3-yl)-3-oxopropyl)-4-fluorobenzyl)amino)methyl)ph enyl)-1-oxoprop-2-yl)pyrrolidine-1-formate (172 mg, 0.21 mmol), tert-butyl (R)-3-((S)-1-(tert-butoxy)-3-(3-(chlorosulfonyl)phenyl)-1-oxoprop-2-yl)pyrrolidine-1-forma te (100 mg, 0.2 mmol), acetonitrile (3 mL), triethylamine (32 mg, 0.32 mmol), and 4-dimethylamino pyridine (9 mg, 0.07 mmol) were added, nitrogen displacement was carried out, and the temperature was raised to 80°C to react for 2 hours.

The reaction was ended, tap water (10 mL) and ethyl acetate (10 mL) were added for extraction, concentration was carried out to dryness so as to obtain a crude product, and the crude product was directly used for the next step.

### Step C: (2S,2'S)-3,3'-(((3-((S)-2-carboxyl-2-((R)-pyrrolid-3-yl)ethyl)benzyl)azadiyl)bis (methylenyl)bis(6-fluoro-3,1-phenylenyl))bis(2-(((R)-pyrrolid-3-yl)propanoic acid)

At a room temperature, the crude product from the last step, dioxane (2.5 mL) and concentrated hydrochloric acid (0.5 mL) were added to react at 45°C for 18 hours.

The reaction was ended, concentration was carried out to dryness, and a residue was purified through preparative high performance liquid chromatography to obtain 18 mg of (2S,2'S)-3,3'-(((3-((S)-2-carboxyl-2-((R)-pyrrolid-3-yl)ethyl)benzyl)azadiyl)bis(methylenyl) bis(6-fluoro-3,1-phenylenyl))bis(2-(((R)-pyrrolid-3-yl)propanoic acid). LCMS: RT=1.58min, [M-H]⁻=777.30.

### Example 3

### Synthesis of (S)-3-(3-(((3-(S)-2-carboxyl-2-((R)-pyrrolid-3-yl)ethyl)-N-(2-((S)2-carboxyl-2-(R)pyrrolid-3-yl)ethyl)phenethyl)phenyl)sulfonamido)methyl)phenyl)-2-((R )-pyrrolid-3-yl)propanoic acid

A specific synthetic route is as follows:

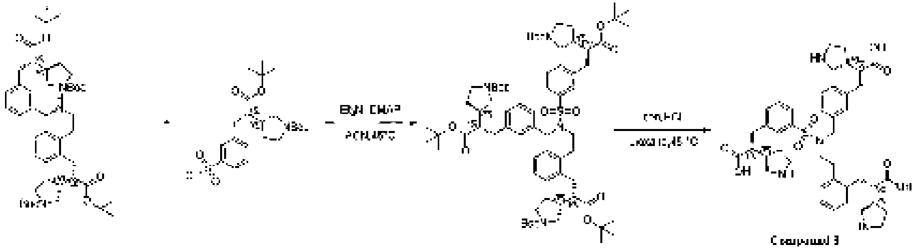

Preparation was performed with reference to the method for the compound 2. LCMS: RT = 1.45 min, [M-H]⁻ = 773.31.

### Example 4

### Synthesis of (S)-3-(3-(N-(5-((S)-2-carboxyl-2-(R)-pyrrolid-3-yl)ethyl)-2-fluorobenzyl)-N-(3-((S)-2-carboxyl-2-((R)-pyrrolid-3-yl)ethyl)benzyl)aminosulfonyl)phenyl)-2-(R)-pyrroli d-3-yl propanoic acid

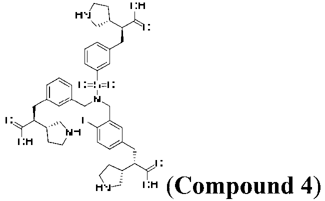

Preparation was performed with reference to the method for the compound 2. LCMS: RT = 1.52min, [M+H]⁺ = 779.16. 1H NMR (400 MHz, D₂O) δ 7.61 (d, J = 6.2 Hz, 1H), 7.53 (s, 1H), 7.47 (d, J = 6.2 Hz, 2H), 7.12 (t, J = 7.6 Hz, 1H), 7.01 - 6.96 (m, 2H), 6.93 (d, J = 7.6 Hz, 1H), 6.84 - 6.78 (m, 3H), 4.31 (s, 4H), 3.51 - 3.28 (m, 6H), 3.21 - 3.12 (m, 3H), 2.98 - 2.73 (m, 6H), 2.72 - 2.24 (m, 9H), 2.10 - 2.03 (m, 3H), 1.74 - 1.61 (m, 3H).

### Example 5

### Synthesis of (S)-3-(3-(S)-2-carboxyl-2-(R)-pyrrolid-3-ethyl)-2-fluorobenzyl)-N-(3-(S)-2-carboxyl-2-(R)-pyrrolid-3-yl)ethyl)benzyl)aminosulfonyl)phenyl)-2-(R)-pyrrolid-3-yl propanoic acid

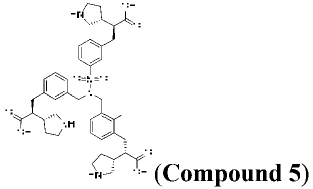

Preparation was performed with reference to the method for the compound 2. LCMS: RT = 1.51min, [M+H]⁺ = 779.21.

### Example 6

### Synthesis of (2S,2'S)-3,3'(3-(S)-2-carboxyl-2-(R)-pyrrolid-3-yl)ethyl)-4-fluorophenyl-sulfonyl)azadiyl)dimethylenyl)bis(3,1-phenylenyl)bis(2-(R)-pyrrolid-3-yl)propanoic acid)

A specific synthetic route is as follows:

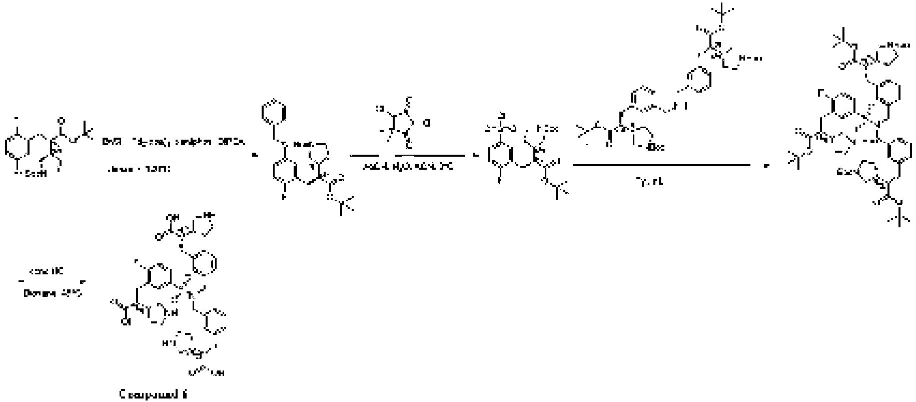

### Step A: synthesis of tert-butyl (R)-3-(S)-3-(5-(benzylthio)-2-fluorophenyl)-1-tert-butoxy-1-oxoprop-2-yl)pyrrolidine-1-carboxylate

At a room temperature, tert-butyl (R)-3-(S)-3-(5-bromo-2-fluorophenyl)-1-tert-butoxy-1-oxoprop-2-yl)pyrrolidine-1-carboxylate (2.0 g, 4.23 mmol), benzyl mercaptane (630 mg, 5.08 mmol), 4,5-bisdiphenylphosphine-9,9-dimethylxanthene (245 mg, 0.42 mmol), tris(dibenzylideneacetone)dipalladium (194 mg, 0.21 mmol) and N,N-diisopropylethylamine (1.1 g, 8.46 mmol) were dissolved in dioxane (40 mL), and a temperature was raised to 100°C to react for 5 hours. Suction filtration was carried out after the reaction was ended, a filtrate was subjected to vacuum concentration to obtain a residue, and the residue was purified through silica-gel column chromatography (eluent: ethyl acetate/n-hexane=1/4) to obtain 2.0 g of tert-butyl (R)-3-(S)-3-(5-(benzylthio)-2-fluorophenyl)-1-tert-butoxy-1-oxopr op-2-yl)pyrrolidine-1-carboxylate. LCMS: RT = 2.52 min, [M+H-Boc]⁺ = 416.13.

### Step B: synthesis of tert-butyl (R)-3-(S)-1-tert-butoxy-3-(5-chlorosulfonyl)-2-fluorophe nyl-1-oxoprop-2-yl)pyrrolidine-1-carboxylate

Tert-butyl (R)-3-(S)-3-(5-(benzylthio)-2-fluorophenyl)-1-tert-butoxy-1-oxoprop-2-yl)pyrrol idine-1-carboxylate (1.0 g, 1.94 mmol) was dissolved in acetic acid/water/acetonitrile (1.1/0.7/30 mL), an acetonitrile solution (2 mL) of 1,3-dichloro-5,5-dimethyl hydantoin (760 mg, 3.88 mmol) was dropwise added into an obtained solution in an ice bath, and after addition, stirring was carried out until the solution turned red. After the reaction was ended, water was added for dilution, a mixture was extracted with ethyl acetate (30 mL × 2 times), combined organic phases were washed using a saturated saline solution (20 mL× 2 times) first and then dried using anhydrous sodium sulfate, vacuum concentration was carried out to obtain a residue, and the residue was purified through silica-gel column chromatography (eluent: ethyl acetate/n-hexane=1/4) to obtain 0.8 g of tert-butyl (R)-3-(S)-1-tert-butoxy-3-(5-chlorosulfonyl)-2-fluorophenyl-1-oxoprop-2-yl)pyrrolidine-1-c arboxylate. LCMS: RT = 2.29 min, [M+H-Boc-tert-butyl]⁺ = 336.01.

Preparation in step C and step D was performed with reference to the method for the compound 2. LCMS: RT = 1.52min, [M+H]⁺ = 779.16. ¹H NMR (400 MHz, D₂O) δ 7.66 (t, J = 6.8 Hz, 1H), 7.62 (d, J = 7.2 Hz, 1H), 7.26 (t, J = 9.2 Hz, 1H), 7.13 (t, J = 7.2 Hz, 2H), 7.05 (d, J = 7.2 Hz, 2H), 6.88 (d, J = 7.6 Hz, 2H), 6.79 (s, 2H), 4.26 (q, 4H), 3.53 - 3.47 (m, 3H), 3.40 - 3.34 (m, 3H), 3.27 - 3.12 (m, 3H), 3.11 - 2.84 (m, 6H), 2.78 - 2.36 (m, 9H), 2.14 - 2.09 (m, 3H), 1.77 - 1.64 (m, 3H).

### Example 7

### Synthesis of (2S,2'S)-3,3'(3-((S)-2-carboxyl-2-(R)-pyrrolid-3-yl)ethyl)-5-fluorophenyl sulfonyl)azadiyl)dimethylenyl)bis(3,1-phenylenyl)bis(2-(R)-pyrrolid-3-yl)propanoic acid)

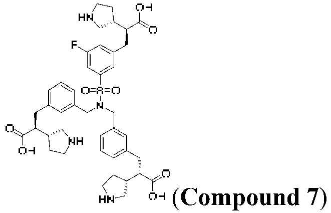

Preparation was performed with reference to the method for the compound 2. LCMS: RT = 1.51min, [M+H]⁺ = 779.21.

### Example 8

### Synthesis of (2S,2'S)-3,3'(3-(S)-2-carboxyl-2-(R)-pyrrolid-3-yl)ethyl)-2-fluorophenyl sulfonyl)azadiyl)dimethylenyl)bis(3,1-phenylenyl)bis(2-(R)-pyrrolid-3-yl)propanoic acid)

A specific synthetic route is as follows:

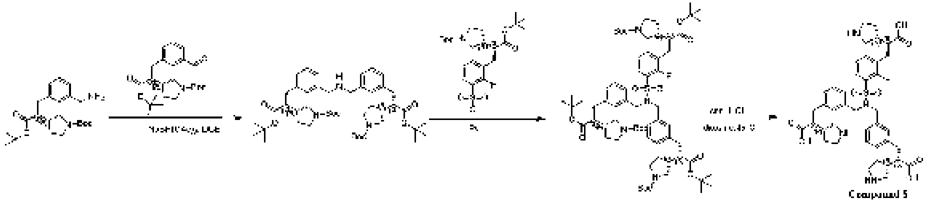

### Step A: synthesis of 3,3'-((2S,2'S)-(azadiyl dimethylenyl)bis(3,1-phenylenyl)bis(3-tert-butoxy)-3-oxoprop-1,2-diyl)(3R,3'R)-bis(pyrrolidine-1-carboxylate)

At a room temperature, tert-butyl (R)-3-(S)-3-(3-aminomethyl)phenyl)-1-tert-butoxy-1 -oxoprop-2-yl)pyrrolidine-1-carboxylate (300 mg, 0.74 mmol) and tert-butyl (R)-3-(S)-1-tert-butoxy-3-(3-formylphenyl)-1-oxoprop-2-yl)pyrrolidine-1-carboxylate (300 mg, 0.74 mmol) were dissolved in DCE (6 mL), triacetoxyl sodium borohydride (551 mg, 2.6 mmol) was added in batches, the temperature was raised to 45°C, and stirring was continued for 16 hours.

After the reaction was complete under TLC monitoring, an aqueous solution (1 mL) was added to quench the reaction, and an obtained crude product was purified through silica-gel column chromatography (eluent: ethyl acetate/n-hexane=1/5). 450 mg of 3,3'-((2S,2'S)-(azadiyl dimethylenyl)bis(3,1-phenylenyl)bis(3-tert-butoxy)-3-oxoprop-1,2-diyl)(3R,3'R)-bis(pyrrolidine-1-carboxylate) was obtained. LC-MS: RT = 2.02 min, [M+H]⁺ =792.61.

### Step B: synthesis of 3,3'-(((3-(S)-3-tert-butoxy)-2-(R)-1-(tert-butoxycarbonyl)pyrrolid-3 -yl)-3-oxopropyl)-2-fluorophenyl)sulfonyl)azadiyl)dimethylenyl)bis(3,1-phenylenyl)bis(3-te rt-butoxy)-3-oxoprop-1,2-diyl)(3R,3'R)-bis(pyrrolidine-1-carboxylate)

At a room temperature, 3,3'-((2S,2'S)-(azadiyl dimethylenyl)bis(3,1-phenylenyl)bis(3-te rt-butoxy)-3-oxoprop-1,2-diyl)(3R,3'R)-bis(pyrrolidine-1-carboxylate) (200 mg, 0.25 mmol) was dissolved in pyridine (4 mL), and an acetonitrile solution (2 mL) of tert-butyl (R)-3-(S)-1-tert-butoxy-3-(3-chlorosulfonyl)-2-fluorophenyl)-1-oxoprop-2-yl)pyrrolidine-1-carboxylate (149 mg, 0.30 mmol) was slowly added to react at the room temperature for 0.5 hours.

After the reaction was detected to be ended through TLC, ethyl acetate (10 mL× 2 times) was used for extraction, combined organic phases were washed using a saturated saline solution (5 mL × 2 times) first and then dried using anhydrous sodium sulfate, vacuum concentration was carried out to obtain a residue, and the residue was purified through silica-gel column chromatography (eluent: n-hexane/ethyl acetate=3/1) to obtain 60 mg of 3,3'-(((3-(S)-3-tert-butoxy)-2-(R)-1-(tert-butoxycarbonyl)pyrrolid-3-yl)-3-oxopropyl)-2-fluo rophenyl)sulfonyl)azadiyl)dimethylenyl)bis(3,1-phenylenyl)bis(3-tert-butoxy)-3-oxoprop-1, 2-diyl)(3R,3'R)-bis(pyrrolidine-1-carboxylate).

### Step C: synthesis of (2S,2'S)-3,3'(3-(S)-2-carboxyl-2-(R)-pyrrolid-3-yl)ethyl)-2-fluoro phenylsulfonyl)azadiyl)dimethylenyl)bis(3,1-phenylenyl)bis(2-(R)-pyrrolid-3-yl)propanoic acid)

At a room temperature, 3,3'-(((3-(S)-3-tert-butoxy)-2-(R)-1-(tert-butoxycarbonyl)pyrro lid-3-yl)-3-oxopropyl)-2-fluorophenyl)sulfonyl)azadiyl)dimethylenyl)bis(3,1-phenylenyl)bis (3-tert-butoxy)-3-oxoprop-1,2-diyl)(3R,3'R)-bis(pyrrolidine-1-carboxylate) (60 mg, 0.05 mmol) was dissolved in a 1,4-dioxane solution (2 mL), concentrated hydrochloric acid (0.1 mL) was added, and the temperature was raised to 45°C to react for 4 hours.

The reaction was under LC-MS monitoring until it was complete, vacuum concentration was carried out, and a residue was purified through preparative high performance liquid chromatography to obtain 16 mg of (2S,2'S)-3,3'(3-(S)-2-carboxyl-2-(R)-pyrrolid-3-yl)ethyl) -2-fluorophenylsulfonyl)azadiyl)dimethylenyl)bis(3,1-phenylenyl)bis(2-(R)-pyrrolid-3-yl)pr opanoic acid). LCMS: RT=1.51 min, [M-H]⁻=777.1.

### Example 9

### Synthesis of (2S,2'S)-3,3'(3-((S)-2-carboxyl-2-(R)-pyrrolid-3-yl)ethyl)phenyl)sulfonyl) azadiyl)dimethylenyl)bis(6-fluoro-3,1-phenylenyl)bis(2-(R)-pyrrolid-3-yl propanoic acid)

A specific synthetic route is as follows:

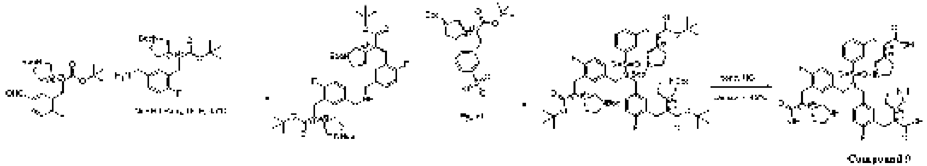

### Step A: synthesis of 3,3'-((2S,2'S)-(azadiyldimethylenyl)bis(6-fluoro-3,1-phenylenyl)bis(3-tert-butoxy)-3-oxoprop-1,2-diyl)(3R,3'R)-bis(pyrrolidine-1-tert-butyl carboxylate)

At a room temperature, tert-butyl (R)-3-(S)-1-tert-butoxy-3-(2-fluoro-5-formylphenyl)-1-oxoprop-2-yl)pyrrolidine-1-carboxylate (350 mg, 0.83 mmol) and tert-butyl (R)-3-(S)-3-(5-aminomethyl)-2-fluorophenyl)-1-tert-butoxy-1-oxoprop-2-yl)pyrrolidine-1-c arboxylate (420 mg, 1.00 mmol) were dissolved in dry 1,2-dichloroethane (5 mL), the temperature was raised to 45°C to react for 3 hours, then triacetoxyl sodium borohydride (528 mg, 2.49 mmol) was added in batches to react at 45°C for 4 hours, after the reaction was ended, water was added for dilution, a mixture was extracted using ethyl acetate (20 mL × 2 times), combined organic phases were washed using a saturated saline solution (20 mL × 2 times) first and then dried using anhydrous sodium sulfate, vacuum concentration was carried out to obtain a residue, and the residue was purified through silica-gel column chromatography (eluent: ethyl acetate/n-hexane=1/3) to obtain 350 mg of 3,3'-((2S, 2'S)-(azadiyl dimethylenyl)bis(6-fluoro-3,1-phenylenyl)bis(3-tert-butoxy)-3-oxoprop-1,2-diyl)(3R,3'R)-bis(pyrrolidine-1-carboxylate). LCMS: RT = 2.11min, [M+H]⁺ = 828.29.

### Step B: synthesis of 3,3'-(3-(S)-3-tert-butoxy)-2-(R)-1-(tert-butoxycarbonyl)pyrrolid-3 -yl)-3-oxopropyl)phenyl)sulfonyl)azadiyl)dimethylenyl)bis(6-fluoro-3,1-phenylenyl)bis(3-te rt-butoxy)-3-oxoprop-1,2-diyl)(3R,3'R)-bis(pyrrolidine-1-tert-butyl carboxylate)

At a room temperature, 3,3'-((2S,2'S)-(azadiyl dimethylenyl)bis(6-fluoro-3,1-phenylenyl)bis (3-tert-butoxy)-3-oxoprop-1,2-diyl)(3R,3'R)-bis(pyrrolidine-1-carboxylate) (170 mg, 0.20 mmol) was dissolved in pyridine (2 mL), and an acetonitrile solution (1.2 mL) of tert-butyl (R)-3-(S)-1-tert-butoxy-3-(3-chlorosulfonyl)phenyl-1-oxoprop-2-yl)pyrrolidine-1-carboxylat e (116 mg, 0.25 mmol) was added to react at the room temperature for 18 hours. After the reaction was ended, vacuum concentration was carried out to obtain a residue, and the residue was purified through silica-gel column chromatography (eluent: ethyl acetate/n-hexane=1/3) to obtain 50 mg of 3,3'-(3-(S)-3-tert-butoxy)-2-(R)-1-(tert-butoxycarbonyl)pyrrolid-3-yl)-3-oxopropyl)phenyl)s ulfonyl)azadiyl)dimethylenyl)bis(6-fluoro-3,1-phenylenyl)bis(3-tert-butoxy)-3-oxoprop-1,2-diyl)(3R,3'R)-bis(pyrrolidine-1-tert-butyl carboxylate).

### Step C: synthesis of (2S,2'S)-3,3'(3-((S)-2-carboxyl-2-(R)-pyrrolid-3-yl)ethyl)phenyl) sulfonyl)azadiyl)dimethylenyl)bis(6-fluoro-3,1-phenylenyl)bis(2-(R)-pyrrolid-3-yl propanoi c acid)

3,3'-(3-(S)-3-tert-butoxy)-2-(R)-1-(tert-butoxycarbonyl)pyrrolid-3-yl)-3-oxopropyl)phe nyl)sulfonyl)azadiyl)dimethylenyl)bis(6-fluoro-3,1-phenylenyl)bis(3-tert-butoxy)-3-oxoprop -1,2-diyl)(3R,3'R)-bis(pyrrolidine-1-tert-butyl carboxylate) (50 mg, 0.040 mmol) was dissolved in 1,4-dioxane (3.0 mL), and concentrated hydrochloric acid (0.3 mL) was added to react at 45°C for 6 hours. After the reaction was ended, vacuum concentration was carried out to obtain a residue, and the residue was separated and purified through high performance liquid chromatography to obtain 26.2 mg of (2S,2'S)-3,3'(3-((S)-2-carboxyl-2-(R)-pyrrolid-3-yl)ethyl)phenyl)sulfonyl)azadiyl)dimethyle nyl)bis(6-fluoro-3,1-phenylenyl)bis(2-(R)-pyrrolid-3-yl propanoic acid).

LCMS: RT = 1.53min, [M-H]⁻ = 795.12. ¹H NMR (400 MHz, D₂O) δ 7.70 - 7.62 (m, 1H), 7.58 (s, 1H), 7.51 (d, J = 6.6 Hz, 2H), 6.92 - 6.80 (m, 4H), 6.77 (d, J = 7.2 Hz, 2H), 4.23 (s, 2H), 4.20 (s, 2H), 3.54 (dd, J = 11.8, 7.8 Hz, 3H), 3.40 - 3.35 (m, 3H), 3.31 - 3.14 (m, 3H), 3.09 - 2.81 (m, 6H), 2.81 - 2.30 (m, 9H), 2.15 - 2.08 (m, 3H), 1.77 - 1.64 (m, 3H).

### Example 10

### Synthesis of (S)-3-(3-(N-(3-((S)-2-carboxyl-2-(R)-pyrrolid-3-ethyl)-4-chlorobenzyl)-N-(3-((S)-2-carboxyl-2-(R)-pyrrolid-3-yl)ethyl)benzyl)aminosulfonyl)phenyl)-2-((R)-pyrrolid-3-yl)propanoic acid

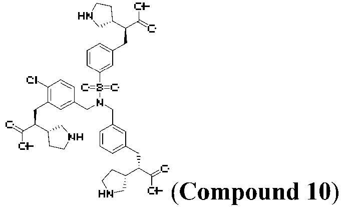

Preparation was performed with reference to the method for the compound 9. LCMS: RT = 1.54min, [M+H]⁺ = 795.11. ¹H NMR (400 MHz, D₂O) δ 7.62 (s, 1H), 7.51 (s, 1H), 7.47 (d, J = 4.8 Hz, 2H), 7.16 (d, J = 8.2 Hz, 1H), 7.11 (t, J = 7.6 Hz, 1H), 7.00 (d, J = 7.6 Hz, 1H), 6.93 (d, J = 7.6 Hz, 1H), 6.87 (d, J = 8.2 Hz, 1H), 6.84 - 6.76 (m, 2H), 4.29 (s, 2H), 4.23 (s, 2H), 3.53 - 3.27 (m, 6H), 3.23 - 3.06 (m, 3H), 2.98 - 2.73 (m, 6H), 2.72 - 2.56 (m, 3H), 2.55 - 2.16 (m, 6H), 2.09 - 2.06 (m, 3H), 1.73 - 1.59 (m, 3H).

### Example 11

### Synthesis of (2S,2'S)-3,3'(3-((S)-2-carboxyl-2-((R)-pyrrolid-3-yl)ethyl)phenyl)sulfonyl) azadiyl)bis(methylenyl))bis(2-fluoro-3,1-phenylenyl)bis((2-(R)pyrrolid-3-yl propanoic acid)

A specific synthetic route is as follows:

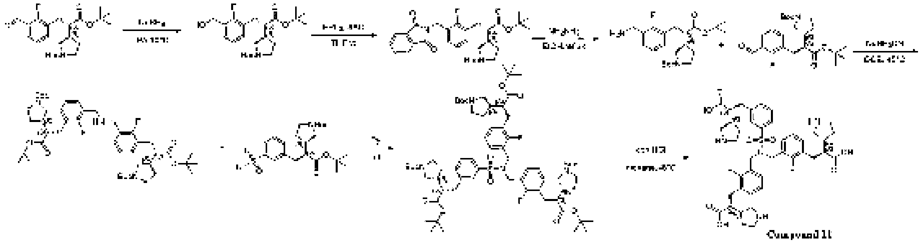

### Step A: tert-butyl (R)-3-((S)-1-(tert-butoxy)-3-(2-fluoro-3-(hydroxymethyl)phenyl)-1-oxoprop-2-yl)pyrrolidine-1-formate

At a room temperature, tert-butyl (R)-3-((S)-1-(tert-butoxy)-3-(2-fluoro-3-formylphenyl )-1-oxoprop-2-yl)pyrrolidine-1-formate (320 mg, 0.76 mmol) and anhydrous isopropanol (5 mL) were added, the temperature was reduced through ice water, and then sodium borohydride (28 mg, 0.76 mmol) was added in three batches to react in an ice water bath for 1 hour.

The reaction was ended, a saturated ammonium chloride aqueous solution (10 mL) was added for quenching, ethyl acetate (10 mL×2 times) was used for extraction, washing was carried out using a saturated sodium chloride aqueous solution (10 mL × 1 times), drying was carried out through sodium sulfate, and concentration was carried out to dryness so as to obtain 350 mg of tert-butyl (R)-3-((S)-1-(tert-butoxy)-3-(2-fluoro-3-(hydroxymethyl)phenyl )-1-oxoprop-2-yl)pyrrolidine-1-formate. LCMS: RT=2.13min, [M-Boc+H]⁺=324.17.

### Step B: tert-butyl (R)-3-((S)-1-(tert-butoxy)-3-(3-((1,3-dioxoisoindolin-2-yl)methyl)-2-fluorophenyl)-1-oxoprop-2-yl)pyrrolidine-1-formate

At a room temperature, tert-butyl (R)-3-((S)-1-(tert-butoxy)-3-(2-fluoro-3-(hydroxymet hyl)phenyl)-1-oxoprop-2-yl)pyrrolidine-1-formate (350 mg, 0.83 mmol), phthalimide (157 mg, 1.07 mmol), triphenylphosphine (434 mg, 1.65 mmol) and tetrahydrofuran (5 mL) were added in sequence, and then diisopropyl azodicarboxylate (333 mg, 1.65 mmol) was slowly added dropwise to react at the room temperature for 2 hours.

The reaction was ended, concentration was carried out to dryness, and a residue was purified through silica-gel column chromatography (eluent: ethyl acetate/n-hexane=1/4) to obtain 630 mg of tert-butyl (R)-3-((S)-1-(tert-butoxy)-3-(3-((1,3-dioxoisoindolin-2-yl)meth yl)-2-fluorophenyl)-1-oxoprop-2-yl)pyrrolidine-1-formate.

### Step C: tert-butyl (R)-3-((S)-1-(tert-butoxy)-3-(2-fluoro-3-(aminomethyl)phenyl)-1-oxo prop-2-yl)pyrrolidine-1-formate

At a room temperature, tert-butyl (R)-3-((S)-1-(tert-butoxy)-3-(3-((1,3-dioxoisoindolin-2-yl)methyl)-2-fluorophenyl)-1-oxoprop-2-yl)pyrrolidine-1-formate (630 mg, 1.14 mmol) was added, ethanol (10 mL) was used for dissolution, then hydrazine hydrate (80%, 178 mg, 2.84 mmol) was added, and the temperature was raised to 85°C to react for 3 hours.

The reaction was ended, white solid was removed by filtration, a filter cake was washed using ethyl acetate (20 mL × 2 times), a filtrate was combined and concentrated to dryness, and a residue was purified through silica-gel column chromatography (eluent: ethyl acetate (containing 0.5% V/V triethylamine)/n-hexane=9/1) to obtain 264 mg of tert-butyl (R)-3-((S)-1-(tert-butoxy)-3-(2-fluoro-3-(aminomethyl)phenyl)-1-oxoprop-2-yl)pyrrolidine-1-formate. LCMS: RT=1.82min, [M+H]⁺=423.16.

### Step D: tert-butyl 3,3'-di-tert-butyl((2S,2'S)-((azadiyl bis(methylenyl))bis(2-fluoro-3,1-phenylenyl))-bis(3-(tert-butoxy)-3-oxoprop-1,2-diyl))(3R,3'R)-dipyrrolidine-1-formate

At a room temperature, tert-butyl (R)-3-((S)-1-(tert-butoxy)-3-(2-fluoro-3-formylphenyl )-1-oxoprop-2-yl)pyrrolidine-1-formate (216 mg, 0.51 mmol), tert-butyl (R)-3-((S)-1-(tert-butoxy)-3-(2-fluoro-3-(aminomethyl)phenyl)-1-oxoprop-2-yl)pyrrolidine-1-formate (260 mg, 0.61 mmol) and 1,2-dichloroethane (6 mL) were added in sequence, the temperature was raised to 45°C, and then triacetoxyl sodium borohydride (326 mg, 1.54 mmol) was added in three batches to react at 45°C for 18 hours.

The reaction was ended, a saturated ammonium chloride aqueous solution (10 mL) was added for quenching, dichloromethane (10 mL × 2 times) was used for extraction, concentration was carried out to dryness, and a residue was purified through silica-gel column chromatography (eluent: ethyl acetate/n-hexane=1/1) to obtain 405 mg of tert-butyl 3,3'-di-tert-butyl((2S,2'S)-((azadiyl bis(methylenyl))bis(2-fluoro-3,1-phenylenyl))-bis(3-(tert -butoxy)-3-oxoprop-1,2-diyl))(3R,3'R)-dipyrrolidine-1-formate. LCMS: RT=2.04min, [M+H]⁺=828.31.

### Step E: 3,3'-di-tert-butyl((2S,2'S)-(3-((S)-3-(tert-butoxy)-2-((R)-1-(tert-butoxycarbonyl) pyrrolid-3-yl)-3-oxopropyl)phenyl)sulfonyl)azadiyl)bis(methylenyl))bis(2-fluoro-3,1-phenyl enyl)bis(3-(tert-butoxy)-3-oxoprop-1,2-diyl))(3R,3'R)-bis(pyrrolidine-1-formate)

At a room temperature, tert-butyl 3,3'-di-tert-butyl((2S,2'S)-((azadiyl bis(methylenyl))bis(2-fluoro-3,1-phenylenyl))-bis(3-(tert-butoxy)-3-oxoprop-1,2-diyl))(3R,3 'R)-dipyrrolidine-1-formate (300 mg, 0.36 mmol) and pyridine (3 mL) were added, and after complete dissolution, tert-butyl (R)-3-((S)-1-(tert-butoxy)-3-(3-(chlorosulfonyl)phenyl)-1-oxoprop-2-yl)pyrrolidine-1-formate (2.1 mL, 100 mg/mL, acetonitrile solution, 0.43 mmol) was added dropwise to react at the room temperature for 2 hours.

The reaction was ended, concentration was carried out to dryness, and a residue was purified through silica-gel column chromatography (eluent: ethyl acetate/n-hexane=1/3) to obtain 226 mg of 3,3'-di-tert-butyl((2S,2'S)-(3-((S)-3-(tert-butoxy)-2-((R)-1-(tert-butoxy carbonyl)pyrrolid-3-yl)-3-oxopropyl)phenyl)sulfonyl)azadiyl)bis(methylenyl))bis(2-fluoro-3 ,1-phenylenyl)bis(3-(tert-butoxy)-3-oxoprop-1,2-diyl))(3R,3'R)-bis(pyrrolidine-1-formate).

### Step F: (2S,2'S)-3,3'(3-((S)-2-carboxyl-2-((R)-pyrrolid-3-yl)ethyl)phenyl)sulfonyl)aza diyl)bis(methylenyl))bis(2-fluoro-3,1-phenylenyl)bis((2-(R)pyrrolid-3-yl propanoic acid)

At a room temperature, 3,3'-di-tert-butyl((2S,2'S)-(3-((S)-3-(tert-butoxy)-2-((R)-1-(tert-butoxycarbonyl)pyrrolid-3-yl)-3-oxopropyl)phenyl)sulfonyl)azadiyl)bis(methylenyl))bis(2-f luoro-3,1-phenylenyl)bis(3-(tert-butoxy)-3-oxoprop-1,2-diyl))(3R,3'R)-bis(pyrrolidine-1-for mate) (220 mg, 0.17 mmol), dioxane (2.5 mL) and concentrated hydrochloric acid (0.5 mL) were added to react at 45°C for 4 hours.

The reaction was ended, concentration was carried out to dryness, and a residue was purified through preparative high performance liquid chromatography to obtain 104 mg of (2S,2'S)-3,3'(3-((S)-2-carboxyl-2-((R)-pyrrolid-3-yl)ethyl)phenyl)sulfonyl)azadiyl)bis(meth ylenyl))bis(2-fluoro-3,1-phenylenyl)bis((2-(R)pyrrolid-3-yl propanoic acid). LCMS: RT=1.48min, [M-H]⁻=795.12. ¹H NMR (400 MHz, DeuteRium Oxide) δ 7.60 - 7.52 (m, 1H), 7.44 (t, J = 7.7 Hz, 3H), 6.99 (dt, J = 16.0, 7.1 Hz, 4H), 6.88 (t, J = 7.6 Hz, 2H), 4.45 - 4.28 (m, 4H), 3.50 - 3.27 (m, 6H), 3.16 (ddd, J = 17.0, 12.6, 7.2 Hz, 3H), 2.97 - 2.69 (m, 5H), 2.57 (d, J = 6.0 Hz, 4H), 2.48 - 2.29 (m, 6H), 2.03 (dd, J = 6.4, 3.4 Hz, 3H), 1.65 (ddt, J = 22.2, 13.1, 9.2 Hz, 3H).

### Example 12

### Synthesis of (S)-3-(3-(N-(3-((S)-2-carboxyl-2-((R)-pyrrolid-3-yl)etyhl)-2-chlorobenzyl )-N-(3-((S)-2-carboxyl-2-((R)pyrrolid-3-yl)ethyl)benzyl)aminosulfonyl)phenyl)-2-((R)-pyrr olid-3-yl)propanoic acid

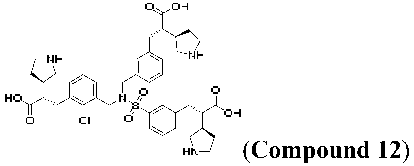

Preparation was performed with reference to the method for the compound 9. LCMS: RT=1.49min, [M-H]⁻=793.07. ¹H NMR (400 MHz, DeuteRium Oxide) δ 7.60 (dt, J = 6.3, 2.3 Hz, 1H), 7.52 (S, 1H), 7.46 (d, J = 6.4 Hz, 2H), 7.11 - 7.00 (m, 4H), 6.96 (d, J = 7.6 Hz, 1H), 6.90 (d, J = 7.6 Hz, 1H), 6.79 (S, 1H), 4.51 - 4.24 (m, 4H), 3.42 (dt, J = 11.5, 7.3 Hz, 2H), 3.32 (ddt, J = 11.6, 7.7, 3.1 Hz, 4H), 3.22 - 3.08 (m, 3H), 2.94 - 2.59 (m, 8H), 2.50 (dd, J = 13.4, 4.4 Hz, 1H), 2.46 - 2.24 (m, 6H), 2.05 (d, J = 16.2 Hz, 3H), 1.75 - 1.57 (m, 3H).

### Example 13

### Synthesis of (2S,2'S)-3,3'-(3-((S)-2-carboxyl-2-(R)-pyrrolid-3-yl)ethyl)phenylsulfonyl )azadiyl)dimethylenyl)bis(4-fluoro-3,1-phenylenyl)bis(2-(R)-pyrrolid-3-yl)propanoic acid)

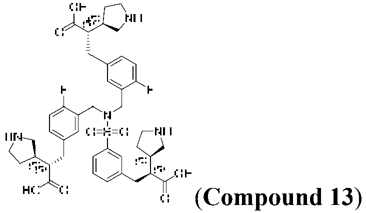

A specific synthetic route is as follows:
Preparation was performed with reference to the method for the compound 11. LCMS: RT = 1.51 min, [M-H]⁻ = 795.09.

### Example 14

### Synthesis of (S)-3-(3-(5-(S)-2-carboxyl-2-(R)-pyrrolid-3-ethyl)-2-fluorobenzyl)-N-(3-(S)-2-carboxyl-2-(R)-pyrrolid-3-yl)ethyl)-4-fluorobenzyl)aminosulfonyl)phenyl)-2-(R)-pyrr olid-3-yl propanoic acid

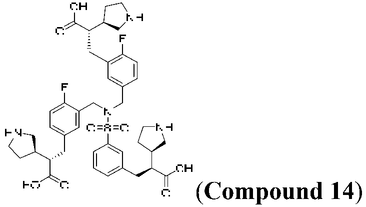

Preparation was performed with reference to the method for the compound 9. LCMS: RT = 1.49 min, [M-H]⁻ = 795.24. ¹H NMR (400 MHz, D₂O)δ 7.60 (d, J = 6.6 Hz, 1H), 7.54 (s, 1H), 7.47 (d, J = 6.6 Hz, 2H), 6.97 (d, J = 2.9 Hz, 1H), 6.90 (d, J = 5.6 Hz, 1H), 6.86 (d, J = 9.5 Hz, 1H), 6.84 - 6.76 (m, 3H), 4.37 - 4.21 (m, 4H), 3.46 (td, J = 13.1, 12.5, 7.4 Hz, 3H), 3.34 (t, J = 4.1 Hz, 3H), 3.18 (td, J = 11.4, 10.1, 5.1 Hz, 3H), 2.98 - 2.79 (m, 5H), 2.69 - 2.56 (m, 4H), 2.54 - 2.33 (m, 6H), 2.06 (s, 3H), 1.77 - 1.60 (m, 3H).

### Example 15

### Synthesis of (S)-3-(3-(N-(5-((S)-2-carboxyl-2-((R)-pyrrolid-3-yl)etyhl)-2,4-difluoro benzyl)-N-(3-(S)2-carboxyl-2-(R)pyrrolid-3-yl)ethyl)benzyl)aminosulfonyl)phenyl)-2-((R)-pyrrolid-3-yl)propanoic acid

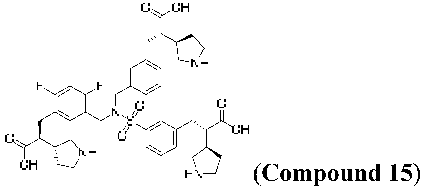

Preparation was performed with reference to the method for the compound 9. LCMS: RT=1.50min, [M-H]⁻=795.20. ¹H NMR (400 MHz, DeuteRium Oxide) δ 7.65 - 7.55 (m, 2H), 7.55 - 7.45 (m, 2H), 7.11 (t, J = 7.6 Hz, 1H), 7.03 (d, J = 7.6 Hz, 1H), 6.91 - 6.80 (m, 3H), 6.66 (t, J = 10.0 Hz, 1H), 4.38 - 4.20 (m, 4H), 3.60 - 3.46 (m, 3H), 3.37 (dd, J = 11.7, 8.6 Hz, 3H), 3.20 (ddd, J = 11.8, 9.7, 7.3 Hz, 3H), 3.07 - 2.85 (m, 5H), 2.74 (dt, J = 16.5, 6.4 Hz, 4H), 2.69 - 2.57 (m, 3H), 2.50 (dq, J = 19.1, 9.4, 9.0 Hz, 3H), 2.18 - 2.06 (m, 3H), 1.70 (dq, J = 13.2, 9.7 Hz, 3H).

### Example 16

### Synthesis of (2S,2'S)-3,3'(3-((S)-2-carboxyl-2-((R)-pyrrolid-3-yl)ethyl)phenyl)sulfonyl) azadiyl)bis(methylenyl))bis(4,6-difluoro-3,1-phenylenyl)bis(2-(R)pyrrolid-3-yl)propanoic acid)

A specific synthetic route is as follows:

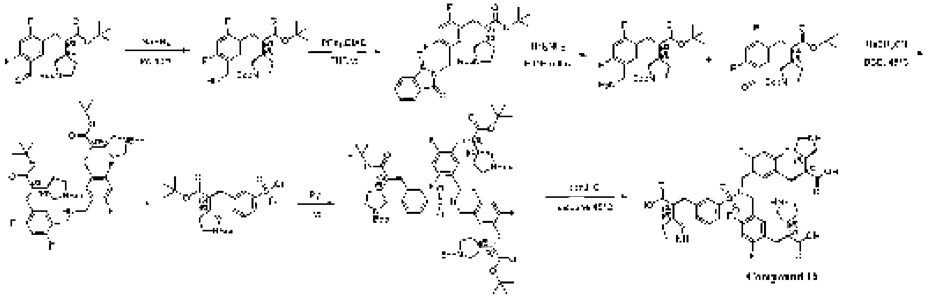

Preparation was performed with reference to the method for the compound 11. LCMS: RT=1.51min, [M-H]⁻=831.06. ¹H NMR (400 MHz, DeuteRium Oxide) δ 7.60 (d, J = 11.6 Hz, 2H), 7.55 - 7.46 (m, 2H), 6.92 (t, J = 8.3 Hz, 2H), 6.69 (t, J = 10.0 Hz, 2H), 4.38 - 4.23 (m, 4H), 3.54 (dd, J = 11.9, 8.1 Hz, 3H), 3.37 (ddd, J = 12.0, 8.4, 3.7 Hz, 3H), 3.26 - 3.15 (m, 3H), 3.01 (q, J = 10.6 Hz, 3H), 2.95 - 2.84 (m, 2H), 2.78 - 2.64 (m, 5H), 2.62 - 2.42 (m, 5H), 2.12 (ddt, J = 13.7, 7.2, 3.8 Hz, 3H), 1.77 - 1.63 (m, 3H).

### Example 17

### Synthesis of (2S,2'S)-3,3'-((3-(S)-2-carboxyl-2-(R)-pyrrolid-3-yl)ethyl)phenyl)sulfonyl) azadiyl)bis(ethane-2,1-diyl)bis(2,1-phenylenyl)bis(2-(R)-pyrrolid-3-yl)propanoic acid)

A specific synthetic route is as follows:

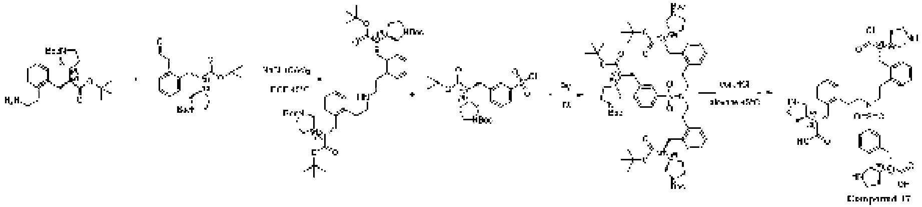

Preparation was performed with reference to the method for the compound 9. LCMS: RT=1.51min, [M+H]⁻ = 787.06.

### Example 18

### Synthesis of (S)-3-(2-(3-(S)-2-carboxyl-2-(R)-pyrrolid-3-yl)ethyl)-N-(2-(S)-2-carboxyl-2-(R)-pyrrolid-3-yl)ethyl)benzyl)phenyl)sulfonamido)ethyl)phenyl)-2-(R)-pyrrolid-3-yl)pro panoic acid

A specific synthetic route is as follows:

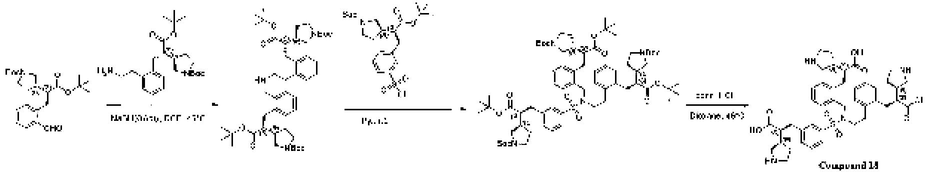

Preparation was performed with reference to the method for the compound 9. LCMS: RT = 1.54min, [M-H]⁻ = 773.12. ¹H NMR (400 MHz, D₂O) δ 7.69 (s, 1H), 7.63 (s, 1H), 7.51 (d, J = 4.6 Hz, 2H), 7.25 (t, J = 7.4 Hz, 1H), 7.21 - 7.11 (m, 2H), 7.12 - 6.99 (m, 4H), 6.76 (d, J = 7.4 Hz, 1H), 4.24 (s, 2H), 3.47 (dd, J = 11.8, 7.2 Hz, 2H), 3.40 - 3.30 (m, 3H), 3.29 - 3.03 (m, 6H), 2.95 (dd, J = 11.8, 7.2 Hz, 2H), 2.86 - 2.66 (m, 6H), 2.68 - 2.24 (m, 9H), 2.05 (s, 3H), 1.84 - 1.39 (m, 3H).

### Example 19

### Synthesis of (S)-3-(3-(2-((3-((S)-2-carboxyl-2-((R)-pyrrolid-3-yl)ethyl)-N-(2-(S)2-carboxyl-2-(R)pyrrolid-3-yl ethyl)phenethyl)phenyl)sulfonamido)ethylphenyl)phenyl)-2-((R)-pyrrolid-3-yl)propanoic acid

A specific synthetic route is as follows:

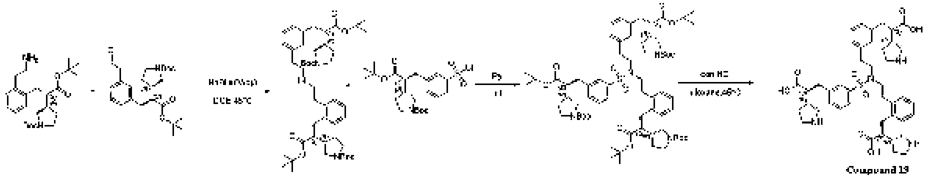

Preparation was performed with reference to the method for the compound 9. LCMS: RT=1.51min, [M-H]⁻=787.20. ¹H NMR (400 MHz, DeuteRium Oxide) δ 7.57 - 7.50 (m, 2H), 7.50 - 7.39 (m, 2H), 7.19 - 7.10 (m, 4H), 7.01 (d, J = 7.4 Hz, 2H), 6.93 (d, J = 7.6 Hz, 1H), 6.83 (S, 1H), 3.54 - 3.42 (m, 3H), 3.35 (tq, J = 8.9, 5.7, 5.2 Hz, 5H), 3.25 - 3.09 (m, 5H), 2.98 (td, J = 10.7, 6.7 Hz, 3H), 2.93 - 2.84 (m, 2H), 2.81 (d, J = 7.0 Hz, 2H), 2.68 (ddt, J = 28.8, 15.1, 7.2 Hz, 9H), 2.48 (dp, J = 25.0, 8.5 Hz, 3H), 2.09 (ddt, J = 14.1, 10.7, 7.1 Hz, 3H), 1.78 - 1.60 (m, 3H).

### Example 20

### Synthesis of (S)-3-(3-(S)-2-carboxyl-2-(R)-pyrrolid-3-yl)ethyl)-N-(3-(S)-2-carboxyl-2-(R)-pyrrolid-3-yl)ethyl)benzyl)phenyl)sulfonamido)ethyl)phenyl)-2-(R)-pyrrolid-3-yl)propa noic acid

A specific synthetic route is as follows:

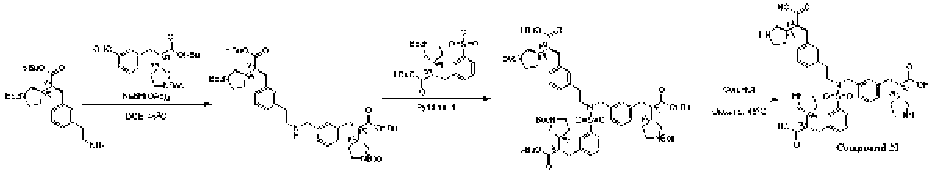

Preparation was performed with reference to the method for the compound 9. LCMS: RT = 1.49 min, [M-H]⁻ = 773.26.

### Example 21

### Synthesis of (2S,2'S)-3,3'(3-((S)-2-carboxyl-2-((R)-pyrrolid-3-yl)ethyl)phenyl)sulfonyl )azadiyl)bis(ethane-2,1-diyl))bis(3,1-phenylenyl)bis(2-(R)-pyrrolid-3-yl)propanoic acid)

A specific synthetic route is as follows:

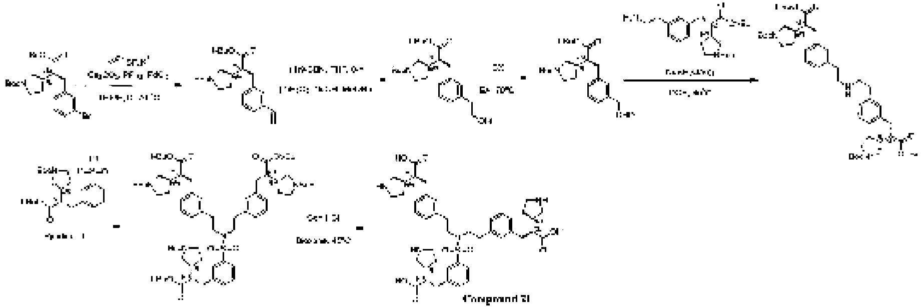

### Step A: synthesis of tert-butyl (R)-3-(S)-1-tert-butoxy-1-oxo-3-(3-vinylphenyl)propyl-2-yl)pyrrolidine-1-carboxylate

At a room temperature, tert-butyl (R)-3-(S)-3-(3-bromophenyl)-1-tert-butoxy-1-oxopro p-2-yl)pyrrolidine-1-carboxylate (15 g, 33.0 mmol), triphenylphosphine (866 mg, 3.30 mmol), cesium carbonate (32.3 g, 99 mmol), palladium dichloride (292 mg, 1.65 mmol) and potassium vinyltrifluoroborate (7.57 g, 49.5 mmol) were added into a reaction flask in sequence, nitrogen displacement was carried out 3 times, then tetrahydrofuran (150 mL) and purified water (30 mL) were added, the temperature was raised to 70°C for a reaction overnight, after the reaction was monitored to be ended via TLC, the temperature was lowered to the room temperature, water (150 mL) was added for dilution, then ethyl acetate (150 mL, 2 times) was used for extraction, combined organic phases were washed using a saturated saline solution (150 mL × 2 times) first and then dried using anhydrous sodium sulfate, vacuum concentration was carried out to obtain a residue, and the residue was purified through silica-gel column chromatography (eluent: ethyl acetate/n-hexane=1/8) to obtain 13.2 g of tert-butyl (R)-3-(S)-1-tert-butoxy-1-oxo-3-(2-vinylphenyl)propyl-2-yl pyrrolidine-1-carboxylate. LCMS: RT = 2.35 min, [M+H]⁺ = 402.23.

### Step B: synthesis of tert-butyl (R)-3-(S)-1-tert-butoxy-3-(3-(2-hydroxyethyl)phenyl)-1-oxoprop-2-yl)pyrrolidine-1-carboxylate

Under an ice water bath, a tetrahydrofuran solution (132 mL, 0.5 mol/L) of 9-borabicyclo[3.3.1]nonane was added dropwise into a tetrahydrofuran (130 mL) solution of tert-butyl (R)-3-(S)-1-tert-butoxy-1-oxo-3-(2-vinylphenyl)propyl-2-yl pyrrolidine-1-carboxy late (13.2 g, 32.87 mmol), stirring was carried out at a room temperature for 18 hours, and then methanol (50 mL), a sodium hydroxide aqueous solution (104 mL, 3 mol/L) and 30% hydrogen peroxide (22.4 g) were added into a reaction liquid under an ice bath to react at the room temperature for 3 hours. After the reaction was monitored to be ended via TLC, an excessive sodium hydrogen sulfate saturated solution was added to quench the reaction, stirring was carried out for 10 minutes, water (100 mL) was added for dilution, then ethyl acetate (200 mL × 2 times) was used for extraction, combined organic phases were washed using a saturated saline solution (150 mL× 2 times) first and then dried using anhydrous sodium sulfate, vacuum concentration was carried out to obtain a residue, and the residue was purified through silica-gel column chromatography (eluent: ethyl acetate/n-hexane=1/2) to obtain 9.6 g of tert-butyl (R)-3-(S)-1-tert-butoxy-3-(2-hydroxyethyl)phenyl-1-oxoprop-2-yl)pyrrolidine-1-carboxylate. LCMS: RT = 2.14 min, [M+H-tert-butyl-tert-butoxycarbonyl]⁺ = 320.22.

### Step C: synthesis of tert-butyl (R)-3-(S)-1-tert-butoxy-1-oxo-3-(3-(2-oxoethyl)phenyl) prop-2-yl)pyrrolidine-1-carboxylate

At a room temperature, 2-iodoxybenzoic acid (3.9 g, 13.90 mmol) was added into an ethyl acetate (100 mL) solution of tert-butyl (R)-3-(S)-1-tert-butoxy-3-(2-hydroxyethyl)phenyl-1-oxoprop-2-yl)pyrrolidine-1-carboxylate (5.3 g, 12.63 mmol), the temperature was raised to 70°C to react for 8 hours, after the reaction was monitored to be ended via TLC, suction filtration was carried out, a filter cake was washed using ethyl acetate, a filtrate was subjected to vacuum concentration to obtain a residue, and the residue was purified through silica-gel column chromatography (eluent: ethyl acetate/n-hexane=1/3) to obtain 2.6 g of tert-butyl (R)-3-(S)-1-tert-butoxy-1-oxo-3-(2-oxoethyl)phenyl)prop-2-yl pyrrolidine-1-carboxylate. LCMS: RT=2.21 min, [M+H]⁺=418.25.

### Step D: synthesis of 3,3'-((2S,2'S)-(azadiyl diethane-2,1-diyl)bis(3,1-phenylenyl)bis(3-tert-butoxy)-3-oxoprop-1,2-diyl)(3R,3'R)-bis(pyrrolidine-1-carboxylate)

Tert-butyl (R)-3-(S)-1-tert-butoxy-1-oxo-3-(3-(2-oxoethyl)phenyl)prop-2-yl)pyrrolidine -1-carboxylate (250 mg, 0.60 mmol), and (R)-3-(S)-3-(3-(2-aminoethyl)phenyl)-1-tert-butoxy-1-oxoprop-2-yl)pyrrolidine-1-carboxyla te (251 mg, 0.60 mmol) were dissolved in 1,2-dichloroethane, and then triacetoxyl sodium borohydride (444 mg, 2.1 mmol) was added in batches to react at 45°C overnight. After the reaction was ended, a solvent was removed through reduced pressure distillation, and a crude product was purified through silica-gel column chromatography (eluent: n-hexane/ethyl acetate=2/1) to obtain 150 mg of 3,3'-((2S,2'S)-(azadiyl diethane-2,1-diyl)bis(3,1-phenylenyl)bis(3-tert-butoxy)-3-oxoprop-1,2-diyl)(3R,3'R)-bis(pyr rolidine-1-carboxylate). LCMS: RT = 2.07 min, [M+H]⁺ = 820.39.

### Step E: synthesis of 3,3'-(3-(S)-3-tert-butoxy-2-(R)-1-(tert-butoxycarbonyl)pyrrolid-3-yl)-3-oxopropyl)phenyl)sulfonyl)azadiyl)bis(ethane-2,1-diyl))bis(3,1-phenylenyl)bis(3-tert-butoxy)-3-oxoprop-1,2-diyl)(3R,3'R)-bis(pyrrolidine-1-carboxylate)

At a room temperature, 3,3'-((2S,2'S)-(azadiyl diethane-2,1-diyl)bis(3,1-phenylenyl)bis (3-tert-butoxy)-3-oxoprop-1,2-diyl)(3R,3'R)-bis(pyrrolidine-1-carboxylate) (250 mg, 0.30 mmol) was dissolved in anhydrous pyridine (2 mL), then an acetonitrile solution (174 mg, 0.37 mmol, 2 mL) of tert-butyl (R)-3-(S)-1-tert-butoxy-3-(3-chlorosulfonyl)phenyl-1-oxopro p-2-yl)pyrrolidine-1-carboxylate was slowly added dropwise, TLC detection was carried out, after the raw materials reacted completely, rotary evaporation was carried out to remove pyridine, and then a crude product was purified through silica-gel column chromatography (eluent: n-hexane/ethyl acetate=3/1) to obtain 100 mg of 3,3'-(3-(S)-3-tert-butoxy-2-(R)-1-(tert-butoxycarbonyl)pyrrolid-3-yl)-3-oxopropyl)phenyl)su lfonyl)azadiyl)bis(ethane-2,1-diyl))bis(3,1-phenylenyl)bis(3-tert-butoxy)-3-oxoprop-1,2-diyl )(3R,3'R)-bis(pyrrolidine-1-carboxylate).

### Step F: synthesis of (2S,2'S)-3,3'(3-((S)-2-carboxyl-2-((R)-pyrrolid-3-yl)ethyl)phenyl) sulfonyl)azadiyl)bis(ethane-2,1-diyl))bis(3,1-phenylenyl)bis(2-(R)-pyrrolid-3-yl)propanoic acid)

3,3'-(3-(S)-3-tert-butoxy-2-(R)-1-(tert-butoxycarbonyl)pyrrolid-3-yl)-3-oxopropyl)phen yl)sulfonyl)azadiyl)bis(ethane-2,1-diyl))bis(3,1-phenylenyl)bis(3-tert-butoxy)-3-oxoprop-1, 2-diyl)(3R,3'R)-bis(pyrrolidine-1-carboxylate) (100 mg, 0.079 mmol) was dissolved in 1,4-dioxane (1.5 mL), and concentrated hydrochloric acid (0.5 mL) was added to react at 45°C overnight. After the reaction was ended, a solvent was removed through reduced pressure distillation, and a crude product was purified through preparative high performance liquid chromatography to obtain 20 mg of (2S,2'S)-3,3'(3-((S)-2-carboxyl-2-((R)-pyrrolid-3-yl)ethyl)phenyl)sulfonyl)azadiyl)bis(ethane-2,1-diyl))bis(3,1-phenylenyl)bis(2-(R)-pyrrolid-3-yl)propanoic acid). LCMS: RT = 1.51 min, [M-H]⁻ = 787.15. ¹H NMR (400 MHz, D₂O) δ 7.46 (dd, J = 18.4, 10.8 Hz, 4H), 7.15 (t, J = 7.6 Hz, 2H), 7.01 (d, J = 7.7 Hz, 2H), 6.91 (d, J = 7.6 Hz, 2H), 6.83 (s, 2H), 3.49 (ddd, J = 19.2, 11.8, 7.9 Hz, 3H), 3.40 - 3.25 (m, 7H), 3.22 - 3.11 (m, 3H), 3.04 - 2.87 (m, 4H), 2.80 - 2.74 (m, 4H), 2.73 - 2.57 (m, 8H), 2.45 (dd, J = 17.2, 8.6 Hz, 3H), 2.16 - 2.04 (m, 3H), 1.68 (p, J = 10.1 Hz, 3H).

### Example 22

### Synthesis of (2S,2'S)-3,3'-(3-((S)-2-carboxyl-2-(R)-pyrrolid-3-yl)ethyl)phenyl)sulfonyl )azadiyl)bis(methylenyl)bis(2,1-phenylenyl)bis(2-(R)-pyrrolid-3-yl)propanoic acid)

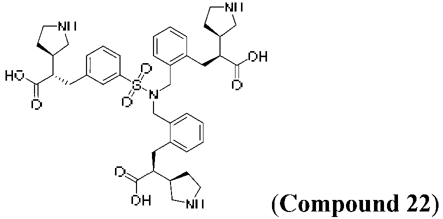

Preparation was performed with reference to the method for the compound 9. LCMS: RT = 1.51 min, [M-H]⁻ = 759.15.

### Example 23

### Synthesis of (S)-3-(3-(((3-(S)-2-carboxyl-2-((R)-pyrrolid-3-yl)ethyl)-N-(2-((S)2-carbo xyl-2-(R)pyrrolid-3-yl)ethyl)benzyl)phenyl)sulfonamido)methyl)phenyl)-2-((R)-pyrrolid-3-yl)propanoic acid

A specific synthetic route is as follows:

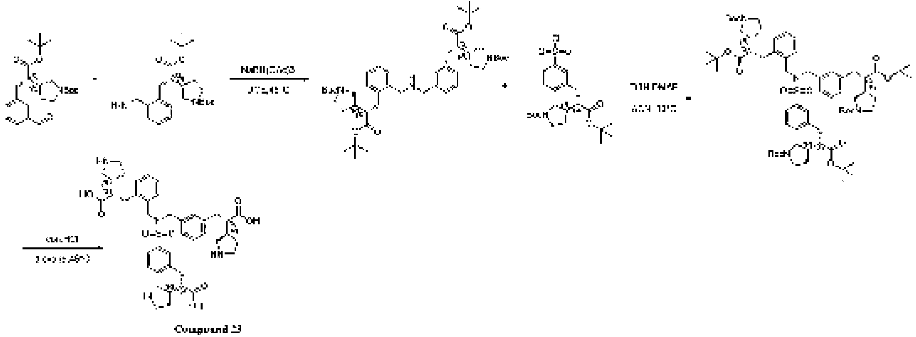

### Step A: tert-butyl (R)-3-((S)-1-(tert-butoxy)-3-(2-(((3-(S)-3-(tert-butoxy)-2-((R)-1-(tert-butoxycarbonyl)pyrrolid-3-yl)-3-oxopropyl)benzyl)amino)methyl)phenyl)-1-oxoprop-2-yl)p yrrolidine-1-formate

At a room temperature, tert-butyl (R)-3-((S)-1-(tert-butoxy)-3-(3-formylphenyl)-1-oxo prop-2-yl)pyrrolidine-1-formate (1.0 g, 2.48 mmol), tert-butyl ((R)-3-((S)-3-(2-(aminomethyl)phenyl)-1-(tert-butoxy)-1-oxoprop-2-yl)pyrrolidine-1-format e (1.2 g, 2.97 mmol) and 1,2-dichloroethane (20 mL) were added in sequence, the temperature was raised to 45°C, and then triacetoxyl sodium borohydride (1.8 mg, 8.68 mmol) was added in three batches to react at 45°C for 18 hours.

The reaction was ended, a saturated ammonium chloride aqueous solution (30 mL) was added for quenching, dichloromethane (20 mL × 2 times) was used for extraction, concentration was carried out to dryness, and a residue was purified through silica-gel column chromatography (eluent: ethyl acetate/n-hexane=1/1) to obtain 1.3 g of tert-butyl (R)-3-((S)-1-(tert-butoxy)-3-(2-(((3-(S)-3-(tert-butoxy)-2-((R)-1-(tert-butoxycarbonyl)pyrrol id-3-yl)-3-oxopropyl)benzyl)amino)methyl)phenyl)-1-oxoprop-2-yl)pyrrolidine-1-formate. LCMS: RT=2.03min, [M+H]⁺=792.08.

### Step B: tert-butyl-(R)-3-((S)-1-(tert-butoxy)-3-(2-(((3-((S)-3-(tert-butoxy)-2-((R)-1-(tert-butoxycarbonyl)pyrrolid-3-yl)-3-oxopropyl)-N-(3-((S)-3-(tert-butoxy)-2-((R)-1-(tert-bu toxycarbonyl)pyrrolid-3-yl)-3-oxopropyl))phenyl)sulfonylamino)methyl)phenyl)-1-oxoprop -2-yl)pyrrolidine-1-formate

At a room temperature, tert-butyl (R)-3-((S)-1-(tert-butoxy)-3-(2-(((3-(S)-3-(tert-butoxy )-2-((R)-1-(tert-butoxycarbonyl)pyrrolid-3-yl)-3-oxopropyl)benzyl)amino)methyl)phenyl)-1 -oxoprop-2-yl)pyrrolidine-1-formate (130 mg, 0.16 mmol), tert-butyl (R)-3-((S)-1-(tert-butoxy)-3-(3-(chlorosulfonyl)phenyl)-1-oxoprop-2-yl)pyrrolidine-1-forma te (95 mg, 0.20 mmol), acetonitrile (3 mL), triethylamine (32 mg, 0.32 mmol), and 4-dimethylamino pyridine (9 mg, 0.07 mmol) were added, nitrogen displacement was carried out, and the temperature was raised to 80°C to react for 2 hours.

The reaction was ended, tap water (10 mL) and ethyl acetate (10 mL) were added for extraction, concentration was carried out to dryness, and a residue was purified through silica-gel column chromatography (eluent: ethyl acetate/n-hexane=1/3) to obtain 110 mg of tert-butyl-(R)-3-((S)-1-(tert-butoxy)-3-(2-(((3-((S)-3-(tert-butoxy)-2-((R)-1-(tert-butoxycarb onyl)pyrrolid-3-yl)-3-oxopropyl)-N-(3-((S)-3-(tert-butoxy)-2-((R)-1-(tert-butoxycarbonyl)p yrrolid-3-yl)-3-oxopropyl))phenyl)sulfonylamino)methyl)phenyl)-1-oxoprop-2-yl)pyrrolidin e-1-formate.

### Step C: (S)-3-(3-(((3-(S)-2-carboxyl-2-((R)-pyrrolid-3-yl)ethyl)-N-(2-((S)2-carboxyl -2-(R)pyrrolid-3-yl)ethyl)benzyl)phenyl)sulfonamido)methyl)phenyl)-2-((R)-pyrrolid-3-yl)p ropanoic acid

At a room temperature, tert-butyl-(R)-3-((S)-1-(tert-butoxy)-3-(2-(((3-((S)-3-(tert-butox y)-2-((R)-1-(tert-butoxycarbonyl)pyrrolid-3-yl)-3-oxopropyl)-N-(3-((S)-3-(tert-butoxy)-2-(( R)-1-(tert-butoxycarbonyl)pyrrolid-3-yl)-3-oxopropyl))phenyl)sulfonylamino)methyl)pheny l)-1-oxoprop-2-yl)pyrrolidine-1-formate (110 mg, 0.09 mmol), dioxane (2.5 mL) and concentrated hydrochloric acid (0.5 mL) were added to react at 45°C for 4 hours.

The reaction was ended, concentration was carried out to dryness, and a residue was purified through preparative high performance liquid chromatography to obtain 44 mg of (S)-3-(3-(((3-(S)-2-carboxyl-2-((R)-pyrrolid-3-yl)ethyl)-N-(2-((S)2-carboxyl-2-(R)pyrrolid-3-yl)ethyl)benzyl)phenyl)sulfonamido)methyl)phenyl)-2-((R)-pyrrolid-3-yl)propanoic acid. LCMS: RT=1.49min, [M-H]⁻=759.19. ¹H NMR (400 MHz, DeuteRium Oxide) δ 7.71 (dq, J = 6.7, 4.5, 3.4 Hz, 1H), 7.63 (S, 1H), 7.54 (d, J = 6.0 Hz, 2H), 7.19 - 7.10 (m, 1H), 7.10 - 7.02 (m, 2H), 7.02 - 6.92 (m, 3H), 6.77 (d, J = 7.6 Hz, 1H), 6.50 (S, 1H), 4.40 - 4.24 (m, 2H), 4.14 (S, 2H), 3.60 - 3.29 (m, 6H), 3.26 - 3.12 (m, 3H), 3.09 - 2.86 (m, 5H), 2.82 - 2.36 (m, 10H), 2.11 (ttd, J = 12.4, 6.4, 5.7, 3.2 Hz, 3H), 1.80 - 1.58 (m, 3H).

### Example 24

### Synthesis of (S)-3-(3-(S)-2-carboxyl-2-(R)-pyrrolid-3-ethyl)-4-fluorobenzyl)-N-(2-(S)-2-carboxyl-2-(R)-pyrrolid-3-yl)ethyl)phenethyl)aminosulfonyl)phenyl)-2-(R)-pyrrolid-3-yl propanoic acid

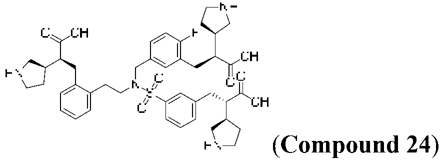

Preparation was performed with reference to the method for the compound 9. LCMS: RT=1.50 min, [M-H]⁻=791.16. NMR data: ¹H-NMR (400 MHz, Deuterium Oxide) δ 7.64 (d, J = 6.6 Hz, 1H), 7.58 (s, 1H), 7.49 (d, J = 6.7 Hz, 2H), 7.09 (m, 3H), 7.01 (d, J = 6.1 Hz, 1H), 6.96 (t, J = 9.1 Hz, 1H), 6.93 - 6.87 (m, 2H), 4.22 (m, 2H), 3.60 - 3.42 (m, 3H), 3.36 (t, J = 9.6 Hz, 3H), 3.30 - 3.13 (m, 5H), 3.07 - 2.86 (m, 5H), 2.85 - 2.72 (m, 2H), 2.67 (m, 7H), 2.47 (d, J = 16.1 Hz, 3H), 2.10 (d, J = 9.6 Hz, 3H), 1.69 (t, J = 10.5 Hz, 3H).

### Example 25

### Synthesis of (S)-3-(3-(N-(3-((S)-2-carboxyl-2-((R)-pyrrolid-3-yl)etyhl)-2-fluorobenzyl) -N-(2-(S)2-carboxyl-2-((R)-pyrrolid-3-yl)ethyl)phenethyl)aminosulfonyl)phenyl)-2-(((R)-py rrolid-3-yl)propanoic acid

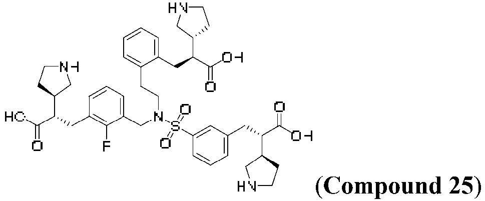

Preparation was performed with reference to the method for the compound 9. LCMS: RT=1.49min, [M-H]⁻=791.19. ¹H NMR (400 MHz, DeuteRium Oxide) δ 7.55 (d, J = 7.0 Hz, 1H), 7.51 - 7.38 (m, 3H), 7.16 - 7.03 (m, 5H), 6.99 (t, J = 7.5 Hz, 1H), 6.92 (d, J = 6.2 Hz, 1H), 4.33 (S, 2H), 3.50 - 3.39 (m, 3H), 3.39 - 3.24 (m, 5H), 3.16 (dd, J = 17.2, 10.8 Hz, 3H), 2.95 - 2.81 (m, 3H), 2.80 - 2.74 (m, 2H), 2.67 (dd, J = 17.2, 6.9 Hz, 6H), 2.49 - 2.32 (m, 6H), 2.04 (S, 3H), 1.73 - 1.59 (m, 3H).

### Example 26

### Synthesis of (S)-3-(3-(5-(S)-2-carboxyl-2-(R)-pyrrolid-3-yl)ethyl)-2-fluorobenzyl)-N-( 2-((S)-2-carboxyl-2-((R)-pyrrolid-3-yl)ethyl)phenethyl)aminosulfonyl)phenyl)-2-(R)-pyrroli d-3-yl)propanoic acid

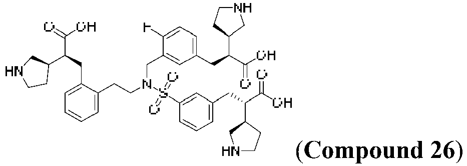

Preparation was performed with reference to the method for the compound 9. LCMS: RT = 1.54min, [M+H]⁺ = 793.16. ¹H NMR (400 MHz, D₂O) 7.55 (dd, J = 5.6, 3.6 Hz, 1H), 7.49 (s, 1H), 7.47 - 7.38 (m, 2H), 7.05 (h, J = 4.8 Hz, 4H), 6.99 - 6.94 (m, 1H), 6.93 - 6.85 (m, 2H), 4.30 (s, 2H), 3.62 - 3.22 (m, 8H), 3.22 - 3.08 (m, 3H), 2.96 - 2.48 (m, 11H), 2.44 - 2.23 (m, 6H), 2.10 - 1.93 (m, 3H), 1.68 - 1.57 (m, 3H).

### Example 27

### Synthesis of (S)-3-(3-(N-(5-(S)-2-carboxyl-2-(R)-pyrrolid-3-yl)ethyl)-2,4-difluorobenz yl)-N-(2-(S)-2-carboxyl-2-((R)-pyrrolid-3-yl)ethyl)phenethyl)aminosulfonyl)phenyl)-2-(R)-pyrrolid-3-yl propanoic acid

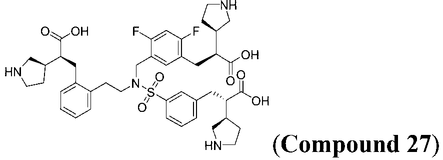

Preparation was performed with reference to the method for the compound 9. LCMS: RT=1.55 min, [M-H]⁻=809.12. NMR data: ¹H-NMR (400 MHz, Deuterium Oxide) δ 8.27 (s, 2H), 7.57 (d, J = 7.0 Hz, 1H), 7.52 (s, 1H), 7.45 (d, J = 7.5 Hz, 2H), 7.11 - 7.02 (m, 3H), 6.97 - 6.88 (m, 2H), 6.75 (t, J = 10.0 Hz, 1H), 4.24 (d, J = 3.1 Hz, 2H), 3.48 (m, 3H), 3.33 (m, 5H), 3.25 - 3.10 (m, 3H), 3.01 - 2.88 (m, 3H), 2.81 (d, J = 7.4 Hz, 2H), 2.68 (m, 6H), 2.55 - 2.33 (m, 6H), 2.16 - 1.97 (m, 3H), 1.76 - 1.56 (m, 3H).

### Example 28

### Synthesis of (S)-3-(3-(2-(S)-2-carboxyl-2-(R)-pyrrolid-3-yl)ethyl)-5-fluorophenethyl)-N -(3-((S)-2-carboxyl-2-(R)-pyrrolid-3-yl)ethyl)benzyl)aminosulfonyl)phenyl)-2-(R)-pyrrolid-3-yl propanoic acid

A specific synthetic route is as follows:

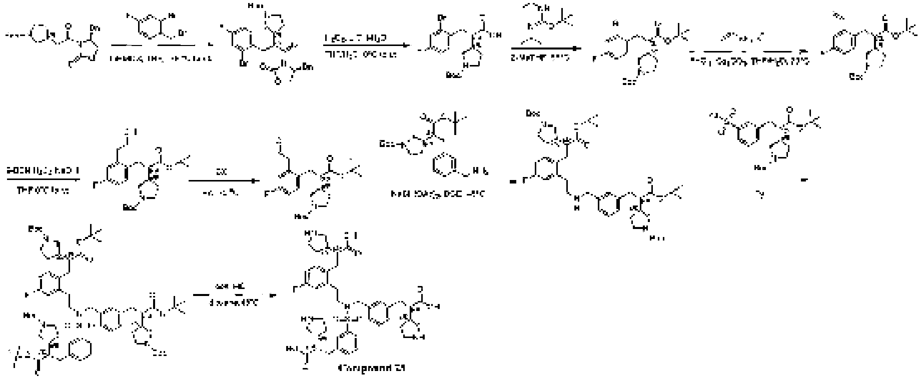

### Step A: synthesis of tert-butyl (R)-3-(S)-1-(S)-4-benzyl-2-oxoxazolidin-3-yl)-3-(2-bro mo-4-fluorophenyl)-1-oxoprop-2-yl)pyrrolidine-1-carboxylate

At -78°C, a tetrahydrofuran solution (67 mL, 1.0 mol/L) of lithium bis(trimethylsilyl)amide was slowly added dropwise into a tetrahydrofuran (200 mL) solution of tert-butyl (R)-3-(2-(S)-4-benzyl-2-oxoxazolidin-3-yl)-2-oxoethyl)pyrrolidine-1-carboxyl ate (20 g, 51.48 mmol). After reaction for 0.5 hours, a tetrahydrofuran solution (30 mL) of 2-bromo-1-bromomethyl-4-fluorobenzene (15 g, 55.99 mmol) was slowly added, and then the temperature was raised to a room temperature naturally to react overnight.

After the reaction was monitored to be ended via LC-MS, a saturated ammonium chloride aqueous solution was added for quenching, ethyl acetate (200 mL ×2 times) was used for extraction, combined organic phases were washed using a saturated saline solution (50 mL×2 times) first and then dried using anhydrous sodium sulfate, vacuum concentration was carried out to obtain a residue, and the residue was purified through silica-gel column chromatography (eluent: n-hexane/ethyl acetate=4/1) to obtain 17 g of tert-butyl (R)-3-(S)-1-(S)-4-benzyl-2-oxoxazolidin-3-yl)-3-(2-bromo-4-fluorophenyl)-1-oxoprop-2-yl) pyrrolidine-1-carboxylate. LC-MS: RT = 2.29 min, [M+H-Boc]⁺ = 475.02.

### Step B: synthesis of (S)-3-(2-bromo-4-fluorophenyl)-2-(R)-1-tert-butoxycarbonyl)pyrrolid-3-yl)propanoic acid

Under an ice water bath, tert-butyl (R)-3-(S)-1-(S)-4-benzyl-2-oxoxazolidin-3-yl)-3-(2-bromo-4-fluorophenyl)-1-oxoprop-2-yl)pyrrolidine-1-carboxylate (15 g, 26.13 mmol) was dissolved in tetrahydrofuran (150 mL), 30% hydrogen peroxide (6 g, 52.26 mmol) was added, after 0.5 hours, an aqueous solution (15 mL) of lithium hydroxide monohydrate (2.2 g, 52.26 mmol) was added, and a temperature was raised to a room temperature to react for 3 hours.

After the reaction was monitored to be ended via LC-MS, an excessive sodium hydrogen sulfate saturated solution was slowly added into a reaction liquid to quench the reaction, stirring was carried out for 10 minutes, then a citric acid saturated aqueous solution was added to adjust pH to 5, ethyl acetate (100 mL × 2 times) was used for extraction, combined organic phases were washed using a saturated saline solution (50 mL× 2 times) first and then dried using anhydrous sodium sulfate, vacuum concentration was carried out to obtain a residue, and the residue was purified through silica-gel column chromatography (eluent: n-hexane/ethyl acetate=2/1) to obtain 7.8 g of (S)-3-(2-bromo-4-fluorophenyl)-2-(R)-1-tert-butoxycarbonyl)pyrrolid-3-yl)propanoic acid. LC-MS: RT = 2.29 min, [M-H]⁻ = 414.04.

### Step C: synthesis of tert-butyl (R)-3-(S)-3-(2-bromo-4-fluorophenyl)-1-tert-butoxy-1-oxoprop-2-yl)pyrrolidine-1-carboxylate

At a room temperature, (S)-3-(2-bromo-4-fluorophenyl)-2-(R)-1-tert-butoxycarbonyl)pyrrolid-3-yl)propanoic acid (7.8 g, 18.8 mmol) and tert-butyl (Z)-N,N'-diisopropylcarbamate (11.3 g, 56.4 mmol) were dissolved in 2-methyltetrahydrofuran (80 mL), nitrogen displacement was carried out 3 times, and the temperature was raised to 65°C to react overnight.

After the reaction was monitored to be ended via LC-MS, filtering was carried out, a filter cake was washed with ethyl acetate, a filtrate was diluted with ethyl acetate, combined organic phases were washed using a saturated saline solution (100 mL ×2 times) first and then dried using anhydrous sodium sulfate, vacuum concentration was carried out to obtain a residue, and the residue was purified through silica-gel column chromatography (eluent: n-hexane/ethyl acetate=9/1) to obtain 6.7 g of tert-butyl (R)-3-(S)-3-(2-bromo-4-fluorophen yl)-1-tert-butoxy-1-oxoprop-2-yl)pyrrolidine-1-carboxylate. LC-MS: RT = 2.29 min, [M+H-Boc]⁺ = 372.10.

### Step D: synthesis of tert-butyl (R)-3-(S)-1-tert-butoxy-3-(4-fluoro-2-vinylphenyl)-1-oxo prop-2-yl)pyrrolidine-1-carboxylate

At a room temperature, tert-butyl (R)-3-(S)-3-(2-bromo-4-fluorophenyl)-1-tert-butoxy-1-oxoprop-2-yl)pyrrolidine-1-carboxylate (6.7 g, 14.2 mmol), potassium vinyltrifluoroborate (2.86 g, 21.3 mmol), palladium chloride (83 mg, 0.71 mmol), triphenylphosphine (370 mg, 1.4 mmol) and cesium carbonate (13.87 g, 42.67 mmol) were dissolved in tetrahydrofuran/water (60 mL/7 mL), N₂ displacement was carried out three times, and then the temperature was raised to 75°C to react overnight.

After the reaction was monitored to be ended via TLC, the temperature was lowered to the room temperature, water (10 mL) was added for dilution, then ethyl acetate (100 mL, 2 times) was used for extraction, combined organic phases were washed using a saturated saline solution (50 mL × 2 times) first and then dried using anhydrous sodium sulfate, vacuum concentration was carried out to obtain a residue, and the residue was purified through silica-gel column chromatography (eluent: n-hexane/ethyl acetate=4/1) to obtain 4.9 g of tert-butyl (R)-3-(S)-1-tert-butoxy-3-(4-fluoro-2-vinylphenyl)-1-oxoprop-2-yl)pyrrolidin e-1-carboxylate.

### Step E: synthesis of tert-butyl (R)-3-(S)-1-tert-butoxy-3-(4-fluoro-2-(2-hydroxyethyl) phenyl)-1-oxoprop-2-yl)pyrrolidine-1-carboxylate

Under an ice water bath, tert-butyl (R)-3-(S)-1-tert-butoxy-3-(4-fluoro-2-vinylphenyl) -1-oxoprop-2-yl)pyrrolidine-1-carboxylate (3 g, 7.15 mmol) was dissolved in tetrahydrofuran (30 mL), a 9-borabicyclo[3.3.1]nonane tetrahydrofuran solution (28.6 mL, 0.5 mol/L) was added, stirring was carried out overnight with a temperature raised to a room temperature, and after methanol (10 mL) was added and stirred for ten minutes, a 30% hydrogen peroxide aqueous solution (4.87 g, 42.9 mmol) and an aqueous solution (10 mL) of sodium hydroxide (2.72 g, 67.9 mmol) were added to continue the reaction for 1 hour.

After the reaction was monitored to be ended via LC-MS, an excessive sodium hydrogen sulfate aqueous solution was slowly poured into the system to quench the reaction, stirring was carried out for 30 minutes, a mixed liquor was extracted with ethyl acetate (80 mL × 2 times), combined organic phases were washed using a saturated saline solution (50 mL × 2 times) first and then dried using anhydrous sodium sulfate, vacuum concentration was carried out to obtain a residue, and the residue was purified through silica-gel column chromatography (eluent: n-hexane/ethyl acetate=3/1) to obtain 1.2 g of tert-butyl (R)-3-(S)-1-tert-butoxy-3-(4-fluoro-2-(2-hydroxyethyl)phenyl)-1-oxoprop-2-yl)pyrrolidine-1-carboxylate. LC-MS: RT = 2.216min, [M+H-Boc-tert-butyl]⁺ = 282.05.

### Step F: synthesis of tert-butyl (R)-3-(S)-1-tert-butoxy-3-(4-fluoro-2-(2-oxoethyl)phenyl )-1-oxoprop-2-yl)pyrrolidine-1-carboxylate

Tert-butyl (R)-3-(S)-1-tert-butoxy-3-(4-fluoro-2-(2-hydroxyethyl)phenyl)-1-oxoprop-2-yl)pyrrolidine-1-carboxylate (1.2 g, 2.75 mmol) was dissolved in ethyl acetate (50 mL), 2-iodoxybenzoic acid (0.85 g, 3.0 mmol) was added, and stirring was carried out for 6 hours with a temperature raised to 70°C.

After the reaction was monitored to be ended via LC-MS, filtering was carried out, a filter cake was removed and flushed with ethyl acetate, organic phases were combined, vacuum concentration was carried out to obtain a residue, and the residue was purified through silica-gel column chromatography (eluent: n-hexane/ethyl acetate=3/1) to obtain 490 mg of tert-butyl (R)-3-(S)-1-tert-butoxy-3-(4-fluoro-2-(2-oxoethyl)phenyl)-1-oxoprop-2-yl)pyrrolidine-1-carboxylate. LC-MS: RT = 2.15 min, [M+H-Boc-tert-butyl]⁺ = 280.08.

### Step G: synthesis of tert-butyl-3-(S)-1-tert-butoxy)-3-(2-(3-(S)-3-tert-butoxy-2-(R)-1-tert-butoxycarbonyl)pyrrolid-3-yl)-3-oxopropyl)benzyl)amino)ethyl)-4-fluorophenyl)-1-oxo prop-2-yl)pyrrolidine-1-carboxylate

At a room temperature, tert-butyl (R)-3-(S)-1-tert-butoxy-3-(4-fluoro-2-(2-oxoethyl)phe nyl)-1-oxoprop-2-yl)pyrrolidine-1-carboxylate (390 mg, 0.9 mmol) and tert-butyl (R)-3-(S)-3-(3-aminomethyl)phenyl)-1-tert-butoxy-1-oxoprop-2-yl)pyrrolidine-1-carboxylat e (362 mg, 0.9 mmol) were dissolved in DCE (8 mL), triacetoxyl sodium borohydride (665 mg, 3.15 mmol) was added in batches, the temperature was raised to 45°C, and stirring was continued for 16 hours.

After the reaction was monitored to be complete via LC-MS, an aqueous solution (1 mL) was added to quench the reaction, and an obtained crude product was purified through silica-gel column chromatography (eluent: ethyl acetate/n-hexane=1/5). 250 mg of tert-butyl-3-(S)-1-tert-butoxy)-3-(2-(3-(S)-3-tert-butoxy-2-(R)-1-tert-butoxycarbonyl)pyrroli d-3-yl)-3-oxopropyl)benzyl)amino)ethyl)-4-fluorophenyl)-1-oxoprop-2-yl)pyrrolidine-1-car boxylate was obtained. LC-MS: RT = 2.02 min, [M+H]⁺ =824.51.

### Step H: synthesis of (R)-3-(S)-1-tert-butoxy)-3-(2-(3-(S)-3-tert-butoxy)-2-((R)-1-(tert-butoxycarbonyl)pyrrolid-3-yl)-3-oxopropyl)-N-(3-(S)-3-tert-butoxy)-2-((R)-1-(tert-butoxyca rbonyl)pyrrolid-3-yl)-3-oxopropyl)benzyl)phenyl)sulfonamido)ethyl)-4-fluorophenyl)-1-oxo prop-2-yl)pyrrolidine-1-carboxylate

At a room temperature, tert-butyl-3-(S)-1-tert-butoxy)-3-(2-(3-(S)-3-tert-butoxy-2-(R)-1-tert-butoxycarbonyl)pyrrolid-3-yl)-3-oxopropyl)benzyl)amino)ethyl)-4-fluorophenyl)-1-o xoprop-2-yl)pyrrolidine-1-carboxylate (250 mg, 0.30 mmol) was dissolved in pyridine (5 mL), and an acetonitrile solution (2 mL) of tert-butyl (R)-3-(S)-1-tert-butoxy-3-(3-chlorosulfonyl)phenyl-1-oxoprop-2-yl)pyrrolidine-1-carboxylat e (172 mg, 0.36 mmol) was slowly added to react at the room temperature for 0.5 hours.

After the reaction was detected to be ended through TLC, ethyl acetate (10 mL× 2 times) was used for extraction, combined organic phases were washed using a saturated saline solution (5 mL× 2 times) first and then dried using anhydrous sodium sulfate, vacuum concentration was carried out to obtain a residue, and the residue was purified through silica-gel column chromatography (eluent: n-hexane/ethyl acetate=3/1) to obtain 150 mg of (R)-3-(S)-1-tert-butoxy)-3-(2-(3-(S)-3-tert-butoxy)-2-((R)-1-(tert-butoxycarbonyl)pyrrolid-3 -yl)-3-oxopropyl)-N-(3-(S)-3-tert-butoxy)-2-((R)-1-(tert-butoxycarbonyl)pyrrolid-3-yl)-3-ox opropyl)benzyl)phenyl)sulfonamido)ethyl)-4-fluorophenyl)-1-oxoprop-2-yl)pyrrolidine-1-ca rboxylate.

### Step I: synthesis of (S)-3-(3-(2-(S)-2-carboxyl-2-(R)-pyrrolid-3-yl)ethyl)-5-fluorophene thyl)-N-(3-((S)-2-carboxyl-2-(R)-pyrrolid-3-yl)ethyl)benzyl)aminosulfonyl)phenyl)-2-(R)-p yrrolid-3-yl propanoic acid

At a room temperature, (R)-3-(S)-1-tert-butoxy)-3-(2-(3-(S)-3-tert-butoxy)-2-((R)-1-(t ert-butoxycarbonyl)pyrrolid-3-yl)-3-oxopropyl)-N-(3-(S)-3-tert-butoxy)-2-((R)-1-(tert-butox ycarbonyl)pyrrolid-3-yl)-3-oxopropyl)benzyl)phenyl)sulfonamido)ethyl)-4-fluorophenyl)-1-oxoprop-2-yl)pyrrolidine-1-carboxylate (150 mg, 0.12 mmol) was dissolved in a 1,4-dioxane solution (3 mL), concentrated hydrochloric acid (0.2 mL) was added, and the temperature was raised to 45°C to react for 4 hours.

The reaction was monitored to be complete via LC-MS, vacuum concentration was carried out, and a residue was purified through preparative high performance liquid chromatography to obtain 34 mg of (S)-3-(3-(2-(S)-2-carboxyl-2-(R)-pyrrolid-3-yl)ethyl)-5-fluorophenethyl)-N-(3-((S)-2-carboxyl-2-(R)-pyrrolid-3-yl)ethyl)benzyl)aminosulfonyl)phe nyl)-2-(R)-pyrrolid-3-yl propanoic acid. LCMS: RT=1.56min, [M-H]⁻=791.12. NMR data: ¹H-NMR (400 MHz, Deuterium Oxide) δ 7.68 - 7.59 (m, 1H), 7.55 (s, 1H), 7.46 (d, J = 6.3 Hz, 2H), 7.17 (t, J = 7.6 Hz, 1H), 7.07 (d, J = 7.7 Hz, 1H), 6.99 (t, J = 7.6 Hz, 2H), 6.89 (s, 1H), 6.79 (m, 1H), 6.66 (m 1H), 4.24 (s, 2H), 3.49 - 3.26 (m, 8H), 3.23 - 3.09 (m, 3H), 2.95 - 2.87 (m, 1H), 2.80 (m, 4H), 2.75 - 2.62 (m, 3H), 2.53 (m, 2H), 2.48 - 2.28 (m, 6H), 2.22 (m, 1H), 2.03 (m, 3H), 1.76 - 1.53 (m, 3H).

### Example 29

### Synthesis of (2S,2'S)-3,3'(5-((S)-2-carboxyl-2-(R)-pyrrolid-3-yl)ethyl)-2-fluorophenyl sulfonyl)azadiyl)dimethylenyl)bis(3,1-phenylenyl)bis(2-(R)-pyrrolid-3-yl)propanoic acid)

A specific synthetic route is as follows:

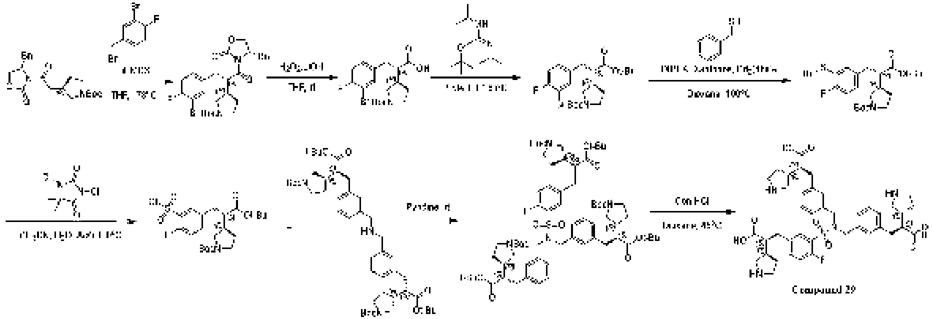

### Step A: synthesis of tert-butyl (3R)-3-(2S)-1-(4-benzyl-2-oxoxazolidin-3-yl)-3-(3-brom o-4-fluorophenyl)-1-oxoprop-2-yl)pyrrolidine-1-carboxylate

At -78°C, lithium bis(trimethylsilyl)amide (19 mL, 19 mmol) was slowly added dropwise into a tetrahydrofuran (60 mL) solution of tert-butyl (R)-3-(2-((S)-4-benzyl-2-oxoxazolidin-3-yl)-2-oxoethyl)pyrrolidine-1-formate (6.0 g, 15.45 mmol), after stirring at -78°C for 30 minutes, a tetrahydrofuran (20 mL) solution of 2-bromo-4-bromomethyl-1-fluorobenzene (4.97 g, 19 mmol) was slowly added into the system, and then the temperature was naturally raised to a room temperature to react overnight. After the reaction was monitored to be ended via TLC, a saturated ammonium chloride aqueous solution was added for quenching, ethyl acetate (100 mL ×2 times) was used for extraction, combined organic phases were washed using a saturated saline solution (100 mL×2 times) first and then dried using anhydrous sodium sulfate, vacuum concentration was carried out to obtain a residue, and the residue was purified through silica-gel column chromatography (eluent: ethyl acetate/n-hexane=1/3) to obtain 3.1 g of tert-butyl (3R)-3-(2S)-1-(4-benzyl-2-oxoxazolidin-3-yl)-3-(3-bromo-4-fluorophenyl)-1-oxoprop-2-yl)pyrrolidine-1-carboxylate. LCMS:RT = 2.25 min, [M+H-Boc]⁺ = 475.01.

### Step B: synthesis of (S)-3-(3-bromo-4-fluorophenyl)-2-(R)-1-tert-butoxycarbonyl)pyrrolid-3-yl)propanoic acid

Under an ice water bath, hydrogen peroxide (1.1 g, 30%, 9.90 mmol) was added into a tetrahydrofuran (40 mL) solution of tert-butyl (3R)-3-(2S)-1-(4-benzyl-2-oxoxazolidin-3-yl)-3-(3-bromo-4-fluorophenyl)-1-oxoprop-2-yl) pyrrolidine-1-carboxylate (3.8 g, 6.60 mmol) to react for 30 minutes, then an aqueous solution (10 mL) of lithium hydroxide monohydrate (237 mg, 9.90 mmol) was added, and a temperature was raised to a room temperature to react for 3 hours. After the reaction was ended, an excessive sodium hydrogen sulfate saturated solution was slowly added into a reaction liquid to quench the reaction, stirring was carried out for 10 minutes, then a citric acid saturated aqueous solution was added to adjust pH to 3, ethyl acetate (50 mL × 2 times) was used for extraction, combined organic phases were washed using a saturated saline solution (200 mL × 2 times) first and then dried using anhydrous sodium sulfate, vacuum concentration was carried out to obtain a residue, and the residue was purified through silica-gel column chromatography (eluent: ethyl acetate/n-hexane=1/3) to obtain 2.2 g of (S)-3-(3-bromo-4-fluorophenyl)-2-(R)-1-tert-butoxycarbonyl)pyrrolid-3-yl)propanoic acid. LCMS: RT = 2.06 min, [M-H]⁻ = 416.00.

### Step C: synthesis of tert-butyl (R)-3-(S)-3-(3-bromo-4-fluorophenyl)-1-tert-butoxy-1-oxoprop-2-yl)pyrrolidine-1-carboxylate

At a room temperature, O-tert-butyl-N,N'-diisopropyl isourea (3.18 g, 15.85 mmol) was added into a 2-methyl tetrahydrofuran (20 mL) solution of (S)-3-(3-bromo-4-fluorophenyl)-2-(R)-1-tert-butoxycarbonyl)pyrrolid-3-yl)propanoic acid (2.2 g, 5.28 mmol), nitrogen displacement was carried out 3 times, and the temperature was raised to 65°C to react overnight. After the reaction was monitored to be ended via TLC, a filter cake was washed with ethyl acetate, a filtrate was diluted with ethyl acetate, combined organic phases were washed using a saturated saline solution (70 mL ×2 times) first and then dried using anhydrous sodium sulfate, vacuum concentration was carried out to obtain a residue, and the residue was purified through silica-gel column chromatography (eluent: ethyl acetate/n-hexane=1/7) to obtain 2.1 g of tert-butyl (R)-3-(S)-3-(3-bromo-4-fluorophen yl)-1-tert-butoxy-1-oxoprop-2-yl)pyrrolidine-1-carboxylate. LCMS:RT = 2.36 min, [M+H-Boc]⁺ = 371.98.

### Step D: synthesis of tert-butyl (R)-3-(S)-3-(3-benzylthio)-4-fluorophenyl)-1-tert-butoxy -1-oxoprop-2-yl)pyrrolidine-1-carboxylate

At a room temperature, tert-butyl (R)-3-(S)-3-(3-bromo-4-fluorophenyl)-1-tert-butoxy-1-oxoprop-2-yl)pyrrolidine-1-carboxylate (2.10 g, 4.62 mmol), benzyl mercaptane (689 mg, 5.55 mmol), N,N-diisopropylethylamine (1.19 g, 9.24 mmol), 4,5-bis(diphenylphosphino)-9,9-dimethyl xanthene (267 mg, 0.46 mmol), and tris(dibenzylideneacetone)dipalladium (211 mg, 0.23 mmol) were dissolved in dioxane, nitrogen was displaced, and under nitrogen protection, a reaction was carried out overnight at 100°C. After the reaction was ended, the temperature was lowered, suction filtration was carried out, a filtrate was subjected to vacuum concentration to obtain a residue, and the residue was purified through silica-gel column chromatography (eluent: ethyl acetate/n-hexane=1/7) to obtain 2.1 g of tert-butyl (R)-3-(S)-3-(3-benzylthio)-4-fluoropheny 1)-1-tert-butoxy-1-oxoprop-2-yl)pyrrolidine-1-carboxylate. LCMS:RT = 2.43 min, [M+H-Boc]⁺ = 398.15.

### Step E: synthesis of tert-butyl (R)-3-(S)-1-tert-butoxy-3-(3-chlorosulfonyl)-4-fluorophe nyl)-1-oxoprop-2-yl)pyrrolidine-1-carboxylate

Under an ice bath, tert-butyl (R)-3-(S)-3-(3-benzylthio)-4-fluorophenyl)-1-tert-butoxy-1-oxoprop-2-yl)pyrrolidine-1-carboxylate (2.10 g, 4.22 mmol) was dissolved in acetonitrile (30 mL), then acetic acid (1.5 mL) and water (1.5 mL) were added, then a 1,3-dichloro-5,5-dimethylhydantoin acetonitrile solution (1.66 g, 8.44 mmol, 10 mL) was added dropwise, and TLC monitoring was performed. After the reaction was ended, water was added into the system, then ethyl acetate (100 mL× 2 times) was used for extraction, organic phases were combined, concentration was carried out, and a residue was purified through silica-gel column chromatography (eluent: ethyl acetate/n-hexane=1/5) to obtain 1.6 g of tert-butyl (R)-3-(S)-1-tert-butoxy-3-(3-chlorosulfonyl)-4-fluorophenyl)-1-oxoprop-2-yl)pyrrolidine-1-carboxylate.

### Step F: synthesis of 3,3'-((2S,2'S)-(3-((S)-3-tert-butoxy)-2-(R)-1-(tert-butoxycarbonyl) pyrrolid-3-yl)-3-oxopropyl)-4-fluorophenyl)sulfonyl)azadiyl)dimethylenyl)bis(3,1-phenylen yl)bis(3-tert-butoxy)-3-oxoprop-1,2-diyl)(3R,3'R)-bis(pyrrolidine-1-carboxylate)

At a room temperature, 3,3'-((2S,2'S)-(azadiyl dimethylenyl)bis(3,1-phenylenyl)bis(3-tert-butoxy)-3-oxoprop-1,2-diyl)(3R,3'R)-bis(pyrroli dine-1-carboxylate) (250 mg, 0.32 mmol) was dissolved in anhydrous pyridine (2 mL), then an acetonitrile solution (187 mg, 0.38 mmol, 2 mL) of tert-butyl (R)-3-(S)-1-tert-butoxy-3-(3-chlorosulfonyl)-4-fluorophenyl-1-oxoprop-2-yl)pyrrolidine-1-c arboxylate was slowly added dropwise, TLC detection was carried out, after the raw materials reacted completely, rotary evaporation was carried out to remove pyridine, and then a crude product was purified through silica-gel column chromatography (eluent: n-hexane/ethyl acetate=3/1) to obtain 60 mg of 3,3'-((2S,2'S)-(3-((S)-3-tert-butoxy)-2-(R)-1-(tert-butoxycarbonyl)pyrrolid-3-yl)-3-oxopropy 1)-4-fluorophenyl)sulfonyl)azadiyl)dimethylenyl)bis(3,1-phenylenyl)bis(3-tert-butoxy)-3-ox oprop-1,2-diyl)(3R,3'R)-bis(pyrrolidine-1-carboxylate).

### Step G: synthesis of (2S,2'S)-3,3'(3-((S)-2-carboxyl-2-(R)-pyrrolid-3-yl)ethyl)-4-fluoro phenylsulfonyl)azadiyl)dimethylenyl)bis(3,1-phenylenyl)bis(2-(R)-pyrrolid-3-yl)propanoic acid)

3,3'-((2S,2'S)-(3-((S)-3 -tert-butoxy)-2-(R)- I -(tert-butoxycarbonyl)pyrrolid-3-yl)-3-oxop ropyl)-4-fluorophenyl)sulfonyl)azadiyl)dimethylenyl)bis(3,1-phenylenyl)bis(3-tert-butoxy)-3-oxoprop-1,2-diyl)(3R,3'R)-bis(pyrrolidine-1-carboxylate) (60 mg, 0.048 mmol) was dissolved in 1,4-dioxane (1.5 mL), and concentrated hydrochloric acid (0.5 mL) was added to react at 45°C overnight. After the reaction was ended, a solvent was removed through reduced pressure distillation, and a crude product was purified through preparative high performance liquid chromatography to obtain 18 mg of (2S,2'S)-3,3'(3-((S)-2-carboxyl-2-(R)-pyrrolid-3-yl)ethyl)-4-fluorophenylsulfonyl)azadiyl)di methylenyl)bis(3,1-phenylenyl)bis(2-(R)-pyrrolid-3-yl)propanoic acid). LCMS: RT = 1.53 min, [M-H]⁻ = 777.12. ¹H NMR (400 MHz, D₂O) δ 7.55 - 7.50 (m, 1H), 7.47 - 7.41 (m, 1H), 7.22 - 7.09 (m, 3H), 7.02 (d, J = 7.6 Hz, 2H), 6.90 (d, J = 7.6 Hz, 2H), 6.80 (s, 2H), 4.33 (s, 4H), 3.44 (dd, J = 11.7, 6.9 Hz, 1H), 3.32 (ddd, J = 17.0, 10.1, 5.1 Hz, 5H), 3.13 (td, J = 10.7, 7.3 Hz, 3H), 2.95 - 2.87 (m, 1H), 2.84 - 2.76 (m, 2H), 2.73 (d, J = 8.9 Hz, 2H), 2.65 (dd, J = 13.6, 8.9 Hz, 2H), 2.56 (dd, J = 13.5, 4.3 Hz, 2H), 2.45 - 2.26 (m, 6H), 2.08 - 1.97 (m, 3H), 1.64 (dq, J = 12.4, 8.9 Hz, 3H).

### Example 30

### Synthesis of (S)-3-(3-(S)-2-carboxyl-2-(R)-pyrrolid-3-yl)ethyl)-2,4-difluorobenzyl)-N-(3-(S)-2-carboxyl-2-(R)-pyrrolid-3-yl)ethyl)benzyl)aminosulfonyl)phenyl)-2-((R)-pyrrolid-3 -yl)propanoic acid

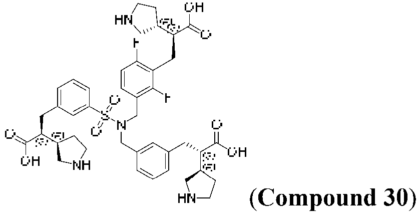

Preparation was performed with reference to the method for the compound 9. LCMS: RT = 1.53 min, [M-H]⁻ = 795.12.

### Example 31

### Synthesis of (2S,2'S)-3,3'-((3-((S)-2-carboxyl-2-(R)-pyrrolid-3-yl)ethyl)phenyl)sulfonyl )azadiyl)bis(methylenyl))bis(2,6-difluoro-3,1-phenylenyl)bis(2-(R)-pyrrolid-3-yl)propanoic acid)

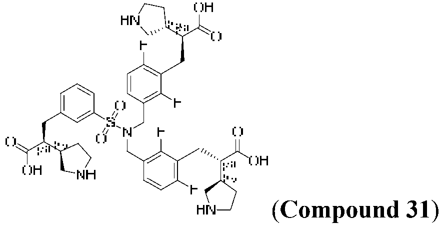

Preparation was performed with reference to the method for the compound 9. LCMS: RT = 1.53 min, [M-H]⁻ = 831.12.

### Example 32

### Synthesis of (S)-3-(3-(S)-2-carboxyl-2-(R)-pyrrolid-3-yl)ethyl)-2,6-difluorobenzyl)-N-( 3-(S)-2-carboxyl-2-(R)-pyrrolid-3-yl)ethyl)benzyl)aminosulfonyl)phenyl)-2-(R)-pyrrolid-3-yl propanoic acid

A specific synthetic route is as follows:

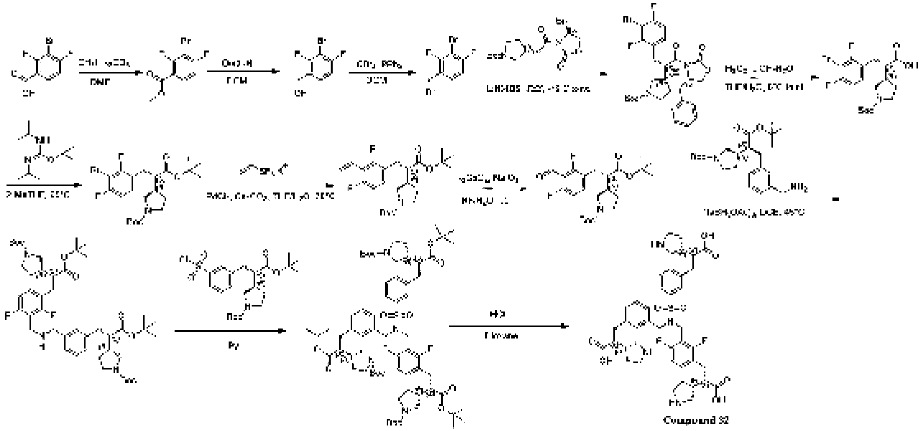

### Step A: synthesis of methyl 3-bromo-2,4-difluorobenzoate

At a room temperature, 3-bromo-2,4-difluorobenzoate (5 g, 21.19 mmol) was dissolved in an N,N-dimethylformamide solution (50 mL), potassium carbonate (5.86 g, 42.4 mmol) was added, after the temperature was lowered to zero degrees Celsius, iodomethane (3.59 g, 25.3 mmol) was slowly added, and the temperature was restored to the room temperature to react for 1 hour.

After the reaction was detected to be complete via TLC, water was added for dilution, ethyl acetate (100 mL × 2 times) was used for extraction, combined organic phases were washed using a saturated saline solution (50 mL × 2 times) first and then dried using anhydrous sodium sulfate, vacuum concentration was carried out to obtain a residue, and the residue was purified through silica-gel column chromatography (eluent: n-hexane/ethyl acetate=4/1) to obtain 5.1 g of white solid methyl 3-bromo-2,4-difluorobenzoate (yield: 96.7%).

### Step B: synthesis of 3-bromo-2,4-difluorobenzylalcohol

Under an ice water bath, an n-hexane solution (41 mL, 1.0 mol/L) of diisobutylaluminium hydride was slowly added dropwise into a dichloromethane (50 mL) solution of methyl 3-bromo-2,4-difluorobenzoate (5.1 g, 20.4 mmol), and a temperature was restored to a room temperature to react for 2 hours.

After the reaction was monitored to be ended via LC-MS, a 1 M/L hydrochloric acid solution (5 mL) was added for quenching, ethyl acetate (200 mL × 2 times) was used for extraction, combined organic phases were washed using a saturated saline solution (50 mL× 2 times) first and then dried using anhydrous sodium sulfate, vacuum concentration was carried out to obtain a residue, and the residue was purified through silica-gel column chromatography (eluent: n-hexane/ethyl acetate=3/1) to obtain 4 g of 3-bromo-2,4-difluorobenzylalcohol.

### Step C: synthesis of 2-bromo-4-bromomethyl-1,3-difluorobenzene

At a room temperature, carbon tetrabromide (6.24 g, 27 mmol) and triphenylphosphine (7.08 g, 27 mmol) were added into a dichloromethane (40 mL) solution of 3-bromo-2,4-difluorobenzylalcohol (4 g, 18.1 mmol) to react for 2 hours.

After the reaction was monitored to be ended via LC-MS, ethyl acetate (100 mL × 2 times) was used for extraction, combined organic phases were washed using a saturated saline solution (50 mL × 2 times) first and then dried using anhydrous sodium sulfate, vacuum concentration was carried out to obtain a residue, and the residue was purified through silica-gel column chromatography (eluent: n-hexane) to obtain 3.3 g of 2-bromo-4-bromomethyl-1,3-difluorobenzene.

### Step D: synthesis of tert-butyl (R)-3-(S)-1-(S)-4-benzyl-2-oxoxazolidin-3-yl)-3-(3-brom o-2,4-difluorophenyl)-1-oxoprop-2-yl)pyrrolidine-1-carboxylate

At -78°C, a tetrahydrofuran solution (14 mL, 1.0 mol/L) of lithium bis(trimethylsilyl)amide was slowly added dropwise into a tetrahydrofuran (40 mL) solution of tert-butyl (R)-3-(2-(S)-4-benzyl-2-oxoxazolidin-3-yl)-2-oxoethyl)pyrrolidine-1-carboxyl ate (4.06 g, 10.5 mmol). After reaction for 0.5 hours, a tetrahydrofuran solution (30 mL) of 2-bromo-4-bromomethyl-1,3-difluorobenzene (3.3 g, 11.5 mmol) was slowly added, and then the temperature was raised to a room temperature naturally to react overnight.

After the reaction was monitored to be ended via LC-MS, a saturated ammonium chloride aqueous solution was added for quenching, ethyl acetate (100 mL ×2 times) was used for extraction, combined organic phases were washed using a saturated saline solution (50 mL ×2 times) first and then dried using anhydrous sodium sulfate, vacuum concentration was carried out to obtain a residue, and the residue was purified through silica-gel column chromatography (eluent: n-hexane/ethyl acetate=4/1) to obtain 2 g of tert-butyl (R)-3-(S)-1-(S)-4-benzyl-2-oxoxazolidin-3-yl)-3-(3-bromo-2,4-difluorophenyl)-1-oxoprop-2 -yl)pyrrolidine-1-carboxylate. LC-MS: RT = 2.09 min, [M+H-Boc]+ = 493.02.

### Step E: synthesis of (S)-3-(3-bromo-2,4-difluorophenyl)-2-(R)-1-tert-butoxycarbonyl)pyrrolid-3-yl propanoic acid

Under an ice water bath, tert-butyl ((R)-3-(S)-1-(S)-4-benzyl-2-oxoxazolidin-3-yl)-3-(3-bromo-2,4-difluorophenyl)-1-oxoprop-2-yl)pyrrolidine-1-carboxylate (2 g, 3.4 mmol) was dissolved in tetrahydrofuran (20 mL), 30% hydrogen peroxide (0.77 g, 6.8 mmol) was added, after 0.5 hours, an aqueous solution (5 mL) of lithium hydroxide monohydrate (0.29 g, 6.8 mmol) was added, and a temperature was raised to a room temperature to react for 3 hours.

After the reaction was monitored to be ended via LC-MS, an excessive sodium hydrogen sulfate saturated solution was slowly added into a reaction liquid to quench the reaction, stirring was carried out for 10 minutes, then a citric acid saturated aqueous solution was added to adjust pH to 5, ethyl acetate (50 mL × 2 times) was used for extraction, combined organic phases were washed using a saturated saline solution (20 mL× 2 times) first and then dried using anhydrous sodium sulfate, vacuum concentration was carried out to obtain a residue, and the residue was purified through silica-gel column chromatography (eluent: n-hexane/ethyl acetate=2/1) to obtain 850 mg of (S)-3-(3-bromo-2,4-difluorophenyl)-2-(R)-1-tert-butoxycarbonyl)pyrrolid-3-yl propanoic acid. LC-MS: RT = 2.07 min, [M-H]⁻ = 431.95.

### Step F: synthesis of tert-butyl (R)-3-(S)-3-(3-bromo-2,4-difluorophenyl)-1-tert-butoxy-1-oxoprop-2-yl)pyrrolidine-1-carboxylate

At a room temperature, (S)-3-(3-bromo-2,4-difluorophenyl)-2-(R)-1-tert-butoxycarbony 1 pyrrolid-3-yl propanoic acid (850 mg, 1.96 mmol) and tert-butyl (Z)-N,N'-diisopropylcarbamate (1.18 g, 5.88 mmol) were dissolved in 2-methyltetrahydrofuran (10 mL), nitrogen displacement was carried out 3 times, and the temperature was raised to 65°C to react overnight.

After the reaction was monitored to be ended via LC-MS, filtering was carried out, a filter cake was washed with ethyl acetate, a filtrate was diluted with ethyl acetate, combined organic phases were washed using a saturated saline solution (10 mL ×2 times) first and then dried using anhydrous sodium sulfate, vacuum concentration was carried out to obtain a residue, and the residue was purified through silica-gel column chromatography (eluent: n-hexane/ethyl acetate=9/1) to obtain 680 mg of tert-butyl (R)-3-(S)-3-(3-bromo-2,4-difluorophenyl)-1-tert-butoxy-1-oxoprop-2-yl)pyrrolidine-1-carbo xylate. LC-MS: RT = 2.34 min, [M+H-Boc]⁺ = 390.01.

### Step G: synthesis of tert-butyl (R)-3-(S)-1-tert-butoxy-3-(2,4-difluoro-3-vinylphenyl)-1-oxoprop-2-yl)pyrrolidine-1-carboxylate

At a room temperature, tert-butyl (R)-3-(S)-3-(3-bromo-2,4-difluorophenyl)-1-tert-but oxy-1-oxoprop-2-yl)pyrrolidine-1-carboxylate (680 mg, 1.39 mmol), potassium vinyltrifluoroborate (279 mg, 2.09 mmol), palladium chloride (8.12 mg, 0.07 mmol), triphenylphosphine (36.4 mg, 0.14 mmol) and cesium carbonate (1.35 g, 4.17 mmol) were dissolved in tetrahydrofuran/water (10 mL/4 mL), N2 displacement was carried out three times, and then the temperature was raised to 75°C to react overnight.

After the reaction was monitored to be ended via TLC, the temperature was lowered to the room temperature, water (10 mL) was added for dilution, then ethyl acetate (50 mL, 2 times) was used for extraction, combined organic phases were washed using a saturated saline solution (20 mL × 2 times) first and then dried using anhydrous sodium sulfate, vacuum concentration was carried out to obtain a residue, and the residue was purified through silica-gel column chromatography (eluent: n-hexane/ethyl acetate=4/1) to obtain 510 mg of tert-butyl (R)-3-(S)-1-tert-butoxy-3-(2,4-difluoro-3-vinylphenyl)-1-oxoprop-2-yl )pyrrolidine-1-carboxylate.

### Step E: synthesis of tert-butyl (R)-3-(S)-1-tert-butoxy-3-(2,4-difluoro-3-formylphenyl)-1-oxoprop-2-yl)pyrrolidine-1-carboxylate

Under an ice water bath, tert-butyl (R)-3-(S)-1-tert-butoxy-3-(2,4-difluoro-3-vinylphen yl)-1-oxoprop-2-yl)pyrrolidine-1-carboxylate (510 mg, 1.17 mmol) was dissolved in tetrahydrofuran/water (8 mL/2 mL), potassium osmate dihydrate (4.29 mg, 0.012 mmol) was added and stirred for 0.5 hours, and then sodium periodate (548 mg, 2.56 mmol) was added to continuously react for 1 hour. The reaction was continued with a temperature raised to a room temperature, after the reaction was monitored to be ended via LC-MS, an excessive sodium hydrogen sulfate aqueous solution was slowly poured into the system to quench the reaction, stirring was carried out for 30 minutes, a mixed liquor was extracted with ethyl acetate (50 mL × 2 times), combined organic phases were washed using a saturated saline solution (20 mL × 2 times) first and then dried using anhydrous sodium sulfate, vacuum concentration was carried out to obtain a residue, and the residue was purified through silica-gel column chromatography (eluent: n-hexane/ethyl acetate=2/1) to obtain 220 mg of tert-butyl (R)-3-(S)-1-tert-butoxy-3-(2,4-difluoro-3-formylphenyl)-1-oxoprop-2-yl)pyrrolidine-1-carboxylate. LC-MS: RT = 2.26min, [M+H-Boc-tert-butyl]⁺ = 284.07.

### Step G: synthesis of (R)-3-(S)-1-tert-butoxy)-3-(3-((3-(S)-3-tert-butoxy)-2-(R)-1-tert-butoxycarbonyl)pyrrolid-3-yl)-3-oxopropyl)-2,6-difluorobenzyl)amino)methyl)phenyl)-1-ox oprop-2-yl)pyrrolidine-1-carboxylate

At a room temperature, tert-butyl (R)-3-(S)-1-tert-butoxy-3-(2,4-difluoro-3-formylphen yl)-1-oxoprop-2-yl)pyrrolidine-1-carboxylate (220 mg, 0.5 mmol) and tert-butyl (R)-3-(S)-3-(3-aminomethyl)phenyl)-1-tert-butoxy-1-oxoprop-2-yl)pyrrolidine-1-carboxylat e (202 mg, 0.5 mmol) were dissolved in DCE (5 mL), triacetoxyl sodium borohydride (371 mg, 1.75 mmol) was added in batches, the temperature was raised to 45°C, and stirring was continued for 16 hours.

After the reaction was monitored to be complete via LC-MS, an aqueous solution (1 mL) was added to quench the reaction, and an obtained crude product was purified through silica-gel column chromatography (eluent: ethyl acetate/n-hexane=1/5). 160 mg of (R)-3-(S)-1-tert-butoxy)-3-(3-((3-(S)-3-tert-butoxy)-2-(R)-1-tert-butoxycarbonyl)pyrrolid-3-yl)-3-oxopropyl)-2,6-difluorobenzyl)amino)methyl)phenyl)-1-oxoprop-2-yl)pyrrolidine-1-ca rboxylate was obtained. LC-MS: RT = 2.05 min, [M+H]⁺ =828.51.

### Step H: synthesis of (R)-3-(S)-1-tert-butoxy)-3-(3-((3-(S)-3-tert-butoxy)-2-(R)-1-(tert-butoxycarbonyl)pyrrolid-3-yl)-3-oxopropyl)-N-(3-(S)-3-tert-butoxy-2-((R)-1-(tert-butoxycar bonyl)pyrrolid-3-yl)-3-oxopropyl)-2,6-difluorobenzyl)phenyl)sulfonamido)methyl)phenyl)-1-oxoprop-2-yl)pyrrolidine-1-carboxylate

At a room temperature, (R)-3-(S)-1-tert-butoxy)-3-(3-((3-(S)-3-tert-butoxy)-2-(R)-1-tert -butoxycarbonyl)pyrrolid-3-yl)-3-oxopropyl)-2,6-difluorobenzyl)amino)methyl)phenyl)-1-o xoprop-2-yl)pyrrolidine-1-carboxylate (160 mg, 0.19 mmol) was dissolved in pyridine (4 mL), and an acetonitrile solution (2 mL) of tert-butyl (R)-3-(S)-1-tert-butoxy-3-(3-chlorosulfonyl)phenyl-1-oxoprop-2-yl)pyrrolidine-1-carboxylat e (110 mg, 0.23 mmol) was slowly added to react at the room temperature for 0.5 hours.

After the reaction was detected to be ended through TLC, ethyl acetate (10 mL× 2 times) was used for extraction, combined organic phases were washed using a saturated saline solution (5 mL× 2 times) first and then dried using anhydrous sodium sulfate, vacuum concentration was carried out to obtain a residue, and the residue was purified through silica-gel column chromatography (eluent: n-hexane/ethyl acetate=3/1) to obtain 170 mg of (R)-3-(S)-1-tert-butoxy)-3-(3-((3-(S)-3-tert-butoxy)-2-(R)-1-(tert-butoxycarbonyl)pyrrolid-3 -yl)-3-oxopropyl)-N-(3-(S)-3-tert-butoxy-2-((R)-1-(tert-butoxycarbonyl)pyrrolid-3-yl)-3-ox opropyl)-2,6-difluorobenzyl)phenyl)sulfonamido)methyl)phenyl)-1-oxoprop-2-yl)pyrrolidin e-1-carboxylate.

### Step I: synthesis of (S)-3-(3-(S)-2-carboxyl-2-(R)-pyrrolid-3-yl)ethyl)-2,6-difluoroben zyl)-N-(3-(S)-2-carboxyl-2-(R)-pyrrolid-3-yl)ethyl)benzyl)aminosulfonyl)phenyl)-2-(R)-pyr rolid-3-yl propanoic acid

At a room temperature, (R)-3-(S)-1-tert-butoxy)-3-(3-((3-(S)-3-tert-butoxy)-2-(R)-1-( tert-butoxycarbonyl)pyrrolid-3-yl)-3-oxopropyl)-N-(3-(S)-3-tert-butoxy-2-((R)-1-(tert-butox ycarbonyl)pyrrolid-3-yl)-3-oxopropyl)-2,6-difluorobenzyl)phenyl)sulfonamido)methyl)phen yl)-1-oxoprop-2-yl)pyrrolidine-1-carboxylate (170 mg, 0.13 mmol) was dissolved in a 1,4-dioxane solution (4 mL), concentrated hydrochloric acid (0.2 mL) was added, and the temperature was raised to 45°C to react for 4 hours.

The reaction was monitored to be complete via LC-MS, vacuum concentration was carried out, and a residue was purified through preparative high performance liquid chromatography to obtain 53 mg of (S)-3-(3-(S)-2-carboxyl-2-(R)-pyrrolid-3-yl)ethyl)-2,6-difluorobenzyl)-N-(3-(S)-2-carboxyl-2-(R)-pyrrolid-3-yl)ethyl)benzyl)aminosulfonyl)phenyl)-2-(R)-pyrrolid-3-yl propanoic acid. LCMS: RT=1.51min, [M-H]⁻=795.05. NMR data: ¹H NMR (400 MHz, Deuterium Oxide) δ 8.25 (s, 1H), 7.59 - 7.38 (m, 4H), 7.09 (t, J = 7.6 Hz, 1H), 6.96 (m, 3H), 6.85 (s, 1H), 6.64 (t, J = 8.9 Hz, 1H), 4.34 (d, J = 23.8 Hz, 4H), 3.44 (m, 3H), 3.33 (m, 3H), 3.16 (m, 3H), 2.90 (m, 3H), 2.80 (d, J = 9.2 Hz, 2H), 2.73 - 2.61 (m, 2H), 2.57 (m, 2H), 2.53 - 2.47 (m, 1H), 2.47 - 2.30 (m, 5H), 2.06 (m, 3H), 1.67 (m, 3H).

### Example 34

### Synthesis of (2S,2'S)-3,3'-(((((3-((S)-2-carboxyl-2-((R)-pyrrolid-3-yl)ethyl)-4-fluoroph enyl)sulfonyl)azadiyl)bis(methylenyl))bis(6-fluoro-3,1-phenylenyl))bis(2-((R)-pyrrolid-3-yl )propanoic acid)

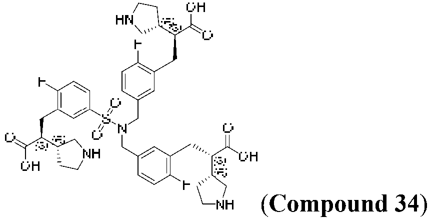

Preparation was performed with reference to the method for the compound 9. LCMS: RT = 1.55 min, [M-H]⁻ = 813.13.

### Example 35

### Synthesis of (2S,2'S)-3,3'-(((((3-((S)-2-carboxyl-2-((R)-pyrrolid-3-yl)ethyl)-4-chloroph enyl)sulfonyl)azadiyl)bis(methylenyl))bis(3,1-phenylenyl))bis(2-((R)-pyrrolid-3-yl)propanoi c acid)

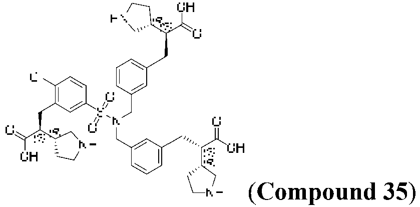

Preparation was performed with reference to the method for the compound 9. LCMS: RT = 1.53 min, [M-H]⁻ = 793.09.

### Example 36

### Synthesis of (2S,2'S)-3,3'-(((((3-((S)-2-carboxyl-2-((R)-pyrrolid-3-yl)ethyl)-4-chloroph enyl)sulfonyl)azadiyl)bis(methylenyl))bis(6-fluoro-3,1-phenylenyl))bis(2-((R)-pyrrolid-3-yl )propanoic acid)

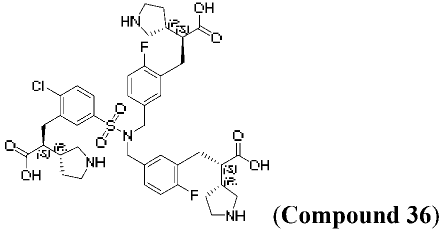

Preparation was performed with reference to the method for the compound 9. LCMS: RT = 1.53 min, [M-H]⁻ = 829.02.

### Example 37

### Synthesis of (S)-3-(3-(2-((3-((S)-2-carboxyl-2-(R)-pyrrolid-3-yl)ethyl)-N-(2-((S)-2-carb oxyl-2-(R)-pyrrolid-3-yl)ethyl)benzyl)phenyl)sulfonamido)ethyl)phenyl)-2-((R)-pyrrolid-3-yl)propanoic acid

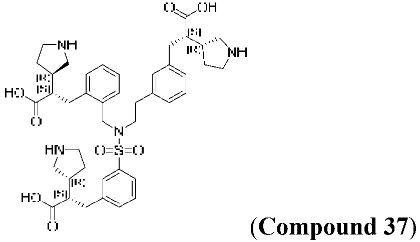

Preparation was performed with reference to the method for the compound 9. LCMS: RT = 1.51 min, [M-H]⁻ = 773.12. ¹H NMR (400 MHz, D₂O) δ 7.67 (dt, J = 5.0, 2.4 Hz, 1H), 7.62 (s, 1H), 7.52 (d, J = 6.0 Hz, 2H), 7.34 - 7.21 (m, 1H), 7.20 - 7.04 (m, 4H), 6.98 (d, J = 7.6 Hz, 1H), 6.73 (d, J = 7.6 Hz, 1H), 6.54 (s, 1H), 4.33 - 4.13 (m, 2H), 3.56 - 3.45 (m, 3H), 3.42 - 3.31 (m, 3H), 3.23 - 3.15 (m, 5H), 3.12 - 2.83 (m, 5H), 2.78 - 2.35 (m, 12H), 2.14 - 2.07 (m, 3H), 1.74 - 1.65 (m, 3H).

### Example 38

### Synthesis of (S)-3-(3-(S)-2-carboxyl-2-(R)-pyrrolid-3-yl)ethyl)-4-chlorobenzyl)-N-(3-(S)-2-carboxyl-2-(R)-pyrrolid-3-yl)ethyl)phenethyl)aminosulfonyl)phenyl)-2-(R)-pyrrolid-3 -yl propanoic acid

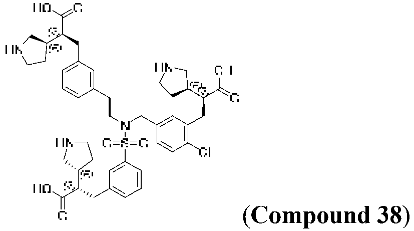

A specific synthetic route is as follows:
Preparation was performed with reference to the method for the compound 9. LCMS: RT = 1.54 min, [M-H]⁻ = 807.11.

### Example 39

### Synthesis of (S)-3-(5-(N-(3-((S)-2-carboxyl-2-((R)-pyrrolid-3-yl)ethyl)-4-chlorobenzyl )-N-(3-((S)-2-carboxyl-2-((R)-pyrrolid-3-yl)ethyl)benzyl)aminosulfonyl)-2-fluorophenyl)-2-((R)-pyrrolid-3-yl)propanoic acid

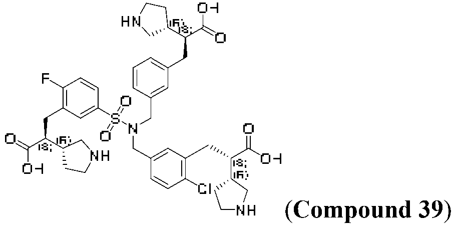

Preparation was performed with reference to the method for the compound 9. LCMS: RT = 1.55 min, [M-H]⁻ = 811.03. ¹H NMR (400 MHz, D₂O) δ 7.69 - 7.58 (m, 1H), 7.57 (dd, J = 6.6, 2.4 Hz, 1H), 7.23 (t, J = 9.2 Hz, 1H), 7.17 (d, J = 8.2 Hz, 1H), 7.12 (t, J = 7.6 Hz, 1H), 7.02 (d, J = 7.6 Hz, 1H), 6.94 (d, J = 7.7 Hz, 1H), 6.92 - 6.85 (m, 1H), 6.84- 6.80 (m, 2H), 4.44 - 4.01 (m, 4H), 3.51 (dd, J = 11.6, 7.4 Hz, 1H), 3.48 - 3.29 (m, 5H), 3.28 - 3.10 (m, 3H), 3.04 - 2.92 (m, 1H), 2.93 - 2.53 (m, 8H), 2.55 - 2.28 (m, 6H), 2.17 - 1.93 (m, 3H), 1.80 - 1.49 (m, 3H).

### Example 40

### Synthesis of (2S,2'S)-3,3'-(3-((S)-2-carboxyl-2-((R)-pyrrolid-3-yl)ethyl)phenyl)sulfonyl )azadiyl)dimethylenyl)bis(6-chloro-3,1-phenylenyl)bis(2-(R)-pyrrolid-3-yl)propanoic acid)

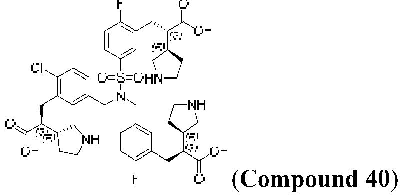

Preparation was performed with reference to the method for the compound 9. LCMS: RT = 1.52 min, [M-H]⁻ = 829.21.

### Example 41

### Synthesis of (2S,2'S)-3,3'-(3-((S)-2-carboxyl-2-((R)-pyrrolid-3-yl)ethyl)phenyl)sulfonyl )azadiyl)dimethylenyl)bis(6-chloro-3,1-phenylenyl)bis(2-(R)-pyrrolid-3-yl)propanoic acid)

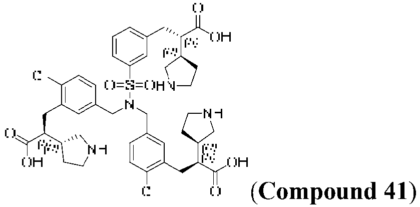

Preparation was performed with reference to the method for the compound 9. LCMS: RT=1.58 min, [M-H]⁻=827.07.

### Example 42

### Synthesis of (2S,2'S)-3,3'(3-((S)-2-carboxyl-2-(R)-pyrrolid-3-yl)ethyl)-4-fluorophenylsu lfonyl)azadiyl)dimethylenyl)bis(6-chloro-3,1-phenylenyl)bis(2-(R)-pyrrolid-3-yl)propanoic acid)

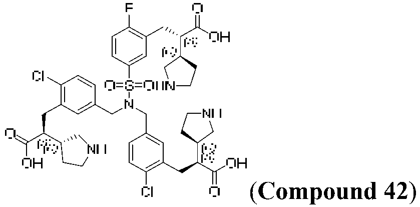

Preparation was performed with reference to the method for the compound 9. LCMS: RT=1.58 min, [M-H]⁻=845.07.

### Examples 33, and 43-47

**Compounds 33 and 43-47 were prepared with reference to preparation methods for the compounds in the aforementioned examples (e.g.: Example 30 or Example 9)**

| No. | Compound | No. | Compound |
|---|---|---|---|
| 33 | 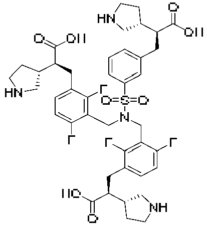 LCMS:[M-H]⁻=831.21 | 43 | 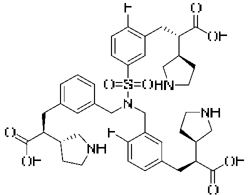 LCMS:[M-H]⁻=795.14 |
| 44 | 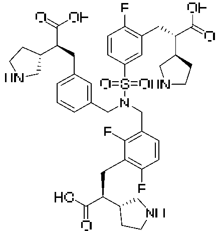 LCMS:[M-H]⁻=813.13 | 45 | 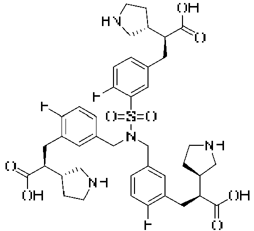 LCMS:[M-H]⁻=813.07 |
| 45 | 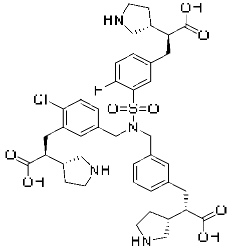 LCMS:[M-H]⁻=811.17 | 47 | 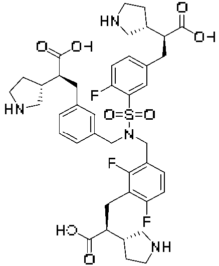 LCMS:[M-H]⁻=813.23 |

### Example 48 In-vitro Lp(a) assembly assay

### Experiment steps

The capability of the compounds in the examples inhibiting the formation of Lp(a) particles in vitro was evaluated through cell-free assembly assay. After incubation at 37°C with 5% CO₂ for 96 h, conditioned media (supplemented with 10% FBS, 20 mM HEPES, and 1 × penicillin/streptomycin DMEM) was collected from confluent wild-type HepG2 cells (source of endogenously expressed ApoB) and an HEK293 stable cell line expressing human Apo(a) protein containing 17 Kringle repeats (selected on 1 ug/ml puromycin). In-vitro assembly assay was performed by combining equal parts of HepG2 and HEK293 conditioned media with compounds under test added in a dilution series (final concentration being 0.03-1000 nM). A reaction was incubated at 37°C for 2 hours, and terminated by adding 6-aminocaproic acid (EACA) to a final concentration of 150 mM. Sandwich ELISA was used to detect Lp(a) using an anti-Apo(a) capture antibody (ab242565) and an HRP-conjugated anti-ApoB detection antibody (ab27622). ELISA was performed using TMB staining, and terminated with 1N sulfuric acid, and signals were read at 450 nm on an Envision 2104 plate reader. By setting an assembly reaction without inhibitors to 0% inhibition and an assembly reaction with the minimum amount of HepG2 conditioned medium (50-fold dilution) to 100% inhibition, the % inhibition of Lp(a) formed under each test condition was determined. The data were fitted to a 4-parameter curve to determine an IC₉₀ value, results as shown in Table 1.

**Table 1 IC₉₀ values of sulfonamide compounds**

| Example compound | IC₉₀(nM) | Example compound | IC₉₀(nM) | Example compound | IC₉₀(nM) |
|---|---|---|---|---|---|
| **1** | 49.89 | **15** | 16.01 | **30** | 28.96 |
| **2** | 62.63 | **16** | 23.76 | **31** | 21.11 |
| **3** | 61.05 | **19** | 40.88 | **32** | 31.45 |
| **4** | 40.28 | **20** | 30.74 | **34** | 17.7 |
| **5** | 37.85 | **21** | 33.92 | **35** | 41.28 |
| **6** | 47.77 | **23** | 76.18 | **36** | 21.58 |
| **7** | 69.92 | **24** | 25.75 | **38** | 81.77 |
| **8** | 26.09 | **25** | 81.7 | **39** | 24.87 |
| **9** | 45.79 | **26** | 86.71 | **40** | 15.72 |
| **10** | 52.31 | **27** | 79.58 | **41** | 38.15 |
| **13** | 11.5 | **28** | 86.47 | **42** | 25.05 |
| **14** | 24.42 | **29** | 24.87 | **43** | 19.5 |

It can be seen from the results in Table 1 that, the sulfonamide compounds in the present application have good LP(a) inhibition activities.

### Example 49

### SD rat pharmacokinetic experiment

### (1) Experiment materials

SD rats: male, 180-250 g, purchased from Guangdong Vitalriver Experimental Animal Technology Co., Ltd.

Reagents: normal saline, EDTA-K₂ (anticoagulant), TCA (trichloroacetic acid), and Propranolol (internal standard) are all commercially available.

Instruments: Nexera LC-40; AB SCIEX QTRAP 5500+.

### (2) Experiment method

Compounds were weighed and dissolved in a normal saline system, after intragastric administration to rats, 200 µL of venous blood was collected at 15 min, 30 min, 1 h, 2 h, 5 h, 7 h, and 24 h (collected at 5 min further for the iv group) into EDTA-K₂ anticoagulant EP tubes, the tubes were centrifuged at 12000 rpm for 2 min, and plasma was taken and frozen at -80°C for testing. A certain amount of samples for test were weighed accurately and dissolved in ultrapure water to 2 mg/mL as stock solutions. An appropriate amount of compound stock solutions were extracted accurately and diluted with 50% acetonitrile-water to prepare standard series solutions. 10 µL of each of the above standard series solutions was extracted accurately, 90 µL of blank plasma was added, even vortex mixing was carried out to prepare plasma samples with plasma concentrations of 1, 3, 5, 10, 30, 100, 300, 1000, and 3000 ng/mL, and the concentrations of quality control samples were 9, 240, and 2400 ng/ml. Two-sample analysis was performed for each concentration, and standard curves were established. 30 µL of plasma (diluted 5-fold by intravenous administration for 5 min, 15 min, and 30 min) was taken, 200 µL of a 5% trichloroacetic acid-water solution of internal-standard Propranolol (50 ng/mL) was added, after even vortex mixing, centrifugation was performed at 4000 rpm for 10 min, supernatants were taken, 150 µL of purified water was added, even vortex mixing was carried out again, and LC-MS/MS analysis was performed. LC-MS/MS detection conditions were as follows:
Chromatographic column: YMC Triart C18, 50*3.0 mm, 2.1 µm.
Mobile phase: water (0.1% formic acid)- acetonitrile. Gradient elution was carried out according to the following table.

**Table 2 Gradient elution table**

| Time (min) | Water (containing 0.1% formic acid) | Acetonitrile |
|---|---|---|
| 0 | 95% | 5% |
| 0.60 | 95% | 5% |
| 1.50 | 30% | 70% |
| 3.00 | 30% | 70% |
| 3.01 | 95% | 5% |
| 3.50 | 95% | 5% |

### (3) Data processing

After plasma concentrations were detected by LC-MS/MS, pharmacokinetic parameters were calculated with a non-compartment model method by adopting software WinNonlin 6.1, results as shown in Table 3.

**Table 3 SD rat pharmacokinetic parameters of compounds in the present application**

| Example compound | Dose (mg/kg) Administrat ion route | AUC₀₋ₜ (h*ng/ml) |
|---|---|---|
| CN114008021A | 5, p.o | 3203 |
| Compound 1 | | |
| Compound 1 | 5, p.o | 3496 |
| Compound 2 | 5, p.o | 4335 |
| Compound 3 | 5, p.o | 4228 |
| Compound 4 | 5, p.o | 6013 |
| Compound 6 | 5, p.o | 5137 |
| Compound 9 | 5, p.o | 8010 |
| Compound 10 | 5, p.o | 8030 |
| Compound 13 | 5, p.o | 3788 |
| Compound 15 | 5, p.o | 4443 |
| Compound 16 | 5, p.o | 4693 |
| Compound 20 | 5, p.o | 4092 |

It can be seen from the results in Table 3 that, the compounds in the present application have a higher exposed quantity, and additionally, exhibit a longer half-life period and a higher bioavailability.

### Example 50

### Pharmacokinetic experiment for monkeys

### (1) Experiment materials

Beagle dogs: male, 8-10 kg, purchased from Beijing Marshall Biotechnology Co., Ltd.

Cynomolgus monkey: male, 3-6 kg, purchased from Guangzhou Huazhen biotechnology Co., Ltd. and Hainan Jingang Biotech Co., Ltd.

Reagents: normal saline, EDTA-K₂ (anticoagulant), TCA (trichloroacetic acid), and Propranolol (internal standard) are all commercially available.

Instruments: Nexera LC-40; AB SCIEX QTRAP 5500+.

### (2) Experiment method

Compounds were weighed and dissolved in a normal saline system, after intragastric or intravenous injection administration to experimental animals, 200 µL of venous blood was collected at 15 min, 30 min, 1 h, 2 h, 4 h, 8 h, and 24 h (collected at 5 min and 48 h further for the iv group) into EDTA-K₂ anticoagulant EP tubes, the tubes were centrifuged at 12000 rpm for 2 min, and plasma was taken and frozen at -80°C for testing. A certain amount of samples for test were weighed accurately and dissolved in ultrapure water to 2 mg/mL as stock solutions. An appropriate amount of compound stock solutions were extracted accurately and diluted with 50% acetonitrile-water to prepare standard series solutions. 10 µL of each of the above standard series solutions was extracted accurately, 90 µL of blank plasma was added, even vortex mixing was carried out to prepare plasma samples with plasma concentrations of 1, 3, 5, 10, 30, 100, 300, 1000, and 3000 ng/mL, and the concentrations of quality control samples were 9, 240, and 2400 ng/mL. Two-sample analysis was performed for each concentration, and standard curves were established. 30 µL of plasma (diluted 5-fold by intravenous administration for 5 min, 15 min, and 30 min) was taken, 200 µL of a 5% trichloroacetic acid-water solution of internal-standard Propranolol (50 ng/mL) was added, after even vortex mixing, centrifugation was performed at 4000 rpm for 10 min, supernatants were taken, 150 µL of purified water was added, even vortex mixing was carried out again, and LC-MS/MS analysis was performed. LC-MS/MS detection conditions were as follows:
Chromatographic column: YMC Triart C18, 50*3.0 mm, 2.1 µm.
Mobile phase: water (0.1% formic acid)- acetonitrile. Gradient elution was carried out according to the following table.

**Table 4 Gradient elution table**

| Time (min) | Water (containing 0.1% formic acid) | Acetonitrile |
|---|---|---|
| 0 | 95% | 5% |
| 0.60 | 95% | 5% |
| 1.50 | 30% | 70% |
| 3.00 | 30% | 70% |
| 3.01 | 95% | 5% |
| 3.50 | 95% | 5% |

### (3) Data processing

After plasma concentrations were detected by LC-MS/MS, pharmacokinetic parameters were calculated with a non-compartment model method by adopting software WinNonlin 6.1, results as shown in Table 5 and Table 6.

**Table 5 Cynomolgus monkey pharmacokinetic parameters of compounds in the present application**

| Example compound | Dose (mg/kg) Administrat ion route | Cₘₐₓ (ng/ml) | AUC₀₋ₜ (h*ng/ml) | F(%) |
|---|---|---|---|---|
| CN114008021A | 5, p.o | 707 | 12600 | 4.32 |
| Compound 1 | | | | |
| Compound 9 | 5, p.o | 1690 | 22100 | 7.49 |

**Table 6 Cynomolgus monkey pharmacokinetic parameters of compounds in the present application**

| Example compound | Dose (mg/kg) Administrat ion route | AUC₀₋ₜ (h*ng/ml) | T_{1/2} (h) |
|---|---|---|---|
| CN114008021A | 0.5, i.v | 35900 | 17.5 |
| Compound 1 | | | |
| Compound 1 | 0.5, i.v | 41000 | 21 |
| Compound 3 | 0.5, i.v | 33300 | 31.6 |

It can be seen from the results in Table 5 and Table 6 that, the compounds in the present application have a higher exposed quantity, a higher bioavailability, and a longer half-life period.

It should be understood that the above examples are preferred implementations of the present application, but the implementations of the present application are not limited by the above examples. For those of ordinary skills in the art, improvements or transformations can be made based on the above description, and all of these improvements and transformations should fall within the scope of protection of the claims attached to the present application.

## Claims

1. A sulfonamide compound, or an isomer thereof, or a racemate thereof, or a pharmaceutic salt thereof, wherein a structure of the sulfonamide compound is as shown in general formula I: 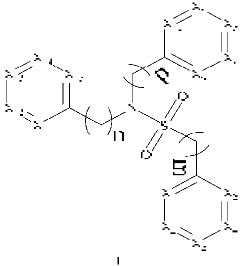
wherein X₁-X₁₅ are each independently selected from the group consisting of -N- and -CR₁, R₁ is selected from the group consisting of H, halogen, -OH, -NH₂, -CN, -NO₂, -COOH, -SO₃H, substituted or unsubstituted C₁-C₆ alkyl, substituted or unsubstituted C₁-C₆ alkoxy, substituted or unsubstituted C₃-C₁₀ cycloalkyl, substituted or unsubstituted 3-10-membered heterocycloalkyl, substituted or unsubstituted C₆-C₁₀ aryl, substituted or unsubstituted 5-10-membered heteroaryl, and 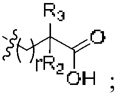
at least one of X₁-X₁₅ is -N-; or
when X₁-X₁₅ are each independently selected from -CR₁, wherein at least one R₁ is not hydrogen, at least one R₁ in X₁-X₅ is independently selected from 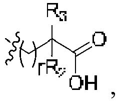 and at least one R₁ in X₆-X₁₀ is independently selected from 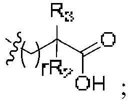 or
when X₁-X₁₅ are each independently selected from -CR₁, any two adjacent R₁ in X₁-X₁₅, together with connected carbon atoms, form substituted or unsubstituted C₆-C₁₀ aryl or substituted or unsubstituted 5-10-membered heteroaryl;
R₂ is selected from the group consisting of -H, halogen, substituted or unsubstituted C₁-C₆ alkyl, and substituted or unsubstituted C₁-C₆ alkoxy;
R₃ is selected from -(CH₂)_{q}-(5-8-membered heterocyclyl);
substituents in substituted C₁-C₆ alkyl, substituted C₁-C₆ alkoxy, substituted C₃-C₁₀ cycloalkyl, substituted 3-10-membered heterocycloalkyl, substituted C₆-C₁₀ aryl or substituted 5-10-membered heteroaryl are independently selected from the group consisting of halogen, hydroxyl, carboxyl, nitro, cyano, C₁-C₆ alkyl, C₁-C₆ halogenated alkyl, C₁-C₆ halogenated alkoxy, C₁-C₆ alkoxy, C₃-C₆ cycloalkyl, 3-6-membered heterocycloalkyl, C₃-C₆ cycloalkoxy, C₆-C₁₀ aryl, 5-10-membered heteroaryl, and a combination thereof; and
n, p and r are integers independently selected from 1, 2 or 3, and m and q are integers selected from 0, 1, 2 or 3.

2. The sulfonamide compound, or the isomer thereof, or the racemate thereof, or the pharmaceutic salt thereof according to claim 1, wherein R₁ is selected from the group consisting of H, halogen, -OH, -NH₂, -CN, -NO₂, -COOH, C₁-C₆ alkyl, C₁-C₆ halogenated alkyl, C₁-C₆ alkoxy, C₁-C₆ halogenated alkoxy, substituted or unsubstituted C₃-C₁₀ cycloalkyl, substituted or unsubstituted 3-10-membered heterocycloalkyl, substituted or unsubstituted C₆-C₁₀ aryl, substituted or unsubstituted 5-10-membered heteroaryl, and 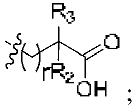 R₂ is selected from the group consisting of -H, and substituted or unsubstituted C₁-C₆ alkyl; R₃ is selected from 5-8-membered heterocyclyl; and substituents in substituted C₃-C₁₀ cycloalkyl, substituted 3-10-membered heterocycloalkyl, substituted C₆-C₁₀ aryl or substituted 5-10-membered heteroaryl are independently selected from the group consisting of halogen, hydroxyl, carboxyl, nitro, cyano, C₁-C₆ alkyl, C₁-C₆ halogenated alkyl, C₁-C₆ halogenated alkoxy, C₁-C₆ alkoxy, C₃-C₆ cycloalkyl, 3-6-membered heterocycloalkyl, C₃-C₆ cycloalkoxy, C₆-C₁₀ aryl, 5-10-membered heteroaryl, and a combination thereof.

3. The sulfonamide compound, or the isomer thereof, or the racemate thereof, or the pharmaceutic salt thereof according to claim 1 or 2, wherein one of X₄ or X₅ is selected from 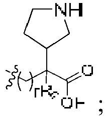 one of X₉ or X₁₀ is selected from 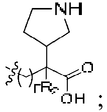 and one of X₁₁ or X₁₂ is selected from

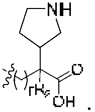

4. The sulfonamide compound, or the isomer thereof, or the racemate thereof, or the pharmaceutic salt thereof according to any one of claims 1-3, wherein a structure of the sulfonamide compound is as shown in general formula II or III: 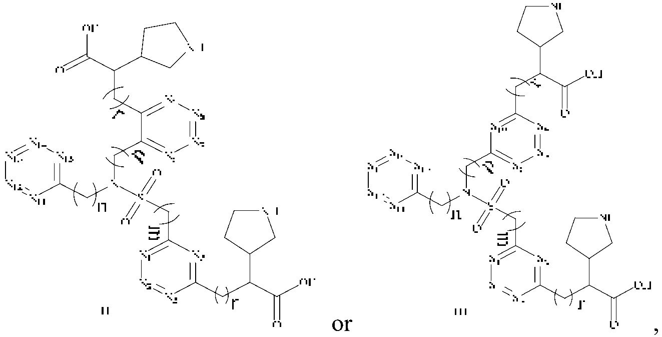 wherein X₁-X₁₅ are each independently selected from the group consisting of -N- and -CR₁, R₁ is selected from the group consisting of H, halogen, substituted or unsubstituted C₁-C₆ alkyl, substituted or unsubstituted C₁-C₆ alkoxy, and 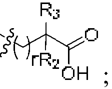 R₂ is selected from the group consisting of H and substituted or unsubstituted C₁-C₆ alkyl; R₃ is selected from 5-8-membered heterocyclyl; and substituents in substituted C₁-C₆ alkyl and substituted C₁-C₆ alkoxy are independently selected from the group consisting of halogen, hydroxyl, carboxyl, nitro, cyano, C₁-C₆ alkyl, C₁-C₆ halogenated alkyl, C₁-C₆ halogenated alkoxy, C₁-C₆ alkoxy, C₃-C₆ cycloalkyl, 3-6-membered heterocycloalkyl, C₃-C₆ cycloalkoxy, C₆-C₁₀ aryl, 5-10-membered heteroaryl, and a combination thereof.

5. The sulfonamide compound, or the isomer thereof, or the racemate thereof, or the pharmaceutic salt thereof according to any one of claims 1-4, wherein a structure of the sulfonamide compound is as shown in general formula IV or V: 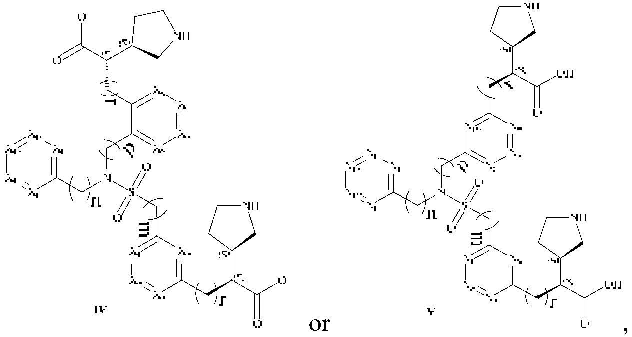 wherein X₁-X₁₅ are each independently selected from the group consisting of -N- and -CR₁, R₁ is selected from the group consisting of H, halogen, substituted or unsubstituted C₁-C₆ alkyl, substituted or unsubstituted C₁-C₆ alkoxy, and 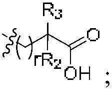 R₂ is selected from the group consisting of H and substituted or unsubstituted C₁-C₆ alkyl; R₃ is selected from 5-8-membered heterocyclyl; substituents in substituted C₁-C₆ alkyl and substituted C₁-C₆ alkoxy are independently selected from the group consisting of halogen, hydroxyl, carboxyl, nitro, cyano, C₁-C₆ alkyl, C₁-C₆ halogenated alkyl, C₁-C₆ halogenated alkoxy, C₁-C₆ alkoxy, C₃-C₆ cycloalkyl, 3-6-membered heterocycloalkyl, C₃-C₆ cycloalkoxy, C₆-C₁₀ aryl, 5-10-membered heteroaryl, and a combination thereof; preferably, any one of X₁₁, X₁₂, X₁₃, X₁₄ or X₁₅ is independently selected from -CR₁, and R₁ is selected from 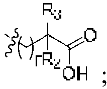 and further preferably, R₁ is any one selected from the group consisting of

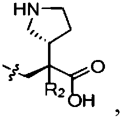

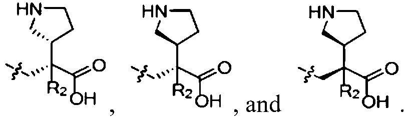

6. The sulfonamide compound, or the isomer thereof, or the racemate thereof, or the pharmaceutic salt thereof according to any one of claims 1-5, wherein R₁ is selected from the group consisting of H, fluorine, chlorine, bromine, iodine, trifluoromethyl, methyl, ethyl, propyl, methoxy, ethoxy, piperazinyl, -CH₂COOH, -CH₂CH₂COOH, -CH₂CH(CH₃)COOH, 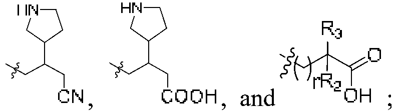 and R₃ is selected from the group consisting of pyrrolidyl, piperidyl, piperazinyl, morpholinyl, pyridyl, pyrimidinyl, and pyrazinyl.

7. The sulfonamide compound, or the isomer thereof, or the racemate thereof, or the pharmaceutic salt thereof according to any one of claims 1-6, wherein 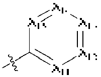 is selected from quinolyl.

8. The sulfonamide compound, or the isomer thereof, or the racemate thereof, or the pharmaceutic salt thereof according to any one of claims 1-7, wherein 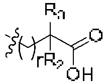 is selected from the group consisting of:

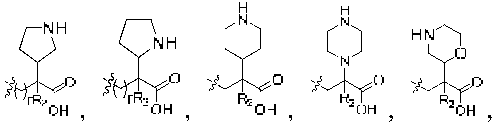

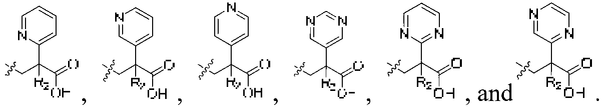

9. The sulfonamide compound, or the isomer thereof, or the racemate thereof, or the pharmaceutic salt thereof according to claim 8, wherein 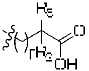 is selected from the group consisting of: 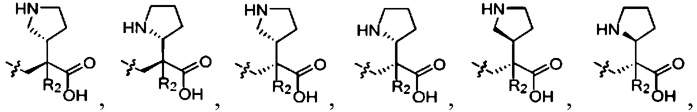 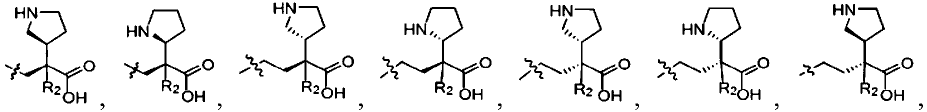 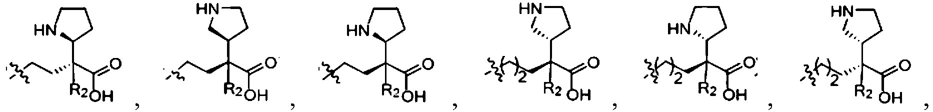 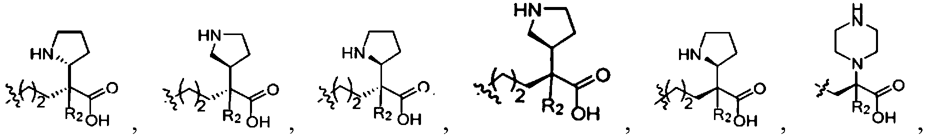 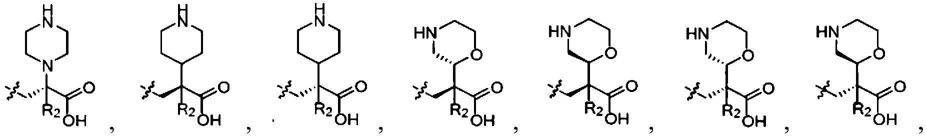 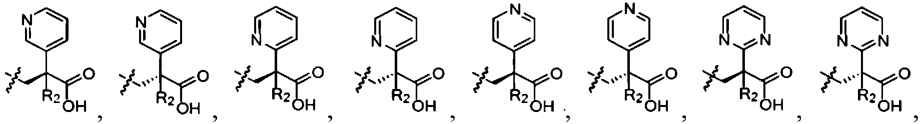 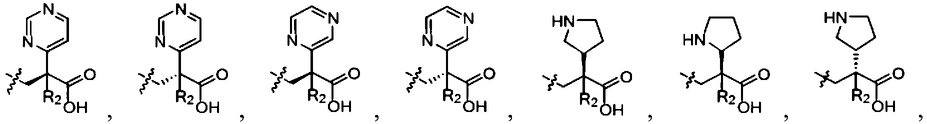 and

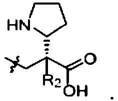

10. The sulfonamide compound, or the isomer thereof, or the racemate thereof, or the pharmaceutic salt thereof according to claim 9, wherein 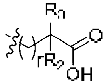 is selected from the group consisting of:

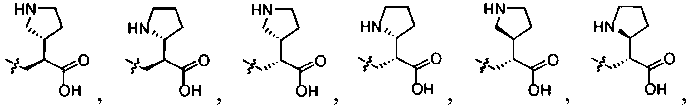

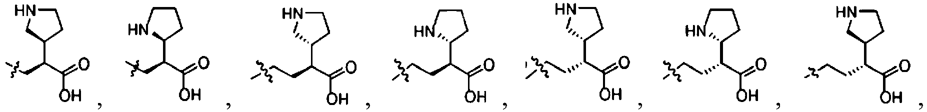

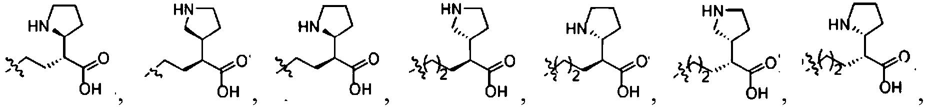

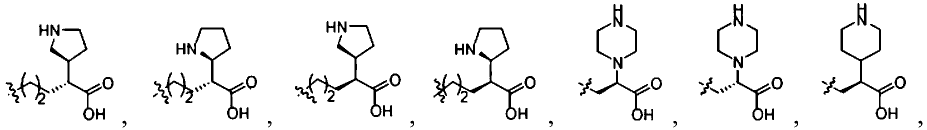

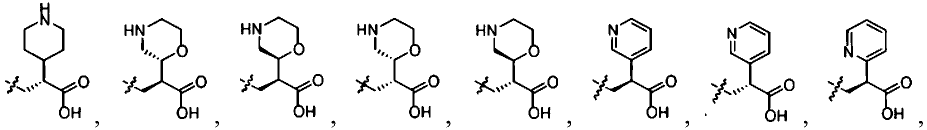

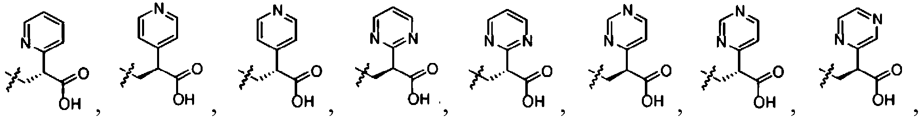

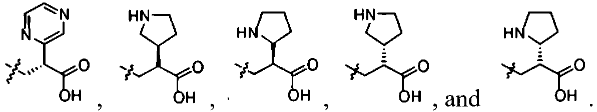

11. The sulfonamide compound, or the isomer thereof, or the racemate thereof, or the pharmaceutic salt thereof according to any one of claims 1-10, wherein the sulfonamide compound is selected from the group consisting of compounds shown in Table 1A or Table 1B.

12. A pharmaceutical composition, comprising the sulfonamide compound, or the isomer thereof, or the racemate thereof, or the pharmaceutic salt thereof according to any one of claims 1-11, and one or more pharmaceutically acceptable excipients and/or carriers.

13. Use of the sulfonamide compound, or the isomer thereof, or the racemate thereof, or the pharmaceutic salt thereof according to any one of claims 1-11 or the pharmaceutical composition according to claim 12 in preparation of medicines for preventing or treating a LP(a)-related disease.

14. The use according to claim 13, wherein the Lp(a)-related disease is selected from a cardiovascular disease.

15. The use according to claim 14, wherein the cardiovascular disease is selected from the group consisting of stroke, atherosclerosis, thrombosis, coronary heart disease, aortic stenosis, and any other disease related to an elevated Lp(a) level.
